(19) Europäisches Patentamt
European Patent Office
Office européen des brevets

(11) EP 4 748 837 A1

(12) EUROPEAN PATENT APPLICATION
published in accordance with Art. 153(4) EPC

(43) Date of publication:
**27.05.2026 Bulletin 2026/22**

(21) Application number: **24844695.7**

(22) Date of filing: **19.07.2024**

(51) International Patent Classification (IPC):
***C07D 401/12*** (2006.01) ***C07D 401/14*** (2006.01)
***C07D 405/14*** (2006.01) ***C07D 413/14*** (2006.01)
***A61K 31/444*** (2006.01) ***A61P 35/00*** (2006.01)

(52) Cooperative Patent Classification (CPC):
**A61K 31/444; A61P 35/00; C07D 401/12; C07D 401/14; C07D 405/14; C07D 413/14**

(86) International application number:
**PCT/CN2024/106286**

(87) International publication number:
**WO 2025/021003 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.07.2023 CN 202310898800**
**09.04.2024 CN 202410417711**

(71) Applicants:
- **Zhejiang Hisun Pharmaceutical Co., Ltd.**
  **Taizhou, Zhejiang 318000 (CN)**
- **Shanghai Aryl Pharmtech Co., Ltd.**
  **Songjiang, Shanghai 201612 (CN)**

(72) Inventors:
- **QIU, Zongxing**
  **Shanghai 201612 (CN)**
- **ZHANG, Panpan**
  **Taizhou, Zhejiang 318000 (CN)**
- **PENG, Jilei**
  **Shanghai 201612 (CN)**
- **CAO, Hai**
  **Shanghai 201612 (CN)**
- **WANG, Zefeng**
  **Shanghai 201612 (CN)**
- **SHI, Yaqiong**
  **Shanghai 201612 (CN)**
- **YE, Cheng**
  **Shanghai 201612 (CN)**
- **XU, Daiwang**
  **Taizhou, Zhejiang 318000 (CN)**
- **XU, Xiaojie**
  **Taizhou, Zhejiang 318000 (CN)**
- **ZHOU, Houjiang**
  **Taizhou, Zhejiang 318000 (CN)**

(74) Representative: **Kador & Partner Part mbB**
**Corneliusstraße 15**
**80469 München (DE)**

# (54) AROMATIC AMIDE DERIVATIVE, AND PREPARATION METHOD THEREFOR AND USE THEREOF

(57) The present invention relates to an aromatic amide derivative, and a preparation method therefor and use of a pharmaceutical composition containing the derivative in medicine. Specifically, the invention relates to an aromatic amide derivative as shown in general formula (I), and a preparation method therefor and a pharmaceutically acceptable salt thereof, and use thereof as a therapeutic agent, in particular a KIF18A inhibitor. The definition of each group in general formula (I) is the same as the definition in the specification.

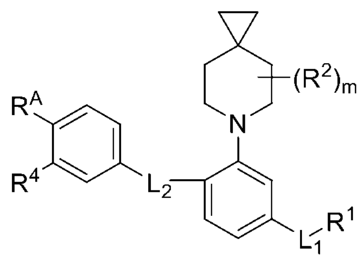

**(I)**

EP 4 748 837 A1

## Description

### TECHNICAL FIELD

The present invention relates to an aromatic amide derivative, a preparation method therefor, a pharmaceutical composition containing the derivative and use thereof as a therapeutic agent, particularly a use as a KIF18A inhibitor.

### BACKGROUND

Kinesin molecule is a motor protein with a microtubule serving as an orbit, and plays an important role in organelle migration, tissue and organ development, signal transduction, mitosis, meiosis, and other processes. Multiple microtubule-associated proteins (MAPs) of kinesin-8 family play a role in regulating the dynamic instability of microtubule by influencing the polymerization and depolymerization of microtubule. KIF18A is a member of kinesin-8 family, can move towards a plus-end of microtubule with the microtubule serving as the orbit, and tends to bind to a longer microtubule, the activity is length-dependent and influences spindle length, which can ensure the timely and smooth completion of sister chromatid alignment, and the function of KIF18A is highly conserved and remarkably similar across different species.

KIF18A is a molecular motor protein moving towards the plus-end of microtubule with the microtubule serving as an orbit, regulates chromosome congression by influencing the dynamic instability of the end of microtubule, and plays a role in a mitosis phase. In a mitosis anaphase, KIF18A undergoes ubiquitination-mediated degradation, which ensures the accurate chromosome separation during mitosis, and promotes the smooth completion of mitosis and cytokinesis. In a mitosis prophase, the localization of KIF18A at the plus-ends of microtubules close to a kinetochores is a necessary condition to exert the function, the localization depends not only on the motor activity of its N-terminal domain, but also critically on the microtubule-binding ability of its tail domain. KIF18A is also modified by reversible protein phosphorylation/dephosphorylation, but there is still a lack of systematic study on how the post-translational modification of this protein regulates the function of KIF18A. The estrogen receptor ERα can bind to KIF18A and promote its transcription, but it is still unclear whether KIF18A is regulated by other transcription factors, so that a gene transcription regulatory mechanism of KIF18A needs to be further studied. During meiosis, cells lacking KIF18A fail to complete meiosis, which will lead to dyszoospermia and testicular dysgenesis of male animals.

Studies have revealed that KIF18A protein is highly expressed in many cancers, comprising but not limited to hepatocellular carcinoma, glioblastoma, colon cancer, breast cancer, lung cancer, cholangiocarcinoma, pancreatic cancer, prostate cancer, bladder cancer, head cancer, neck cancer, cervical cancer, ovarian cancer, synovial sarcoma, rhabdomyosarcoma, and the like, which suggests that KIF18A is closely associated with the occurrence and development of tumors, making it a potential target for molecular diagnosis and treatment of various tumors. The expression of KIF18A is related to the development of clinical colorectal cancer. Studies have shown that KIF18A can induce Akt phosphorylation, knockout of KIF18A in mice significanlty promote apoptosis, and it is hypothezised that KIF18A facilitates the occurrence and development of colorectal cancer by activating the PI3K-Akt signaling pathway. KIF18A is also highly expressed in human breast cancer cells, and its overexpression is related to the grade, migration and prognosis of breast tumor. Studies on breast cancer cells have found that overexpression of KIF18A leads to the generation of multinucleated cells, while low expression of KIF18A can greatly weaken a cell proliferation ability in vitro and in vivo, which is due to cell apoptosis induced by microtubule stabilization at the end of microtubule through KIF18A and the inactivation of PI3K-Akt signal transduction pathway. In addition, KIF18A is up-regulated at transcriptional and translational levels in lung adenocarcinoma, and its abnormal expression is related to a clinical pathological malignancy degree. KIF18 gene mutation can be observed in lung adenocarcinoma, its expression is also regulated by a DNA copy number, and knockout of KIF18A can inhibit the proliferation of lung adenocarcinoma cells in vivo and in vitro, thus inducing apoptosis and G2/M phase arrest. Genes highly co-expressed with KIF18A are predominantly enriched in cell cycle signaling pathways, so that it is of great clinical significance to further study an action mechanism of KIF18A in tumors.

There is no new drug on the market for inhibitors targeting KIF18A, and at present, only AMG-650 from Amgen Company of the United States has entered Phase I clinical trials. As a relatively advanced research direction, studies on KIF18A as a therapeutic target hold significant exploratory potential, warranting continued efforts to elucidate its mechanisms of action and develop new inhibitors..

### SUMMARY

Aiming at the above technical problems, the present invention provides an aromatic amide derivative as shown in general formula (I), or a stereoisomer thereof, a tautomer thereof or a pharmaceutically acceptable salt thereof:

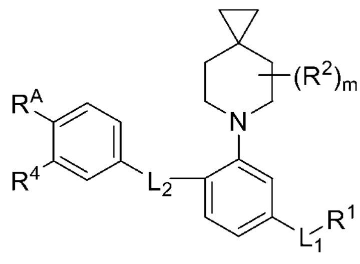

**(I)**

wherein:

$R^A$ is selected from $-OR^B$, $-NR^BR^C$, $-S(=O)_rR^B$, $-C(=O)R^5$, $-C(=O)OR^5$, $-NHC(=O)R^5$, $-NHC(=O)OR^5$, $-C(=O)NR^6R^7$, $-CH_2OR^5$, $-CH_2NR^6R^7$, 5-10 membered aryl, 5-10 membered heteroaryl or 6-14 membered fused ring, wherein the aryl, the heteroaryl or the fused ring is optionally further substituted by one or more $R^a$;

each $R^B$ is the same or different, and is independently selected from deuterated alkyl, 5-10 membered aryl, 5-10 membered heteroaryl or 6-14 membered fused ring, wherein the aryl, the heteroaryl or the fused ring is optionally further substituted by one or more $R^a$;

$R^C$ is selected from a hydrogen atom or alkyl;

each $R^a$ is the same or different, and is independently selected from halogen, hydroxyl, cyano, alkyl, cycloalkyl or alkoxy, wherein the alkyl, the cycloalkyl or the alkoxy is optionally further substituted by one or more substituents selected from halogen, hydroxyl, cyano, alkyl or alkoxy;

$R^1$ is selected from a hydrogen atom, cyano, halogen, alkyl, hydroxyl, cycloalkyl, heterocyclyl, aryl, heteroaryl, $-OR^5$, $-C(=O)R^5$, $-C(=O)OR^5$, $-NHC(=O)R^5$, $-NHC(=O)OR^5$, $-NR^6R^7$, $-C(=O)NR^6R^7$, $-CH_2NHC(=O)OR^5$, $-CH_2NR^6R^7$ or $-S(=O)_rR^5$, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^{8,}$ $-OC(=O)R^8$, $-NR^9R^{10}$, $-C(=O)NR^9R^{10}$, $-SO_2NR^9R^{10}$ or $-NR^9C(=O)R^{10}$;

$L_1$ is selected from a bond, $-C_{1-6}$ alkylene-, $-C_{0-4}$ alkylene-$NR^cS(=O)(=NH)$-$C_{0-4}$ alkylene-, $-C_{0-4}$ alkylene-S-$C_{0-4}$ alkylene-, $-C_{0-4}$ alkylene-S(=O)-$C_{0-4}$ alkylene-, $-C_{0-4}$ alkylene-$SO_2$-$C_{0-4}$ alkylene-, $-C_{0-4}$ alkylene-S(=O)(=NH)-$C_{0-4}$ alkylene-, $-C_{0-4}$ alkylene-$NR^cSO_2$-$C_{0-4}$ alkylene-, $-C_{0-4}$ alkylene-$SO_2NR^c$-$C_{0-4}$ alkylene-, $-C_{0-4}$ alkylene-O-$C_{0-4}$ alkylene-, $-C_{0-4}$ alkylene-$NR^c$-$C_{0-4}$ alkylene-, $-C_{0-4}$ alkylene-$NR^cSO_2NR^c$-$C_{0-4}$ alkylene-, $-C_{0-4}$ alkylene-$NR^cC(O)NR^c$-$C_{0-4}$ alkylene-, $-C_{0-4}$ alkylene-C(O)$NR^c$-$C_{0-4}$ alkylene-, $-C_{0-4}$ alkylene-$NR^cC(O)$-$C_{0-4}$ alkylene-, $-C_{0-4}$ alkylene-P-$C_{0-4}$ alkylene-, $-C_{0-4}$ alkylene-$P(=O)_2$-$C_{0-4}$ alkylene, $-C_{0-4}$ alkylene-C(=O)-$C_{0-4}$ alkylene- or $-C_{0-4}$ alkylene-C(=N(OH))-$C_{0-4}$ alkylene-, wherein the $-C_{1-6}$ alkylene- or the $-C_{0-4}$ alkylene- is optionally further substituted by one or more substituents selected from halogen, hydroxyl, cyano, cycloalkyl or alkoxy;

$R^c$ is selected from a hydrogen atom or alkyl;

each $R^2$ is the same or different, and is independently selected from halogen, hydroxyl, cyano, alkyl, cycloalkyl or alkoxy, wherein the alkyl, the cycloalkyl or the alkoxy is optionally further substituted by one or more substituents selected from halogen, hydroxyl, cyano, alkyl or alkoxy;

$L_2$ is selected from

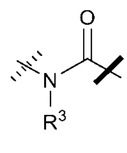

or

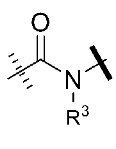

,

wherein ",,,,,," represents a linking site of the group to

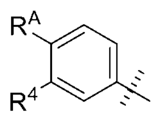

in general formula (I); and " - " represents a linking site of the group to

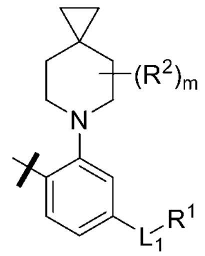

in general formula (I);

each $R^3$ is independently selected from a hydrogen atom or alkyl, wherein the alkyl is optionally further substituted by one or more substituents selected from halogen, hydroxyl, cyano or alkoxy; and $R^3$ is preferably a hydrogen atom;

$R^4$ is selected from cyano, halogen, alkyl, hydroxyl, cycloalkyl, heterocyclyl, aryl, heteroaryl, $-OR^5$, $-C(=O)R^5$, $-C(=O)OR^5$, $-NHC(=O)R^5$, $-NHC(=O)OR^5$, $-NR^6R^7$, $-C(=O)NR^6R^7$, $-CH_2NHC(=O)OR^5$, $-CH_2NR^6R^7$ or $-S(O)_rR^5$, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

each $R^5$ is independently selected from a hydrogen atom, alkyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

each of $R^6$ and $R^7$ is independently selected from a hydrogen atom, hydroxyl, halogen, alkyl, alkoxy, cycloalkyl, heterocyclyl, aryl or heteroaryl, wherein the alkyl, the alkoxy, the cycloalkyl, the heterocyclyl, the aryl or the heteroaryl is optionally further substituted by one or more substituents selected from hydroxyl, halogen, nitro, cyano, alkyl, alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

or, $R^6$ and $R^7$ form a 4-8 membered heterocyclyl together with atoms to which $R^6$ and $R^7$ are linked, wherein the 4-8 membered heterocyclyl contains one or more N, O or S(O)r, and the 4-8 membered heterocyclyl is optionally further substituted by one or more substituents selected from hydroxyl, halogen, nitro, cyano, alkyl, alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$or $-NR^9C(=O)R^{10}$;

each of $R^8$, $R^9$ and $R^{10}$ is independently selected from a hydrogen atom, alkyl, amino, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or a carboxylate group;

m is 0, 1 or 2; and

each r is independently 0, 1 or 2.

In a preferred solution of the present invention, the compound as shown in general formula (I), or a stereoisomer thereof, a tautomer thereof or a pharmaceutically acceptable salt thereof is a compound as shown in formula (II), or a stereoisomer thereof, a tautomer thereof or a pharmaceutically acceptable salt thereof,

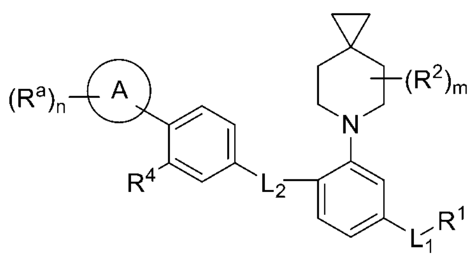

**(II)**

wherein: ring A is selected from 5-10 membered aryl, 5-10 membered heteroaryl or 6-14 membered fused ring;

each $R^a$ is the same or different, and is independently selected from halogen, hydroxyl, cyano, $C_1$-$C_3$ alkyl, cyclopropyl

or methoxy;
n is 0, 1 or 2; and
$L_1$, $L_2$, $R^1$, $R^2$, $R^4$ and m are as defined in general formula (I).

In a preferred solution of the present invention, in the compound as shown in general formula (I), or the stereoisomer thereof, the tautomer thereof or the pharmaceutically acceptable salt thereof, $R^A$ is selected from $-OCD_3$, $-C(=O)OH$, $-C(=O)NH_2$, $-C(=O)NHCH_3$, $-C(=O)N(CH_3)_2$, $-C(=O)CH_3$, $-CH_2OH$, $-CH_2OCH_3$ and

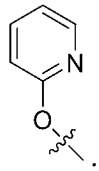.

In a preferred solution of the present invention, in the compound as shown in general formula (II), or the stereoisomer thereof, the tautomer thereof or the pharmaceutically acceptable salt thereof, n is 0 or 1.
In a preferred solution of the present invention, in the compound as shown in general formula (II), or the stereoisomer thereof, the tautomer thereof or the pharmaceutically acceptable salt thereof,

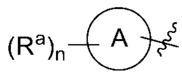

is selected from the following groups:

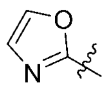, 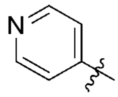,

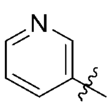, 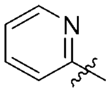, 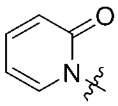, 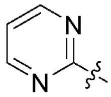, 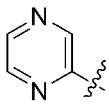, 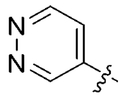, 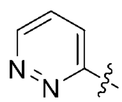, 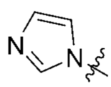, 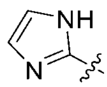,

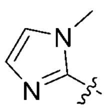, 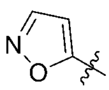, 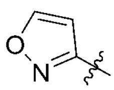, 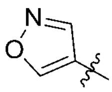, 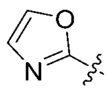, 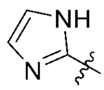, 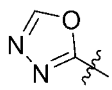, 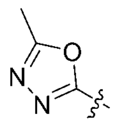, 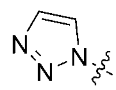,

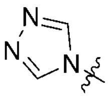, 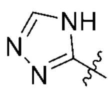, 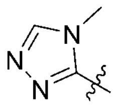, 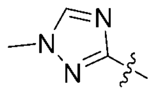, 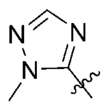, 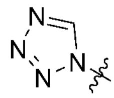, 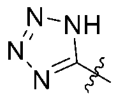, 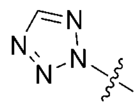,

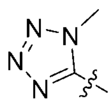, 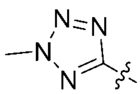 or 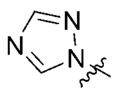.

In a preferred solution of the present invention, in the compound as shown in general formula (I) or (II), or the stereoisomer thereof, the tautomer thereof or the pharmaceutically acceptable salt thereof, m is 0.
In a preferred solution of the present invention, in the compound as shown in general formula (I) or (II), or the stereoisomer thereof, the tautomer thereof or the pharmaceutically acceptable salt thereof,

$L_1$ is selected from a bond, $-NR^c-C_{0-4}$ alkylene-, $-NR^cSO_2-C_{0-4}$ alkylene-, $-SO_2NR^c-C_{0-4}$ alkylene-, $-NR^cSO_2NR^c-$, -S(=O)(=NH)-, $-NR^cS(=O)(=NH)-$, $-C_{1-4}$ alkylene-, -S(=O)-, -O-, -C(=O)-, $-C(=O)NR^c-C_{0-4}$ alkylene-, $-C_{0-4}$ alkylene-$SO_2-C_{0-4}$ alkylene-, -C=N(OH)- or $-NR^c-C(=O)-$, wherein the $-C_{0-4}$ alkylene- or the $-C_{1-4}$ alkylene- is optionally further

substituted by one or more substituents selected from halogen, hydroxyl, cyano, cyclopropyl or methoxy; and each $R^c$ is independently selected from a hydrogen atom or methyl.

In a preferred solution of the present invention, in the compound as shown in general formula (I) or (II), or the stereoisomer thereof, the tautomer thereof or the pharmaceutically acceptable salt thereof, $L_1$ is selected from a bond, $-NHSO_2CH_2CH_2-$, $-SO_2NHCH_2CH_2-$, $-SO_2-$, $-CH_2SO_2-$, $-NHSO_2-$, $-SO_2NH-$, $-NHC(CH_3)_2CH_2-$, $-C(=O)NHCH_2CH_2-$, $-C(=O)NHC(CH_3)_2CH_2-$, $-C(=O)N(CH_3)CH_2CH_2-$, $-CH(CH_3)(OH)CH_2-$, $-NHSO_2CH(CH_3)CH_2-$, $-SO_2NHC(CH_3)_2CH_2-$, $-C(=O)NH-$, $-NHCH_2CH_2$ or $-CH_2SO_2CH_2CH_2-$.

In a preferred solution of the present invention, in the compound as shown in general formula (I) or (II), or the stereoisomer thereof, the tautomer thereof or the pharmaceutically acceptable salt thereof, $R^1$ is selected from a hydrogen atom, hydroxyl, alkyl, heterocyclyl, cycloalkyl or heteroaryl, wherein the alkyl, the heterocyclyl, the cycloalkyl or the heteroaryl is optionally further substituted by one or more substituents selected from hydroxyl or alkyl.

In a preferred solution of the present invention, in the compound as shown in general formula (I) or (II), or the stereoisomer thereof, the tautomer thereof or the pharmaceutically acceptable salt thereof,

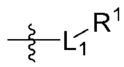

is

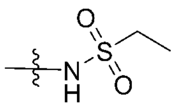
,
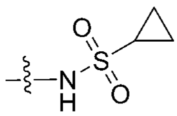
or
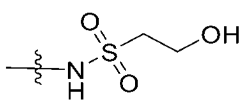
.

In a preferred solution of the present invention, in the compound as shown in general formula (I) or (II), or the stereoisomer thereof, the tautomer thereof or the pharmaceutically acceptable salt thereof, $L_2$ is selected from

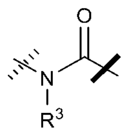
,

wherein "ıııııı" represents a linking site of the group to

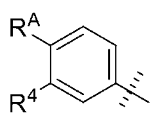

in general formula (I); and " - " represents a linking site of the group to

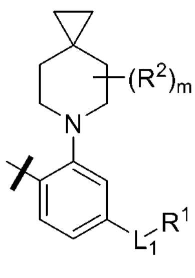

in general formula (I); and
$R^3$ is a hydrogen atom.

In a preferred solution of the present invention, in the compound as shown in general formula (I) or (II), or the stereoisomer thereof, the tautomer thereof or the pharmaceutically acceptable salt thereof, $R^4$ is selected from $-OR^b$, $-NHR^b$, 3-6 membered cycloalkyl or 3-6 membered heterocyclyl, wherein the cycloalkyl or the heterocyclyl is optionally further substituted by one or more substituents selected from halogen, alkyl and hydroxyl; and

$R^b$ is selected from alkyl, 3-6 membered cycloalkyl or 3-6 membered heterocyclyl, wherein the alkyl, the cycloalkyl or the heterocyclyl is optionally further substituted by one or more halogens.

In a preferred solution of the present invention, in the compound as shown in general formula (I) or (II), or the stereoisomer thereof, the tautomer thereof or the pharmaceutically acceptable salt thereof, $R^4$ is halogen, methyl, difluoromethyl, trifluoromethyl, methoxy, trifluoroethoxy,

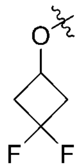 , 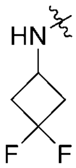 , 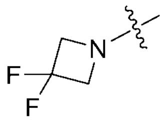 , 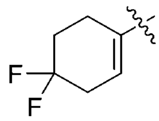 , 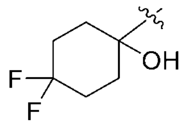 , 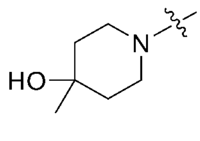 ,

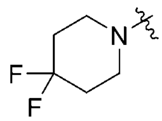 or 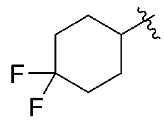 .

In a preferred solution of the present invention, the compound as shown in the general formula is selected from:

| **Serial number of compounds** | **Structure** | **Name** |
|---|---|---|
| Example 1 | 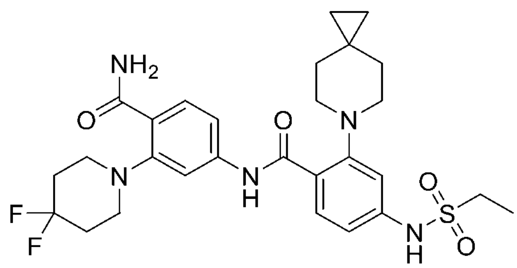 | N-(4-carbamoyl-3-(4,4-difluor opiperidin-1-yl)phenyl)-4-(eth ylsulfonamido)-2-(6-azaspiro[ 2.5]octan-6-yl)ben-zamide |
| Example 2 | 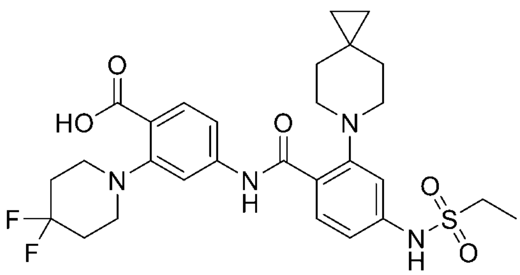 | 2-(4,4-difluoropiperidin-1-yl)-4-(4-(ethylsulfonamido)-2-(6-a zaspiro[2.5]octan-6-yl)benzam ido)benzoic acid |
| Example 3 | 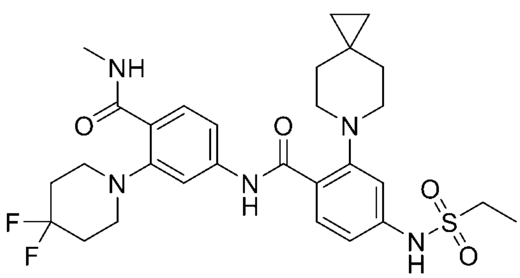 | 2-(4,4-difluoropiperidin-1-yl)-4-(4-(ethylsulfonamido)-2-(6-a zaspiro[2.5]octan-6-yl)benzam ido)-N-methylbenzamide |

(continued)

| Serial number of compounds | Structure | Name |
|---|---|---|
| Example 4 | 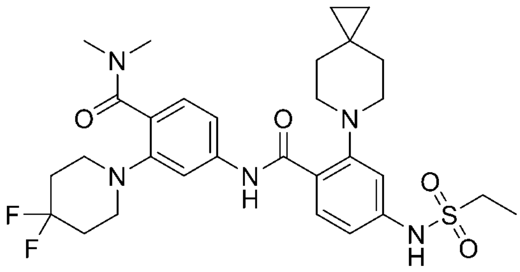 | 2-(4,4-difluoropiperidin-1-yl)-4-(4-(ethylsulfonamido)-2-(6-a zaspiro[2.5]octan-6-yl)benzam ido)-N,N-dimethylbenzamide |
| Example 5 | 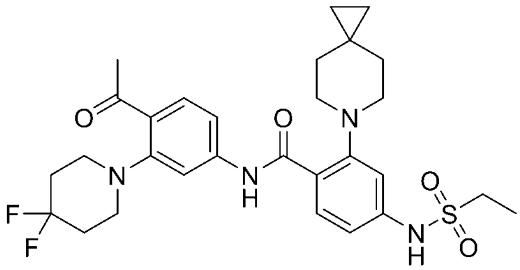 | N-(4-acetyl-3-(4,4-difluoropip eridin-1-yl)phenyl)-4-(ethylsul fonamido)-2-(6-azaspiro[2.5]o ctan-6-yl)benzamide |
| Example 6 | 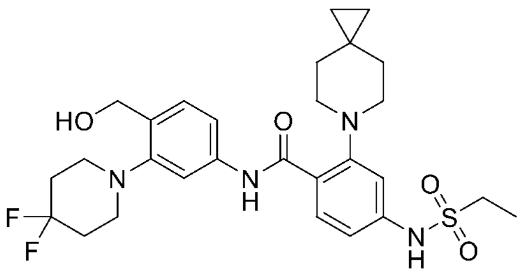 | N-(3-(4,4-difluoropiperidin-1-yl)-4-(hydroxymethyl)phenyl)-4-(ethylsulfonamido)-2-(6-aza spiro[2.5]octan-6-yl)benzamid e |
| Example 7 | 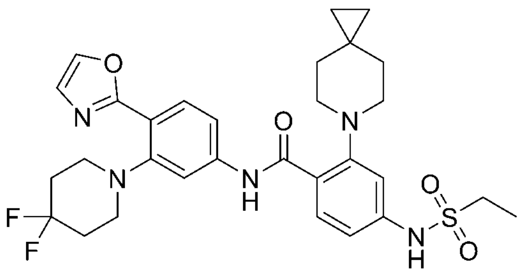 | N-(3-(4,4-difluoropiperidin-1-yl)-4-(oxazol-2-yl)phenyl)-4-( ethylsulfonamido)-2-(6-azaspi ro[2.5]octan-6-yl)benzamide |
| Example 8 | 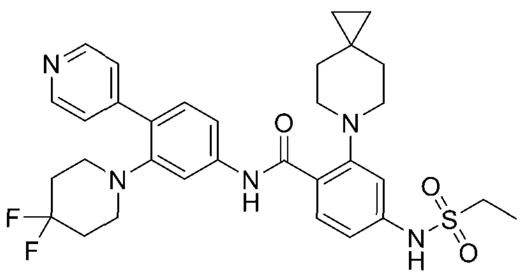 | N-(3-(4,4-difluoropiperidin-1-yl)-4-(pyridin-4-yl)phenyl)-4-( ethylsulfonamido)-2-(6-azaspi ro[2.5]octan-6-yl)benzamide |

(continued)

| Serial number of compounds | Structure | Name |
|---|---|---|
| Example 9 | 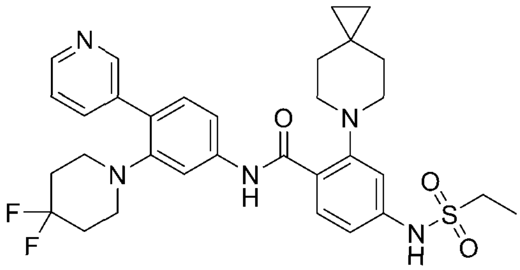 | N-(3-(4,4-difluoropiperidin-1-yl)-4-(pyridin-3-yl)phenyl)-4-( ethylsulfonamido)-2-(6-azaspi ro[2.5]octan-6-yl)benzamide |
| Example 10 | 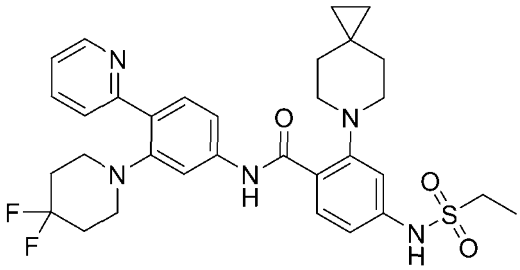 | N-(3-(4,4-difluoropiperidin-1-yl)-4-(pyridin-2-yl)phenyl)-4-( ethylsulfonamido)-2-(6-azaspi ro[2.5]octan-6-yl)benzamide |
| Example 11 | 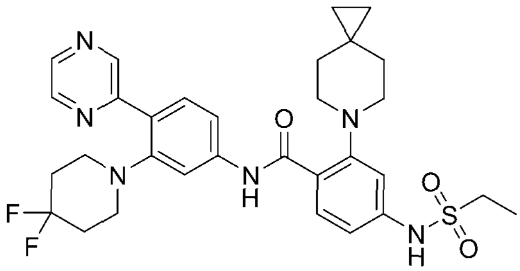 | N-(3-(4,4-difluoropiperidin-1-yl)-4-(pyrazin-2-yl)phenyl)-4-( ethylsulfonamido)-2-(6-azaspi ro[2.5]octan-6-yl)benzamide |
| Example 12 | 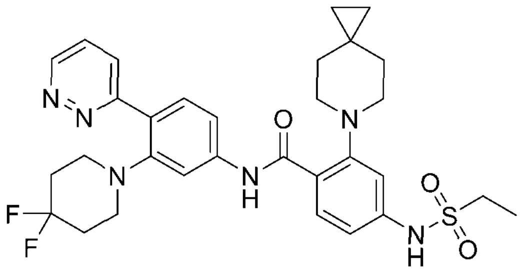 | N-(3-(4,4-difluoropiperidin-1-yl)-4-(pyridazin-3-yl)phenyl)-4-(ethylsulfonamido)-2-(6-aza spiro[2.5]octan-6-yl)benzamid e |
| Example 13 | 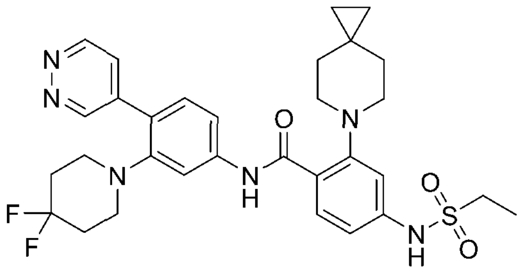 | N-(3-(4,4-difluoropiperidin-1-yl)-4-(pyridazin-4-yl)phenyl)-4-(ethylsulfonamido)-2-(6-aza spiro[2.5]octan-6-yl)benzamid e |

(continued)

| Serial number of compounds | Structure | Name |
|---|---|---|
| Example 14 | 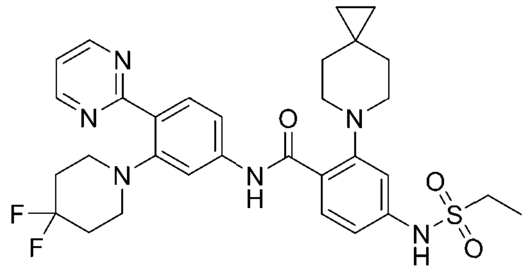 | N-(3-(4,4-difluoropiperidin-1-yl)-4-(pyrimidin-2-yl)phenyl)-4-(ethylsulfonamido)-2-(6-aza spiro[2.5]octan-6-yl)benzamid e |
| Example 15 | 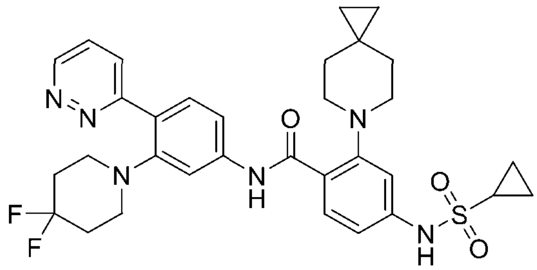 | 4-(cyclopropanesulfonamido)-N-(3-(4,4-difluoropiperidin-1-yl)-4-(pyridazin-3-yl)phenyl)-2-(6-azaspiro[2.5]octan-6-yl)b enzamide |
| Example 16 | 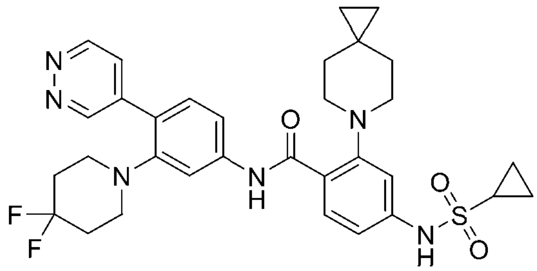 | 4-(cyclopropanesulfonamido)-N-(3-(4,4-difluoropiperidin-1-yl)-4-(pyridazin-4-yl)phenyl)-2-(6-azaspiro[2.5]octan-6-yl)b enzamide |
| Example 17 | 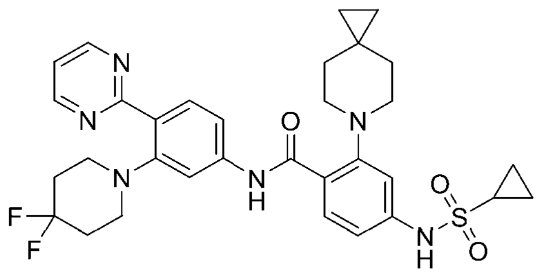 | 4-(cyclopropanesulfonamido)-N-(3-(4,4-difluoropiperidin-1-yl)-4-(pyrimidin-2-yl)phenyl)-2-(6-azaspiro[2.5]octan-6-yl)b enzamide |
| Example 18 | 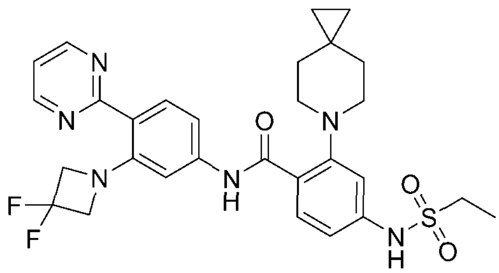 | N-(3-(3,3-difluoroazetidin-1-y 1)-4-(pyrimidin-2-yl)phenyl)-4 -(ethylsulfonamido)-2-(6-azas piro[2.5]octan-6-yl)benzamide |

(continued)

| Serial number of compounds | Structure | Name |
|---|---|---|
| Example 19 | 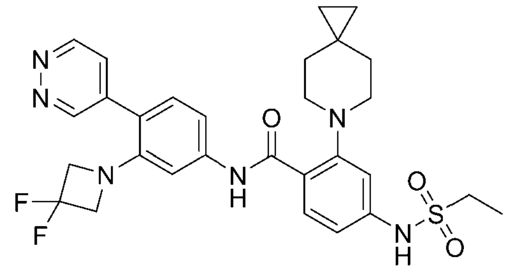 | N-(3-(3,3-difluoroazetidin-1-y 1)-4-(pyridazin-4-yl)phenyl)-4-(ethylsulfonamido)-2-(6-azasp iro[2.5]octan-6-yl)benzamide |
| Example 20 | 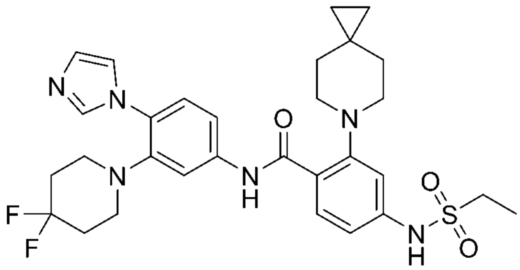 | N-(3-(4,4-difluoropiperidin-1-yl)-4-(1H-imidazol-1-yl)pheny 1)-4-(ethylsulfonamido)-2-(6-a zaspiro[2.5]octan-6-yl) benzam ide |
| Example 21 | 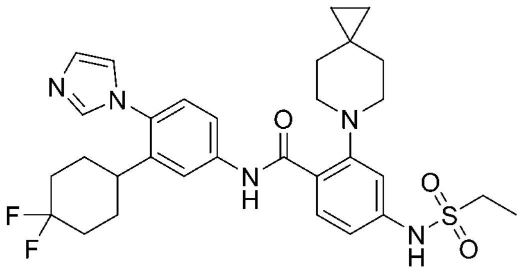 | N-(3-(4,4-difluorocyclohexyl)-4-(1H-imidazol-1-yl)phenyl)-4 -(ethylsulfonamido)-2-(6-azas piro[2.5]octan-6-yl)benzamide |
| Example 22 | 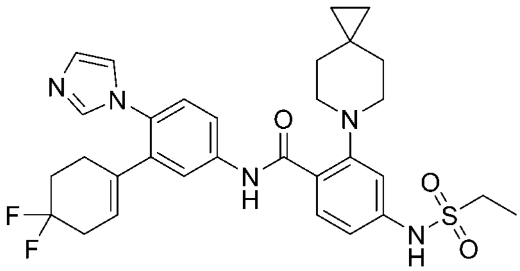 | N-(4',4'-difluoro-6-(1H-imida zol-1-yl)-2',3',4',5'-tetrahydro -[1,1'-biphenyl]-3-yl)-4-(ethyl sulfonamido)-2-(6-azaspiro[2. 5]octan-6-yl)benzamide |
| Example 23 | 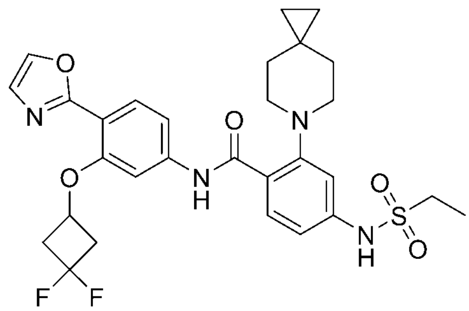 | N-(3-(3,3-difluorocyclobutoxy )-4-(oxazol-2-yl)phenyl)-4-(et hylsulfonamido)-2-(6-azaspiro [2.5]octan-6-yl)benzamide |

(continued)

| Serial number of compounds | Structure | Name |
|---|---|---|
| Example 24 | 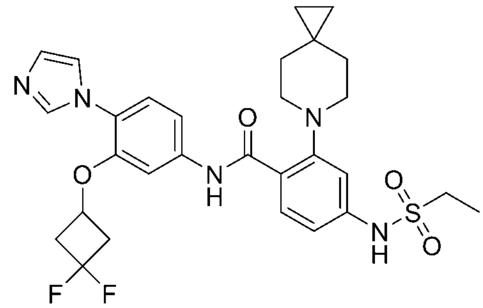 | N-(3-(3,3-difluorocyclobutoxy )-4-(1H-imidazol-1-yl)phenyl) -4-(ethylsulfonamido)-2-(6-az aspiro[2.5]octan-6-yl) benzami de |
| Example 25 | 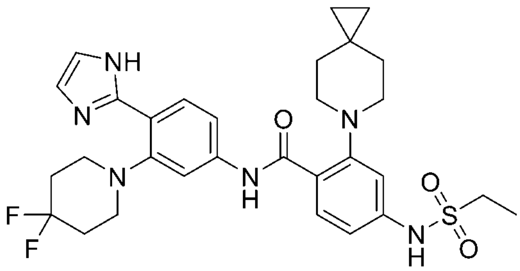 | N-(3-(4,4-difluoropiperidin-1-yl)-4-(1H-imidazol-2-yl)pheny 1)-4-(ethylsulfonamido)-2-(6-a zaspiro[2.5]octan-6-yl) benzam ide |
| Example 26 | 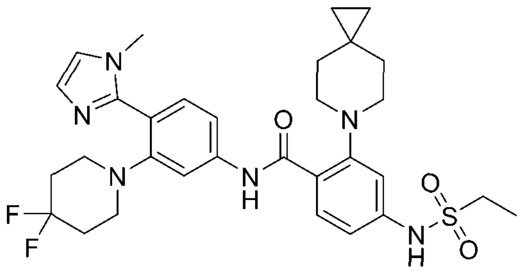 | N-(3-(4,4-difluoropiperidin-1-yl)-4-(1-methyl-1H-imidazol-2 -yl)phenyl)-4-(ethylsulfonami do)-2-(6-azaspiro [2.5]octan-6-yl)benzamide |
| Example 27 | 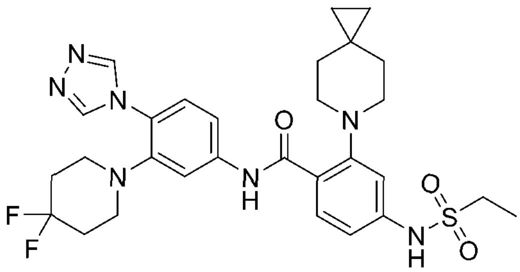 | N-(3-(4,4-difluoropiperidin-1-yl)-4-(4H-1,2,4-triazol-4-yl)ph enyl)-4-(ethylsulfonamido)-2-( 6-azaspiro [2.5]octan-6-yl)benz amide |
| Example 28 | 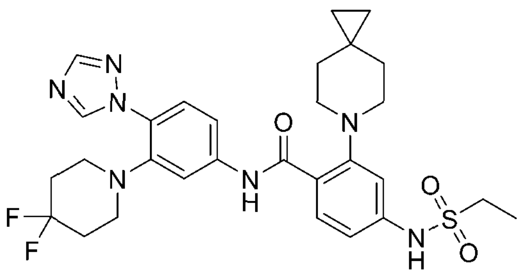 | N-(3-(4,4-difluoropiperidin-1-yl)-4-(1H-1,2,4-triazol-1-yl)ph enyl)-4-(ethylsulfonamido)-2-( 6-azaspiro [2.5]octan-6-yl)benz amide |

(continued)

| Serial number of compounds | Structure | Name |
|---|---|---|
| Example 29 | 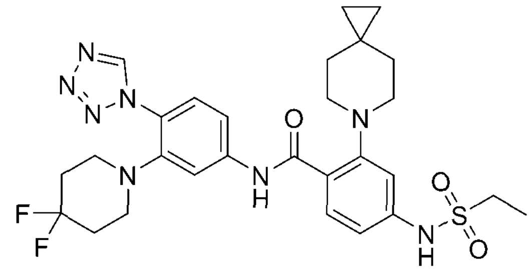 | N-(3-(4,4-difluoropiperidin-1-yl)-4-(1H-tetrazol-1-yl)phenyl )-4-(ethylsulfonamido)-2-(6-az aspiro[2.5]octan-6-yl)benzami de |
| Example 30 | 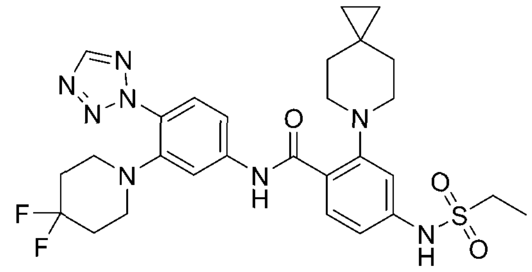 | N-(3-(4,4-difluoropiperidin-1-yl)-4-(2H-tetrazol-2-yl)phenyl )-4-(ethylsulfonamido)-2-(6-az aspiro[2.5]octan-6-yl)benzami de |
| Example 31 | 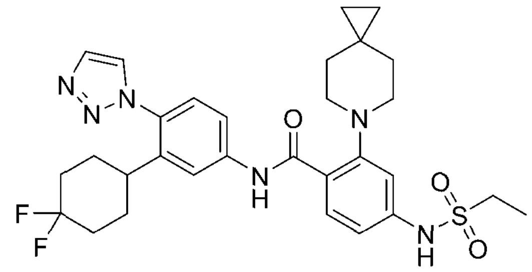 | N-(3-(4,4-difluorocyclohexyl)-4-(1H-1,2,3-triazol-1-yl)pheny 1)-4-(ethylsulfonamido)-2-(6-a zaspiro [2.5]octan-6-yl)benzam ide |
| Example 32 | 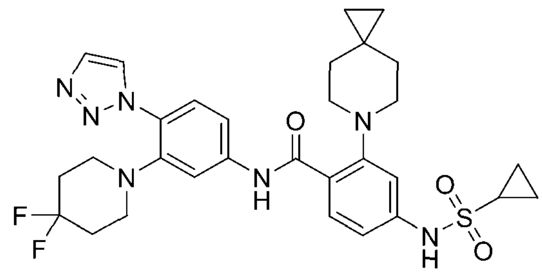 | 4-(cyclopropanesulfonamido)-N-(3-(4,4-difluoropiperidin-1-yl)-4-(1H-1,2,3-triazol-1-yl)ph enyl)-2-(6-azaspiro[2.5]octan-6-yl)benzamide |
| Example 33 | 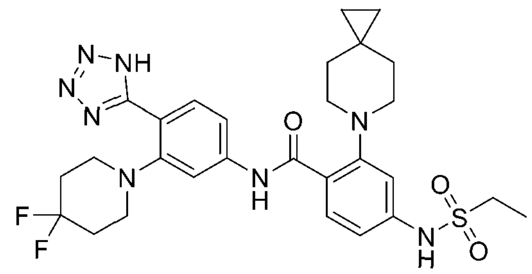 | N-(3-(4,4-difluoropiperidin-1-yl)-4-(1H-tetrazol-5-yl)phenyl )-4-(ethylsulfonamido)-2-(6-az aspiro[2.5]octan-6-yl)benzami de |

(continued)

| Serial number of compounds | Structure | Name |
|---|---|---|
| Example 34 | 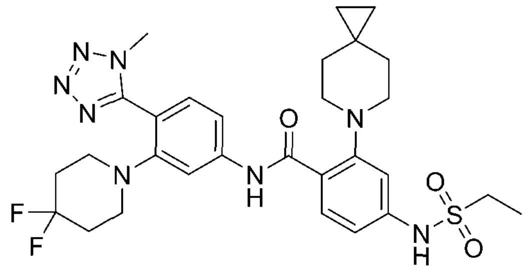 | N-(3-(4,4-difluoropiperidin-1-yl)-4-(1-methyl-1H-tetrazol-5-yl)phenyl)-4-(ethylsulfonamid o)-2-(6-azaspiro [2.5]octan-6-y l)benzamide |
| Example 35 | 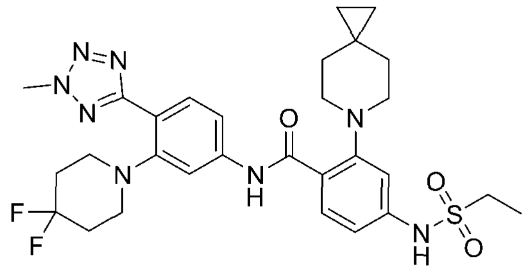 | N-(3-(4,4-difluoropiperidin-1-yl)-4-(2-methyl-2H-tetrazol-5-yl)phenyl)-4-(ethylsulfonamid o)-2-(6-azaspiro [2.5]octan-6-y 1)benzamide |
| Example 36 | 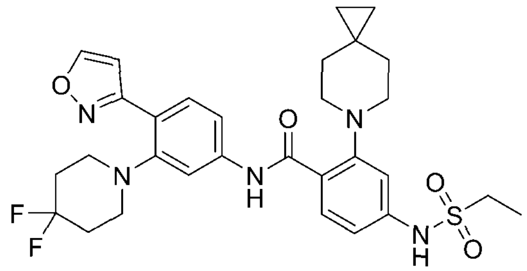 | N-(3-(4,4-difluoropiperidin-1-yl)-4-(isoxazol-3-yl)phenyl)-4-(ethylsulfonamido)-2-(6-azasp iro[2.5]octan-6-yl)benzamide |
| Example 37 | 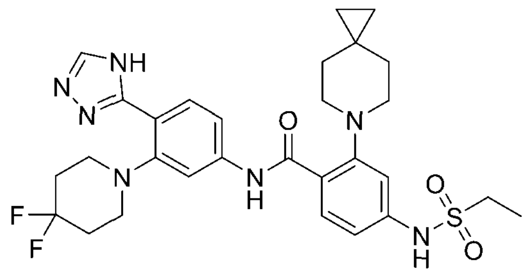 | N-(3-(4,4-difluoropiperidin-1-yl)-4-(4H-1,2,4-triazol-3-yl)ph enyl)-4-(ethylsulfonamido)-2-( 6-azaspiro [2.5]octan-6-yl)benz amide |
| Example 38 | 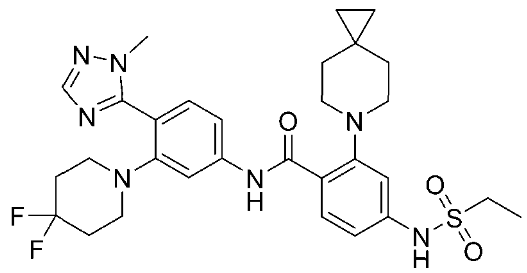 | N-(3-(4,4-difluoropiperidin-1-yl)-4-(1-methyl-1H-1,2,4-triaz ol-5-yl)phenyl)-4-(ethylsulfon amido)-2-(6-azaspiro [2.5]octa n-6-yl)benzamide |

(continued)

| Serial number of compounds | Structure | Name |
|---|---|---|
| Example 39 | 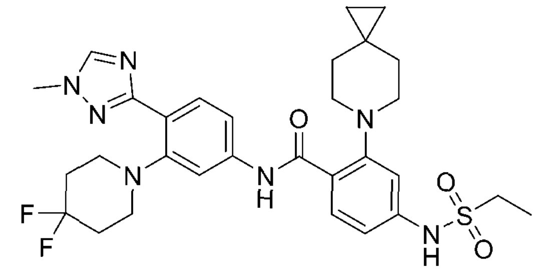 | N-(3-(4,4-difluoropiperidin-1-yl)-4-(1-methyl-1H-1,2,4-triaz ol-3-yl)phenyl)-4-(ethylsulfon amido)-2-(6-azaspiro [2.5]octa n-6-yl)benzamide |
| Example 40 | 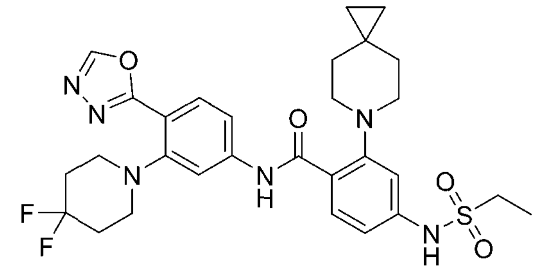 | N-(3-(4,4-difluoropiperidin-1-yl)-4-(1,3,4-oxadiazol-2-yl)ph enyl)-4-(ethylsulfonamido)-2-( 6-azaspiro [2.5]octan-6-yl)benz amide |
| Example 41 | 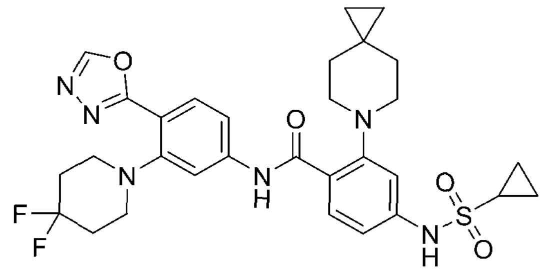 | 4-(cyclopropanesulfonami-do)-N-(3-(4,4-difluoropiperidin-1-yl)-4-(1,3,4-oxadiazol-2-yl)ph enyl)-2-(6-azaspiro[2.5]octan-6-yl)ben-zamide |
| Example 42 | 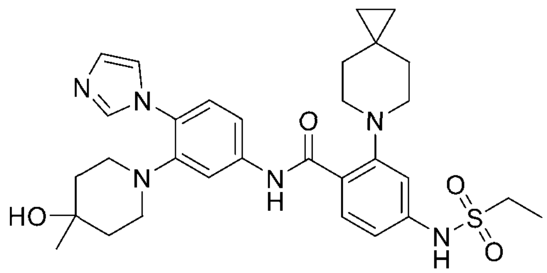 | 4-(ethylsulfonamido)-N-(3-(4-hydroxy-4-methylpiperidin-1-yl)-4-(1H-imidazol-1-yl)pheny 1)-2-(6-azaspiro[2.5]octan-6-yl )benzamide |
| Example 43 | 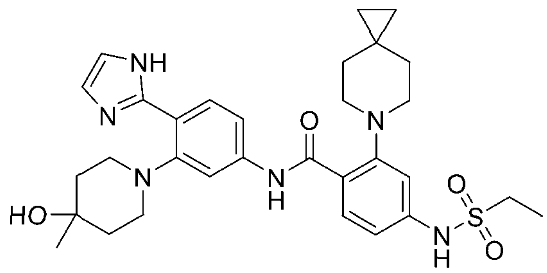 | 4-(ethylsulfonamido)-N-(3-(4-hydroxy-4-methylpiperidin-1-yl)-4-(1H-imidazol-2-yl)pheny 1)-2-(6-azaspiro[2.5]oc-tan-6-yl )benzamide |

(continued)

| Serial number of compounds | Structure | Name |
|---|---|---|
| Example 44 | 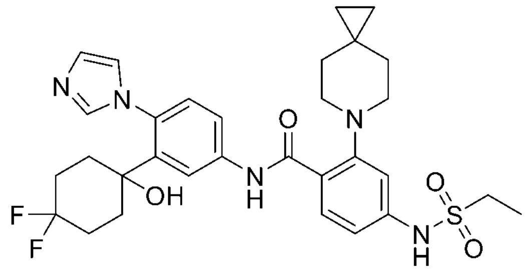 | N-(3-(4,4-difluoro-1-hydroxyc yclohexyl)-4-(1H-imidazol-1-yl)phenyl)-4-(ethylsulfonamid o)-2-(6-azaspiro [2.5]octan-6-y l)benzamide |
| Example 45 | 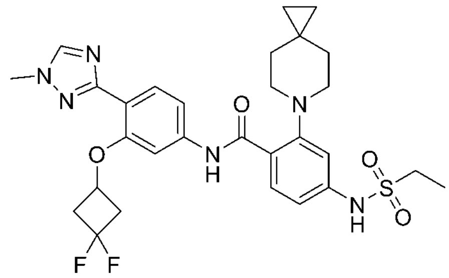 | N-(3-(3,3-difluorocyclobutoxy )-4-(1-methyl-1H-1,2,4-triazol -3-yl)phenyl)-4-(ethylsulfona mido)-2-(6-azaspiro [2.5]octan -6-yl)benzamide |
| Example 46 | 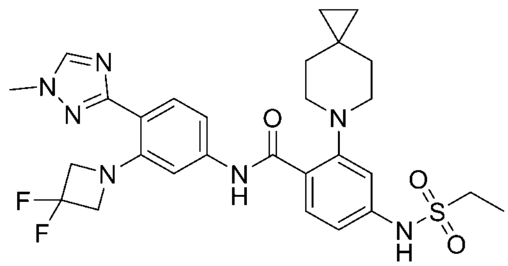 | N-(3-(3,3-difluoroazetidin-1-y 1)-4-(1-methyl-1H-l,2,4-triazo 1-3-yl)phenyl)-4-(ethylsulfona mido)-2-(6-azaspiro [2.5]octan -6-yl)benzamide |
| Example 47 | 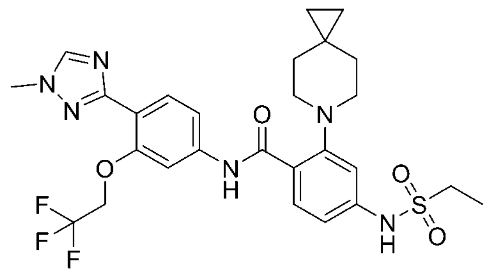 | 4-(ethylsulfonamido)-N-(4-(1-methyl-1H-1,2,4-triazol-3-yl)-3-(2,2,2-trifluoroethoxy)pheny 1)-2-(6-azaspiro [2.5]octan-6-yl )benzamide |
| Example 48 | 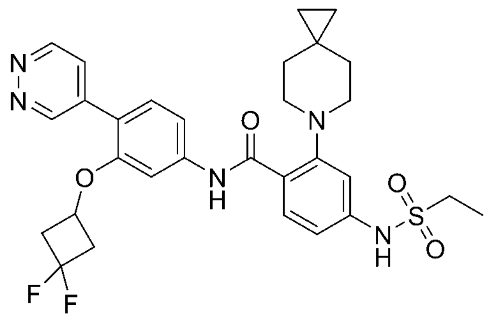 | N-(3-(3,3-difluorocyclobutoxy )-4-(pyridazin-4-yl)phenyl)-4-( ethylsulfonamido)-2-(6-azaspi ro[2.5]octan-6-yl)ben-zamide |

(continued)

| Serial number of compounds | Structure | Name |
|---|---|---|
| Example 49 | 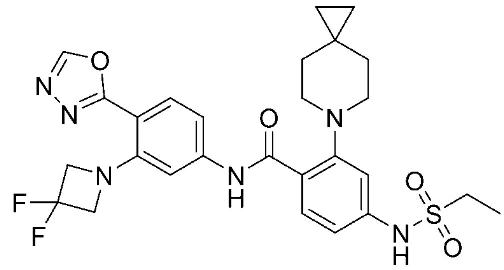 | N-(3-(3,3-difluoroazetidin-1-y 1)-4-(1,3,4-oxadiazol-2-yl)phe nyl)-4-(ethylsulfonamido)-2-(6 -azaspiro [2.5]octan-6-yl)benza mide |
| Example 50 | 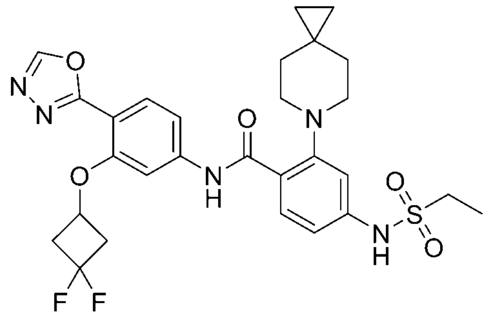 | N-(3-(3,3-difluorocyclobutoxy)-4-(1,3,4-oxadiazol-2-yl)phen yl)-4-(ethylsulfonamido)-2-(6-azaspiro[2.5]octan-6-yl) benza mide |
| Example 51 | 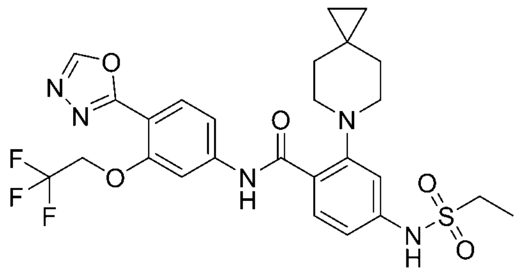 | N-(4-(1,3,4-oxadiazol-2-yl)-3-(2,2,2-tri-fluoroethoxy)phenyl)-4-(ethylsulfonami-do)-2-(6-aza spiro[2.5]octan-6-yl)ben-zamid e |
| Example 52 | 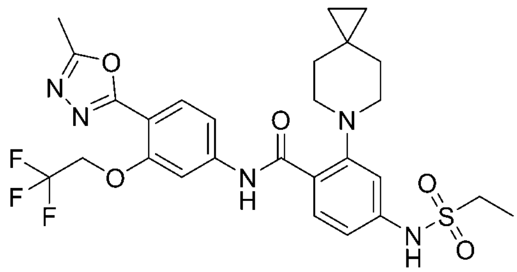 | 4-(ethylsulfonamido)-N-(4-(5-methyl-1,3,4-oxadiazol-2-yl)-3 -(2,2,2-trifluoroethoxy)phenyl) -2-(6-azaspiro [2.5]octan-6-yl) benzamide |
| Example 53 | 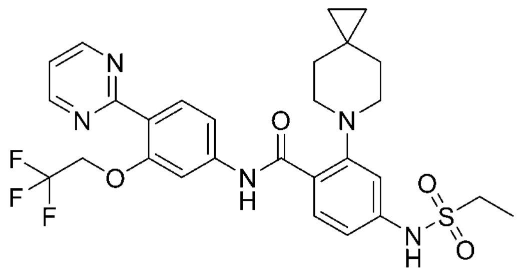 | 4-(ethylsulfonamido)-N-(4-(py rimidin-2-yl)-3-(2,2,2-trifluoro ethoxy)phenyl)-2-(6-azaspiro[ 2.5]octan-6-yl)ben-zamide |

(continued)

| Serial number of compounds | Structure | Name |
|---|---|---|
| Example 54 | 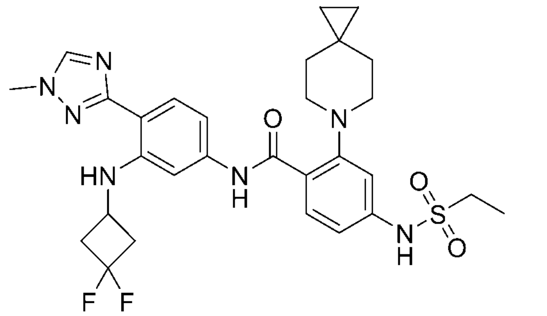 | N-(3-((3,3-difluorocyclobutyl) amino)-4-(1-methyl-1H-1,2,4-t riazol-3-yl) phenyl)-4-(ethylsul fonamido)-2-(6-azaspiro[2.5]o ctan-6-yl)benzamide |
| Example 55 | 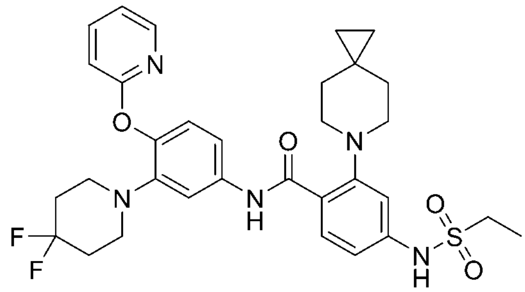 | N-(3-(4,4-difluoropiperidin-1-yl)-4-(pyridin-2-yloxy)phenyl) -4-(ethylsulfonamido)-2-(6-az aspiro[2.5]octan-6-yl)benzami de |
| Example 56 | 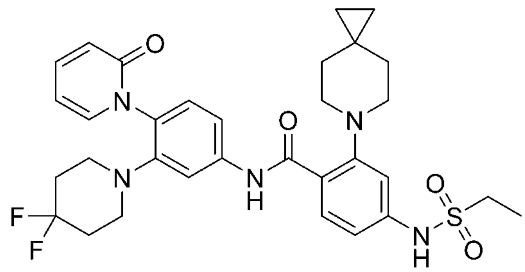 | N-(3-(4,4-difluoropiperidin-1-yl)-4-(2-oxopyridin-1 (2H)-yl) phenyl)-4-(ethylsulfonamido)-2-(6-azaspiro[2.5]octan-6-yl)b enzamide |
| Example 57 | 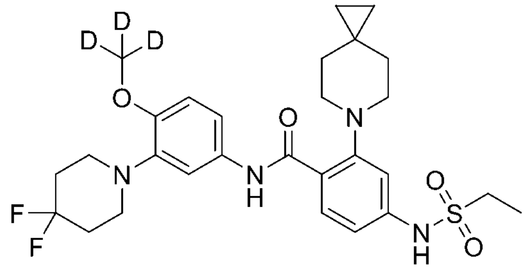 | N-(3-(4,4-difluoropiperidin-1-yl)-4-(methoxy-d3)phenyl)-4-( ethylsulfonamido)-2-(6-azaspi ro[2.5]octan-6-yl)benzamide |
| Example 58 | 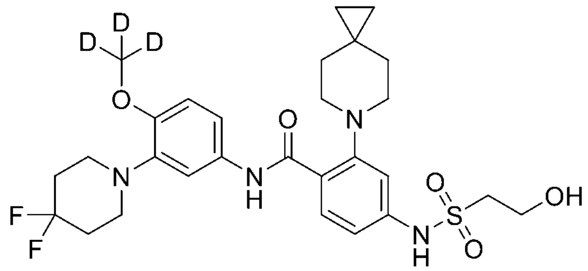 | N-(3-(4,4-difluoropiperidin-1-yl)-4-(methoxy-d3)phenyl)-4-( (2-hydroxyethyl)sulfonamido) -2-(6-azaspiro [2.5]octan-6-yl) benzamide |

or a stereoisomer thereof, a tautomer thereof or a pharmaceutically acceptable salt thereof. Note: if there is a difference between the drawn structure and the given name of the structure, the drawn structure will be given greater weight.

Further, the present invention provides a pharmaceutical composition, wherein the pharmaceutical composition contains an effective amount of the compound as shown in general formula (I) or (II), or the stereoisomer thereof, the tautomer thereof or the pharmaceutically acceptable salt thereof, and a pharmaceutically acceptable carrier, an excipient or a combination of the pharmaceutically acceptable carrier and the excipient.

The present invention provides use of the compound as shown in general formula (I) or (II), or the stereoisomer thereof, the tautomer thereof or the pharmaceutically acceptable salt thereof, or the pharmaceutical composition thereof in the preparation of a KIF18A inhibitor.

The present invention further provides use of the compound as shown in general formula (I) or (II), or the stereoisomer thereof, the tautomer thereof or the pharmaceutically acceptable salt thereof, or the pharmaceutical composition thereof in the preparation of a medicament for treating a KIF18A-mediated disease, wherein the KIF18A-mediated disease is preferably cancer; wherein the KIF18A-mediated disease is selected from hepatocellular carcinoma, glioblastoma, colon cancer, breast cancer, lung cancer, cholangiocarcinoma, pancreatic cancer, prostate cancer, bladder cancer, head cancer, neck cancer, cervical cancer, ovarian cancer, synovial sarcoma, rhabdomyosarcoma, colorectal cancer and lung adenocarcinoma.

The present invention further provides use of the compound as shown in general formula (I) or (II), or the stereoisomer thereof, the tautomer thereof or the pharmaceutically acceptable salt thereof, or the pharmaceutical composition thereof in the preparation of a medicament for treating cancer.

The present invention provides use of the compound as shown in general formula (I) or (II), or the stereoisomer thereof, the tautomer thereof or the pharmaceutically acceptable salt thereof, or the pharmaceutical composition thereof in the preparation of a medicament for treating hepatocellular carcinoma, glioblastoma, colon cancer, breast cancer, lung cancer, cholangiocarcinoma, pancreatic cancer, prostate cancer, bladder cancer, head cancer, neck cancer, cervical cancer, ovarian cancer, synovial sarcoma, rhabdomyosarcoma, colorectal cancer and lung adenocarcinoma.

Detailed description of the invention

Unless stated to the contrary, some terms used in the specification and the claims of the present invention are defined as follows.

"Alkyl" refers to an aliphatic hydrocarbon group comprising a $C_1$-$C_{20}$ straight chain or branched chain when taken as one group or a part of one group. Preferably, the alkyl is $C_1$-$C_{10}$ alkyl, and more preferably, the alkyl is $C_1$-$C_6$ alkyl. Embodiments of an alkyl group comprise, but are not limited to, methyl, ethyl, n-propyl, isopropyl, n-butyl, isobutyl, tert-butyl, sec-butyl, n-pentyl, 1,1-dimethyl propyl, 1,2-dimethyl propyl, 2,2-dimethyl propyl, 1-ethyl propyl, 2-methyl butyl, 3-methyl butyl, n-hexyl, 1-ethyl-2-methyl propyl, 1,1,2-trimethyl propyl, 1,1-dimethyl butyl, 1,2-dimethyl butyl, 2,2-dimethyl butyl, 1,3-dimethyl butyl, 2-ethyl butyl, 2-methyl pentyl, 3-methyl pentyl, 4-methyl pentyl, 2,3-dimethyl butyl, and the like. The alkyl may be substituted or unsubstituted.

"$C_{\alpha-\beta}$ alkylene" refers to an aliphatic hydrocarbon group comprising minimum $\alpha$ and maximum $\beta$ carbon atoms in a branched or linear relationship, which has 2 residues derived from removing two hydrogen atoms from the same carbon atom or two different carbon atoms of parent alkane, wherein $\alpha$ and $\beta$ represent integers, an index in $C_0$ alkylene represents a straight chain, and examples of $C_{1-6}$ alkylene comprise, but are not limited to, methylene, 1,1-ethylene, 1,2-ethylene, 1,1-propylene, 1,2-propylene, 1,3-propylene, 1,4-butylene, and the like. $C_{\alpha-\beta}$ alkylene may be substituted or unsubstituted.

"Cycloalkyl" refers to non-aromatic cyclic alkyl, wherein one or more ring-forming atoms are carbon atoms, comprising a monocyclic ring, a polycyclic ring, a fused ring, a bridged ring and a spirocyclic ring, and preferably having a 5-7 membered monocyclic ring or a 7-10 membered bicyclic or tricyclic ring, and the ring contains 0, 1 or more double bonds, but no ring has a fully conjugated $\pi$ electron aromatic system. Examples of the "cycloalkyl" comprise, but are not limited to, cyclopropyl, cyclopentyl, cyclobutyl and

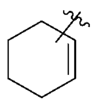
.

The cycloalkyl may be substituted or unsubstituted.

"Spiroalkyl" refers to a polycyclic group with 5 to 18 atoms, two or more cyclic structures, and single rings sharing one carbon atom (called a spiro atom) with each other, and the ring contains 0, 1 or more double bonds, but no ring has a fully conjugated $\pi$ electron aromatic system. Preferably, the spiroalkyl is a 6 to 14 memebered, and more preferably, the spiroalkyl is a 7 to 10 memebered. The spiroalkyl is divided into mono-, di- or multi-spiroalkyl according to the number of shared spiro-atoms between rings, is preferably the mono- and di-spiroalkyl, and preferably is a 4 memebered/5 membered, 4 membered/6 membered, 5 membered/5 membered or 5 membered/6 memebered. Non-limiting embodiments of the "spiroalkyl" comprise, but are not limited to, spiro[4.5]decyl, spiro[4.4]nonyl, spiro[3.5]nonyl and spiro[2.4]heptyl.

"Fused cycloalkyl" refers to a full-carbon polycyclic group with 5 to 18 atoms, and two or more cyclic structures sharing one pair of carbon atoms with each other, and one or more rings may contain 0, 1 or more double bonds, but no ring has a fully conjugated $\pi$ electron aromatic system. Preferably, the fused cycloalkyl is a 6 to 12 membered, and more preferably, the fused cycloalkyl is a 7 to 10 membered. The fused cycloalkyl may be divided into bicyclic, tricyclic, tetracyclic or polycyclic fused cycloalkyl according to the number of constituent rings, is preferably the bicyclic or tricyclic fused cycloalkyl, and is more preferably a 5 membered/5 membered bicycloalkyl or a 5 memebered/6 membered bicycloalkyl. Non-limiting embodiments of the "fused cycloalkyl" comprise, but are not limited to, bicyclo[3.1.0]hexyl, bicyclo[3.2.0]heptyl-1-alkenyl, bicyclo[3.2.0]heptyl, decahydronaphthyl or tetrahydrophenyl.

"Bridged cycloalkyl" refers to a full-carbon polycyclic group with 5 to 18 atoms, and contains two or more cyclic structures sharing two carbon atoms not directly linked to each other, and one or more rings may contain 0, 1 or more double bonds, but no ring has a fully conjugated $\pi$ electron aromatic system. Preferably, the bridged cycloalkyl is a 6 to 12 membered, and more preferably, the bridged cycloalkyl is a 7 to 10 membered. Preferably, the bridged cycloalkyl is a 6 to 14 memebered, and more preferably, the bridged cycloalkyl is a 7 to 10 membered. The bridged cycloalkyl may be divided into bicyclic, tricyclic, tetracyclic or polycyclic bridged cycloalkyl according to the number of constituent rings, is preferably the bicyclic, tricyclic or tetracyclic bridged cycloalkyl, and is more preferably the bicyclic or tricyclic bridged cycloalkyl. Non-limiting embodiments of the "bridged cycloalkyl" comprise, but are not limited to, (1s,4s)-bicyclo [2.2.1] heptyl, bicyclo [3.2.1] octyl, (1s,5s)-bicyclo [3.3.1] nonyl, bicyclo [2.2.2] octyl and (1r,5r)-bicyclo [3.3.2] decyle.

"Heterocyclyl", "heterocycloalkyl", "heterocycle" or "heterocyclic" may be used interchangeably in the present application, and each refers to non-aromatic heterocyclyl, wherein one or more ring-forming atoms are heteroatomsselected from nitrogen, oxygen or $S(O)_t$ (wherein t is selected from 0, 1 or 2), the heterocyclyl comprises a monocyclic ring, a polycyclic ring, a fused ring, a bridged ring and a spirocyclic ring. Preferably, the heterocyclyl has a 5 to 7 membered monocyclic ring or a 7 to 10 membered bicyclic or tricyclic ring, which may contain 1, 2 or 3 atoms selected from nitrogen, oxygen and/or sulfur. Examples of the "heterocyclyl" comprise, but are not limited to, morpholinyl, oxetanyl, thiomorpholinyl, tetrahydrofuranyl, tetrahydropyranyl, 1,1-dioxo-thiomorpholinyl, piperidinyl, 2-oxopiperidinyl, pyrrolidinyl, 2-oxopyrrolidinyl, piperazine-2-one, 8-oxa-3-aza-bicyclo [3.2.1] octyl, piperazinyl and hexahydropyrimidine.

The heterocyclyl may be substituted or unsubstituted.

"Spiro-heterocyclyl" refers to a polycyclic group with 5 to 18 atoms, comprising two or more cyclic structures, and single rings sharing one atom with each other, and the ring contains one or more double bonds, but no ring has a fully conjugated $\pi$ electron aromatic system, wherein one or more ring atoms are heteroatoms selected from nitrogen, oxygen or $S(O)_t$ (wherein t is selected from 0, 1 or 2), and the remaining ring atoms are carbon. Preferably, the spiro-heterocyclyl is a 6 to 14 membered, and more preferably, the spiro-heterocyclyl is a 7 to 10 membered. The spiro-heterocyclyl is divided into mono-, di- or multi-spiro-heterocyclyl according to the number of shared spiro atoms between rings, is preferably the mono- and di-spiro-heterocyclyl, and is more preferably a 4 membered/4 membered mono-spiro-heterocyclyl, a 4 membered/5 membered mono-spiro-heterocyclyl, a 4 membered/6 membered mono-spiro-heterocyclyl, a 5 membered /5 membered mono-spiro-heterocyclyl or a 5 membered /6 membered mono-spiro-heterocyclyl . Non-limiting embodiments of the "spiro-heterocyclyl" comprise, but are not limited to, 1,7-dioxane [4.5] decyl, 2-oxa-7-azaspiro [4.4] nonyl, 7-oxaspiro [3.5] nonyl, 5-oxaspiro [2.4] heptyl,

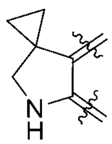 and 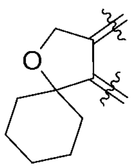 .

"Fused heterocyclyl" refers to a full carbon polycyclic group with two or more cyclic structures sharing one pair of atoms with each other, and one or more rings may contain one or more double bonds, but no ring has a fully conjugated $\pi$ electron aromatic system, wherein one or more ring atoms are heteroatoms selected from nitrogen, oxygen or $S(O)_t$ (wherein t is selected from 0, 1 or 2), and the remaining ring atoms are carbon. Preferably, the fused heterocyclyl is a 6 to 14 membered, and more preferably, the fused heterocyclyl is a 7 to 10 membered. The fused heterocyclyl may be divided into bicyclic, tricyclic, tetracyclic or polycyclic fused heterocyclyl according to the number of constituent rings, is preferably a bicyclic or tricyclic fused heterocyclyl, and is more preferably a 5 membered/5 membered bicyclic fused heterocyclyl or a 5 membered/6 membered bicyclic fused heterocyclyl. Non-limiting embodiments of the "fused heterocyclyl" comprise, but are not limited to, octahydropyrrolo [3,4-c] pyrrolyl, octahydro-1H-isoindolyl, 3-zabicyclo [3.1.0] hexyl and octahydrobenzo [b][1,4] dioxine.

"Bridged heterocyclyl" refers to a polycyclic group with 5 to 14 atoms, 5 to 18 atoms, and comprises two or more cyclic structures sharing two atoms not directly linked to each other, and one or more rings may contain one or more double

bonds, but no ring has a fully conjugated π electron aromatic system, wherein one or more ring atoms are heteroatoms selected from nitrogen, oxygen or $S(O)_t$ (wherein t is selected from 0, 1 or 2), and the remaining ring atoms are carbon. Preferably, the bridged heterocyclyl is a 6 to 14 membered, and more preferably, the bridged heterocyclyl is a 7 to 10 membered. The bridged heterocyclyl may be divided into bicyclic, tricyclic, tetracyclic or polycyclic bridged heterocyclyl according to the number of constituent rings, is preferably the bicyclic, tricyclic or tetracyclic bridged heterocyclyl, and is more preferably the bicyclic or tricyclic bridged heterocyclyl. Non-limiting embodiments of the "bridged heterocyclyl" comprise, but are not limited to, 2-azabicyclo[2.2.1]heptyl, 2-azabicyclo[2.2.2]octyl and 2-azabicyclo[3.3.2]decyl.

"Aryl" refers to a carbocyclic aromatic system containing one or two rings, wherein the rings may be linked together in a fused manner. The term "aryl" comprises monocyclic or bicyclic aryl, such as aromatic groups of phenyl, naphthyl and tetrahydronaphthyl. Preferably, the aryl is $C_6$-$C_{10}$ aryl, more preferably, the aryl is phenyl and naphthyl, and most preferably, the aryl is naphthyl. The aryl may be substituted or unsubstituted.

"Heteroaryl" refers to an aromatic monocyclic ring with 5 to 6 atoms or an aromatic bicyclic ring with 8 to 10 atoms, which may contain 1 to 4 atoms selected from nitrogen, oxygen and/or sulfur. Embodiments of the "heteroaryl" comprise, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothiophenyl, benzimidazolyl, indolyl, isoindolyl, 1,3-dioxo-isoindolyl, quinolyl, indazolyl, benzisothiazolyl, benzoxazolyl, benzisoxazolyl, isothiazolyl, 1H-1,2,4-triazolyl, 4H-1,2,4-triazolyl, pyridyl, pyridin-2(1H)-one group, pyrimidyl, pyrazin-2(1H)-one group, pyrimidin-4(3H)-one group, pyrimidin-2(1H)-one group, pyridazin-3(2H)-one group, 1H-indolyl, 1H-benzo[d]imidazolyl, 1H-pyrrolo[2,3-c]pyridyl, 3H-imidazo[4,5-c]pyridyl, isoquinolinyl, quinazolinyl, 2H-isoindolyl, furan[3,2-b]pyridyl, furan[2,3-c]pyridyl, thieno[2,3-c]pyridyl, benzofuryl, benzo[b]thienyl, 1H-pyrrolo[3,2-b]pyridyl and 2H-pyrrolo[3,4-c]pyridyl.

The heteroaryl may be substituted or unsubstituted.

"Fused ring" refers to a polycyclic group with two or more cyclic structures sharing one pair of atoms with each other, wherein at least one ring has a fully conjugated π electron aromatic system. Meanwhile, one or more rings may contain one or more double bonds, but at least one ring does not have the fully conjugated π electron aromatic system, wherein 0, 1 or more ring atoms are heteroatoms selected from nitrogen, oxygen or $S(O)_r$ (wherein r is selected from 0, 1 or 2), and the remaining ring atoms are carbon. The fused ring preferably comprises a bicyclic or tricyclic fused ring, wherein the bicyclic fused ring is preferably a fused ring of aryl or heteroaryl and monocyclic heterocyclyl or monocyclic cycloalkyl. Preferably, the fused ring is 6 to 14 membered, and more preferably, the fused ring is 8 to 10 membered. Embodiments of the "fused ring" comprise, but are not limited to:

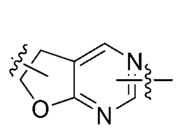 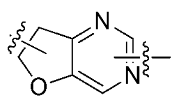 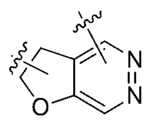 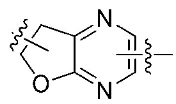 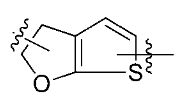 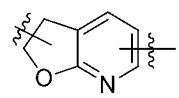

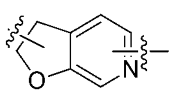 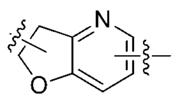 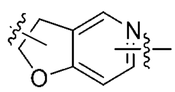 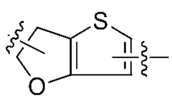 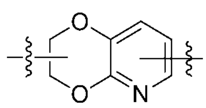 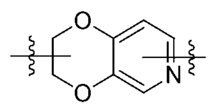

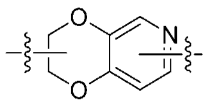 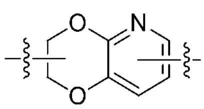 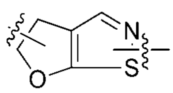 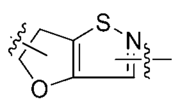 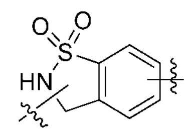 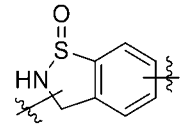

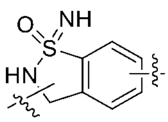 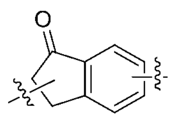 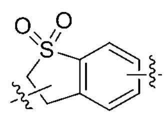 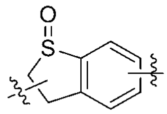 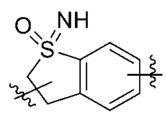 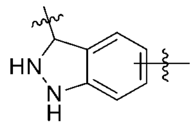

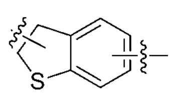 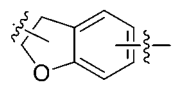 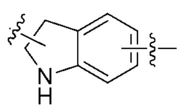 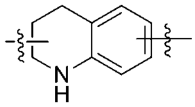 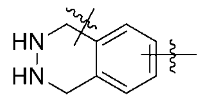 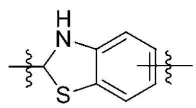

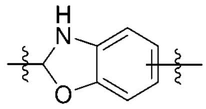 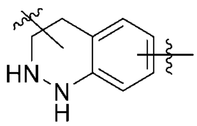 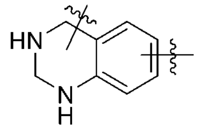 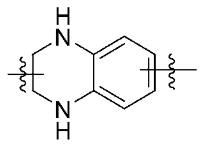 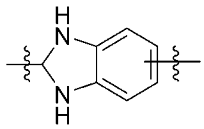 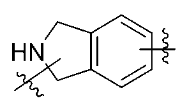

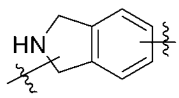 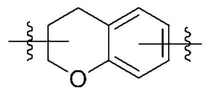 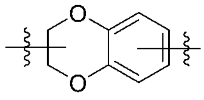 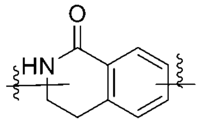 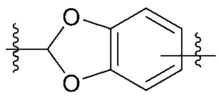 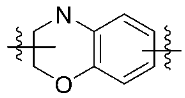

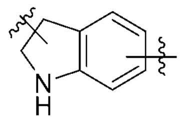 or 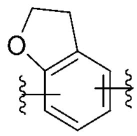 .

"Alkoxy" refers to a (alkyl-O-) group. The alkyl is defined herein. $C_1$-$C_6$ alkoxy is preferably selected. Examples of alkoxy comprise, but are not limited to, methoxy, ethoxy, n-propoxy, isopropoxy, n-butoxy, isobutoxy, tert-butoxy, and the like.

"Nitro" refers to a -$NO_2$ group.

"Hydroxyl" refers to a -OH group.

"Halogen" refers to fluorine, chlorine, bromine and iodine.

"Amino" refers to -$NH_2$.

"Cyano" refers to -CN.

"Benzyl" refers to -$CH_2$-phenyl.

"Carboxyl" refers to -C(=O)OH.

"Carboxylate group" refers to -C(O)O-alkyl or -C(O)O-cycloalkyl, wherein the alkyl and the cycloalkyl are defined as above.

"Hydroxyalkyl" refers to hydroxyl-substituted alkyl, wherein the alkyl is defined as above.

"Aminoalkyl" refers to amino-substituted alkyl, wherein the alkyl is defined as above.

"Halogenated alkyl" refers to halogen-substituted alkyl, wherein the alkyl is defined as above.

"Halogenated alkoxy" refers to halogen-substituted alkoxy, wherein the alkoxy is defined as above.

"DMSO" refers to dimethyl sulfoxide.

"BOC" refers to tert-butoxycarbonyl.

"Bn" refers to benzyl.

"THP" refers to 2-tetrahydropyranyl.

"TFA" refers to trifluoroacetic acid

"Ts" refers to p-toluenesulfonyl.

"Leaving group" is an atom or functional group detached from a larger molecule in a chemical reaction, which is a term used in a nucleophilic substitution reaction and an elimination reaction. In the nucleophilic substitution reaction, a reactant attacked by a nucleophilic reagent is called a substrate, and an atom or an atomic group broken together with a pair of electrons from a substrate molecule is called the leaving group. A group that accepts electrons easily and bears negative charges strongly is a good leaving group. When pKa of a conjugated acid of the leaving group is smaller, the leaving group is easier to be separated from other molecules. It is because that, when the pKa of the conjugated acid is smaller, the corresponding leaving group does not need to be combined with other atoms, and the trend of existence in the form of anion (or electrically neutral leaving group) is also enhanced. Common leaving groups comprise, but are not limited to, halogen, methanesulfonyl, -OTs or -OH.

"Substituted" refers to that one or more hydrogen atoms, preferably at most 5 hydrogen atoms, and more preferably 1 to 3 hydrogen atoms, in a group, are independently substituted by a corresponding number of substituents. It goes without saying that the substituents are only in their possible chemical positions, and those skilled in the art can determine (by experiment or theory) possible or impossible substitutions without excessive efforts. For example, amino or hydroxyl with free hydrogen may be unstable when combined with carbon atoms with unsaturated (such as olefinic) bonds.

"Substitution" or "substituted" in the specification, unless otherwise specified, refers to that the group may be substituted by one or more substituents selected from the following groups: alkyl, alkenyl, alkynyl, alkoxy, alkylthio, alkyl amino, halogen, sulfhydryl, hydroxyl, nitro, cyano, cycloalkyl, heterocyclyl, aryl, heteroaryl, cycloalkoxy, heterocycloalkoxy, cycloalkylthio, heterocycloalkylthio, amino, halogenated alkyl, hydroxyalkyl, carboxyl, carboxylate group, =O, $OR^5$, -C(=O)$R^5$, -C(=O)$OR^5$, -NHC(=O)$R^5$, -NHC(=O)$OR^5$, -$NR^6R^7$, -C(=O)$NR^6R^7$, -$CH_2$NHC(=O)$OR^5$, -$CH_2NR^6R^7$ or

$-S(O)_rR^5$;

each $R^5$ is independently selected from a hydrogen atom, alkyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;
each of $R^6$ and $R^7$ is independently selected from a hydrogen atom, hydroxyl, halogen, alkyl, alkoxy, cycloalkyl, heterocyclyl, aryl or heteroaryl, wherein the alkyl, the alkoxy, the cycloalkyl, the heterocyclyl, the aryl or the heteroaryl is optionally further substituted by one or more substituents selected from hydroxyl, halogen, nitro, cyano, alkyl, alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;
or, $R^6$ and $R^7$ form a 4-8 membered heterocyclyl together with atoms to which the $R^6$ and the $R^7$ are linked, wherein the 4-8 membered heterocyclyl contains one or more N, O or S(O)r, and the 4-8 membered heterocyclyl is optionally further substituted by one or more substituents selected from hydroxyl, halogen, nitro, cyano, alkyl, alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$or $-NR^9C(=O)R^{10}$;
each of $R^8$, $R^9$ and $R^{10}$ is independently selected from a hydrogen atom, alkyl, amino, cycloalkyl, heterocyclyl, aryl or heteroaryl, wherein the alkyl, the cycloalkyl, the heterocyclyl, the aryl or the heteroaryl is optionally further substituted by one or more substituents selected from: hydroxyl, halogen, nitro, amino, cyano, alkyl, alkoxy, cycloalkyl, heterocyclyl, aryl, heteroaryl, carboxyl or a carboxylate group; and
each r is independently 0, 1 or 2.

The compound of the present invention may contain an asymmetric center or a chiral center, thus existing in different stereoisomeric forms. It is expected that all stereoisomeric forms of the compound of the present invention comprise, but are not limited to, a diastereomer, an enantiomer, and an atropisomer and a geometric (conformational) isomer and a mixture thereof, such as a racemic mixture, which are all within the scope of the present invention.
Unless otherwise specified, the structure described in the present invention further comprises all isomers of this structure (such as forms of a diastereomer, an enantiomer, and an atropisomer and a geometric (conformational) isomer; such as R and S configurations of asymmetric centers, (Z) and (E) double-bond isomers, and (Z) and (E) conformational isomers). Therefore, a single stereoisomer, and an enantiomeric mixture, a diastereomeric mixture and a geometric (conformational) isomer mixture of the compound of the present invention are all within the scope of the present invention.
"Pharmaceutically acceptable salt" refers to some salts of the above compounds capable of maintaining original biological activity and suitable for medical use. The pharmaceutically acceptable salt of the compound as shown in general formula (I) may be a metal salt or an amine salt formed with a suitable acid.
"Pharmaceutical composition" refers to a mixture containing one or more compounds described herein or their physiologically acceptable salts or prodrugs and other chemical components, and other components such as physiologically acceptable carriers and excipients. The purpose of the pharmaceutical composition is to promote the administration to organisms, which is beneficial for the absorption of active ingredients, thus exerting biological activity.

Synthesis method for compounds of the present invention

In order to achieve the objective of the present invention, the following technical solution is used in the present invention.
The present invention provides a preparation method for a compound as shown in general formula (I), or a stereoisomer thereof, a tautomer thereof or a pharmaceutically acceptable salt thereof, and the method comprises:

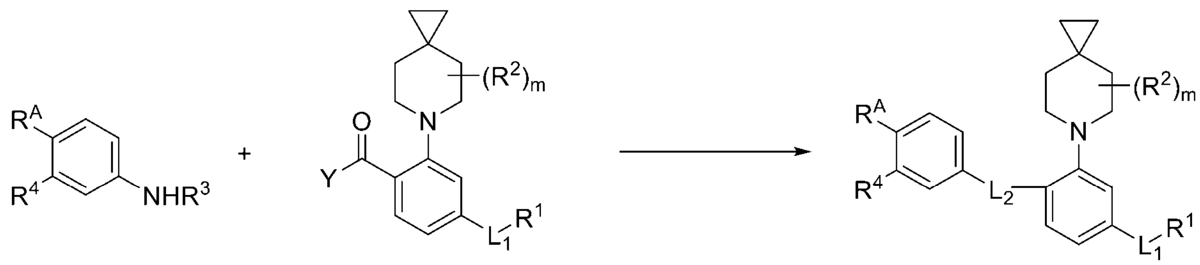

(IA) (IB) (I)

carrying out a condensation reaction on a compound as shown in general formula (IA) and a compound as shown in general formula (IB), and optionally further carrying out a substitution reaction to obtain the compound as shown in general formula (I),
wherein:

$L_2$ is selected from

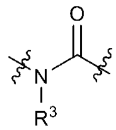
;

$R^3$ is selected from a hydrogen atom;
Y is selected from hydroxyl or chlorine; and
$L_1$, $R^1$, $R^2$, $R^4$, $R^A$ and m are as defined in general formula (I).

EMBODIMENT

The present invention is further described hereinafter with reference to the examples, but these exampless are not intended to limit the scope of the present invention.
The preparation of a representative compound as shown in formula (I) and related structural identification data are given in the examples. It must be noted that the following embodiments are used to illustrate the present invention and are not intended to limit the present invention. A $^1$H NMR spectrum is measured by a Bruker instrument (400 MHz), and a chemical shift is represented by ppm. Tetramethylsilane is used as an internal standard (0.00 ppm). In a representation method of $^1$H NMR: s = single peak, d = double peak, t = triple peak, m = multiple peak, br = broadening, dd = double peak of double peak, and dt = double peak of triple peak. When a coupling constant is provided, the unit of the coupling constant is Hz.
A mass spectrum is measured by a LC/MS instrument, and an ionization mode may be ESI or APCI.
A silica gel plate used for thin-layer chromatography is a Yantai Huanghai HSGF254 or Qingdao GF254 silica gel plate, the silica gel plate used for thin-layer chromatography (TLC) adopts a specification of 0.15 mm-0.2 mm, and a product separated and purified by thin-layer chromatography adopts a specification of 0.4 mm-0.5 mm.
200-300-mesh silica gel of Yantai Huanghai silica gel is generally used as a carrier in column chromatography.
In the following embodiments, unless otherwise specified, all temperatures are Celsius temperatures. Unless otherwise specified, various starting materials and reagents are commercially available or synthesized according to known methods, and the commercially available raw materials and reagents are directly used without further purification. Unless otherwise specified, manufacturers of the commercially available raw materials and reagents comprise, but are not limited to, Aldrich Chemical Company, ABCR GmbH & Co.KG, Acros Organics, Guangzan Chemical Technology Co., Ltd., Jingyan Chemical Technology Co., Ltd., etc.

$CD_3OD$: deuterated methanol.
$CDCl_3$: deuterated chloroform.
DMSO-$d_6$: deuterated dimethyl sulfoxide.

Argon atmosphere means that a reaction bottle is connected with an argon balloon with a volume of about 1 L.
Unless otherwise specified in the examples, a solution in the reaction refers to an aqueous solution.
The compound is purified by silica gel column chromatography and reversed-phase column chromatography, wherein an eluent system is selected from: an A system: petroleum ether and ethyl acetate system; a B system: dichloromethane and methanol system; a C system: dichloromethane and ethyl acetate system; and a D system: trifluoroacetic acid aqueous solution and acetonitrile system. A volume ratio of a solvent varies according to the polarity of the compound, and may also be adjusted by adding a small amount of acidic or alkaline reagent, such as acetic acid or triethylamine.

Example 1

N-(4-carbamoyl-3-(4,4-difluoropiperidin-1-yl)phenyl)-4-(ethylsulfonamido)-2-(6-azaspiro[2.5]o ctan-6-yl)benzamide

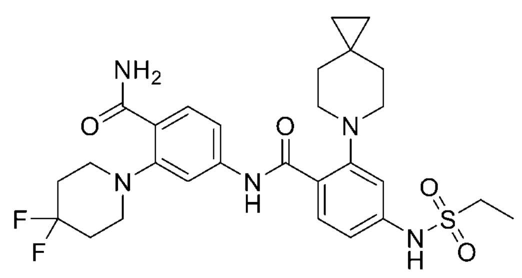

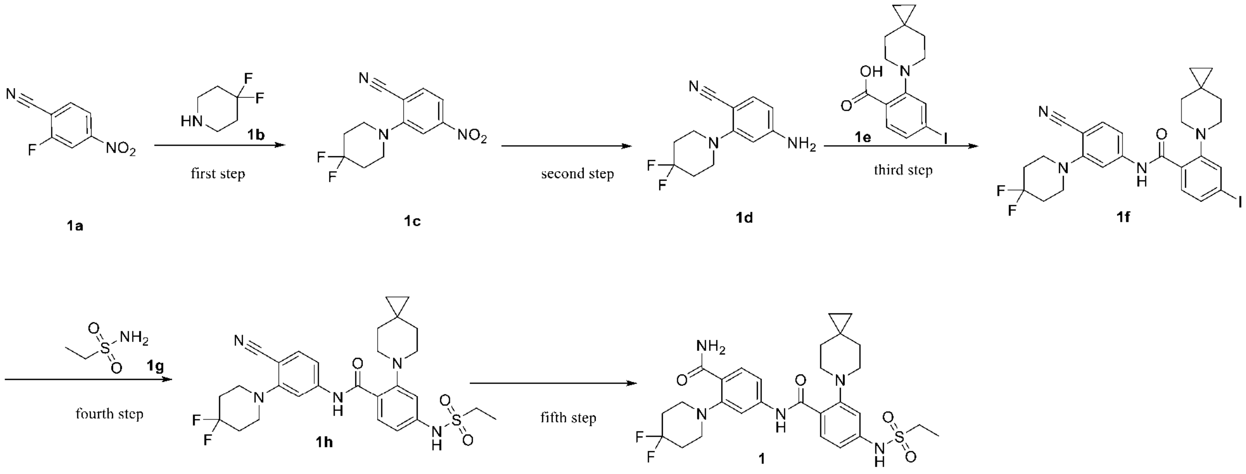


First step

2-(4,4-difluoropiperidin-1-yl)-4-nitrobenzonitrile

2-Fluoro-4-nitro-benzonitrile **1a** (1 g, 6.02 mmol, commercially available), 4,4-difluorpiperidine **1b** (802.13 mg, 6.62 mmol, commercially available) and potassium carbonate (2.50 g, 18.06 mmol) were dissolved in N,N-dimethylformamide (5 mL), heated to 80°C, and stirred for 4 hours. Water (20 mL) was added and the obtained system was extracted with ethyl acetate (30 mL×2), and organic phases were combined, washed with a saturated sodium chloride solution (20 mL), dried with anhydrous sodium sulfate, filtered, and concentrated. The crude product was dissolved in ethyl acetate, then petroleum ether was added to precipitate a solid, the system was filtered, and dried to obtain 2-(4,4-difluoropiperidin-1-yl)-4-nitrobenzonitrile **1c** (1.0 g), with a yield of 62.16%.
MS m/z (ESI): 268.1 [M+1]

Second step

4-amino-2-(4,4-difluoropiperidin- 1 -yl)benzonitrile

2-(4,4-Difluoropiperidin-1-yl)-4-nitrobenzonitrile **1c** (1.0 g, 3.74 mmol) was dissolved in methanol (10 mL), 10% palladium on carbon (119.47 mg, 1.12 mmol) was added, the system was subjected to nitrogen purging for 3 times, and stirred at room temperature for 6 hours. The mixture was filtered and concentrated under reduced pressure. The residue was separated and purified by silica gel column chromatography (eluent: A system) to obtain 4-amino-2-(4,4-difluoropiperidin-1-yl)benzonitrile **1d** (850 mg), with a yield of 95.74%.
MS m/z (ESI): 238.1 [M+1]

Third step

N-(4-cyano-3-(4,4-difluoropiperidin-1-yl)phenyl)-4-iodo-2-(6-azaspiro[2.5]octan-6-yl)benzamid e

2-(6-Azaspiro[2.5]octan-6-yl)-4-iodo-benzoic acid **1e** (662.44 mg, 1.85 mmol, prepared by the method disclosed in WO2020132648) and 2-amino-6-(4,4-difluoropiperidin-1-yl)benzonitrile **1d** (400 mg, 1.69 mmol) were dissolved in 1,4-dioxane (1 mL), N,N,N',N'-tetramethylchloroformamidinium hexafluorophosphate (1.42 g, 5.06 mmol) and N-methylimidazole (415.26 mg, 5.06 mmol) were added, and the system was heated to 100°C to react for 3 hours. Water (20 mL) was added to the reaction solution and the system was extracted with ethyl acetate (30 mL×2), and a combined organic phase

was washed with a saturated sodium chloride solution (10 mL), dried with anhydrous sodium sulfate, filtered, and concentrated. The residue was separated and purified by silica gel column chromatography (eluent: A system) to obtain N-(4-cyano-3-(4,4-difluoropiperidin-1-yl)phenyl)-4-iodo-2-(6-azaspiro[2.5]octan-6-yl)benzamid e **1f** (700 mg), with a yield of 72.03%.
MS m/z (ESI): 576.2 [M+1]

Fourth step

N-(4-cyano-3-(4,4-difluoropiperidin-1-yl)phenyl)-4-(ethylsulfonamido)-2-(6-azaspiro[2.5]octan-6-yl)benzamide

Sarcosine (77.28 mg, 867.43 μmol), cuprous iodide (82.60 mg, 433.71 μmol) and potassium phosphate (920.64 mg, 4.34 mmol) were added to a N,N-dimethylformamide solution (5 mL) of N-(4-cyano-3-(4,4-difluoropiperidin-1-yl) phenyl)-4-iodo-2-(6-azaspiro[2.5]octan-6-yl)benzamid e **1f** (500 mg, 867.43 μmol) and ethyl sulfonamide **1g** (94.68 mg, 867.43 μmol, commercially available) , and the system was stirred at 100°C under an $N_2$ atmosphere for 16 hours, until mass spectrometry showed that the reaction was complete. The reaction solution was filtered and concentrated. The residue was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(4-cyano-3-(4,4-difluoropiperidin-1-yl)phenyl)-4-(ethylsulfonamido)-2-(6-azaspiro[2.5]octan-6-yl)benzamide **1h** (235 mg), with a yield of 47.35%.

MS m/z (ESI): 558.6 [M+1]
1H NMR (400 MHz, ) δ 11.83 (s, 1H), 10.19 (s, 1H), 7.82 - 7.68 (m, 3H), 7.50 - 7.42 (m, 1H), 7.16 (d, J = 2.0 Hz, 1H), 7.03 (dd, J = 8.5, 2.1 Hz, 1H), 3.28 (d, J = 6.6 Hz, 4H), 3.20 (q, J = 7.3 Hz, 2H), 2.96 (t, J = 5.2 Hz, 4H), 2.22 - 2.12 (m, 4H), 1.51 (s, 4H), 1.21 (t, J = 7.3 Hz, 3H), 0.34 (s, 4H).

Fifth step

N-(4-carbamoyl-3-(4,4-difluoropiperidin-1-yl)phenyl)-4-(ethylsulfonamido)-2-(6-azaspiro[2.5]o ctan-6-yl)benzamide

N-(4-cyano-3-(4,4-difluoropiperidin-1-yl)phenyl)-4-(ethylsulfonamido)-2-(6-azaspiro[2.5]o ctan-6-yl)benzamide **1h** (200 mg, 358.65 μmol) was dissolved in dimethyl sulfoxide (1 mL), an aqueous solution of ethanol (1 mL) and sodium hydroxide (28.69 mg, 717.29 μmol) were added, hydrogen peroxide (0.5 mL, 25%) was added dropwise to react at room temperature for 2 hours. After mass spectrometry showed that the reaction was complete, water (20 mL) was added and the system was extracted with ethyl acetate (30 mL×2), and organic phases were combined, washed with saturated sodium chloride (20 mL), dried with anhydrous sodium sulfate, filtered, and concentrated. The residue was separated and purified by silica gel column chromatography (eluent: A system) to obtain N-(4-carbamoyl-3-(4,4-difluoropiperidin-1-yl)phenyl)-4-(ethylsulfonamido)-2-(6-azaspiro[2.5]o ctan-6-yl)benzamide 1 (143.1 mg), with a yield of 67.65%.

MS m/z (ESI): 576.3 [M+1]
1H NMR (400 MHz) δ 11.81 (s, 1H), 10.17 (s, 1H), 8.18 (s, 1H), 7.82 (d, J = 8.4 Hz, 1H), 7.71 (d, J = 8.4 Hz, 1H), 7.62 (d, J = 2.0 Hz, 1H), 7.52 (dd, J = 8.4, 1.9 Hz, 1H), 7.41 (s, 1H), 7.17 (d, J = 2.1 Hz, 1H), 7.04 (dd, J = 8.5, 2.1 Hz, 1H), 3.20 (q, J = 7.3 Hz, 2H), 3.05 (t, J = 5.5 Hz, 4H), 2.98 (d, J = 10.7 Hz, 4H), 2.19 (d, J = 14.1 Hz, 4H), 1.54 (s, 4H), 1.21 (t, J = 7.3 Hz, 3H), 0.35 (s, 4H).

Example 2

2-(4,4-difluoropiperidin-1-yl)-4-(4-(ethylsulfonamido)-2-(6-azaspiro[2.5]octan-6-yl)benzamido) benzoic acid

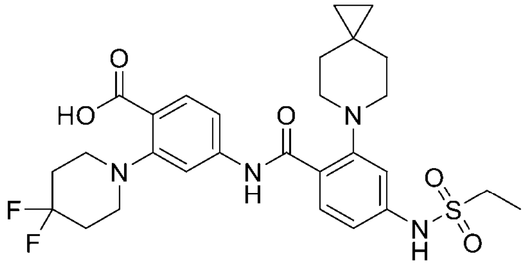

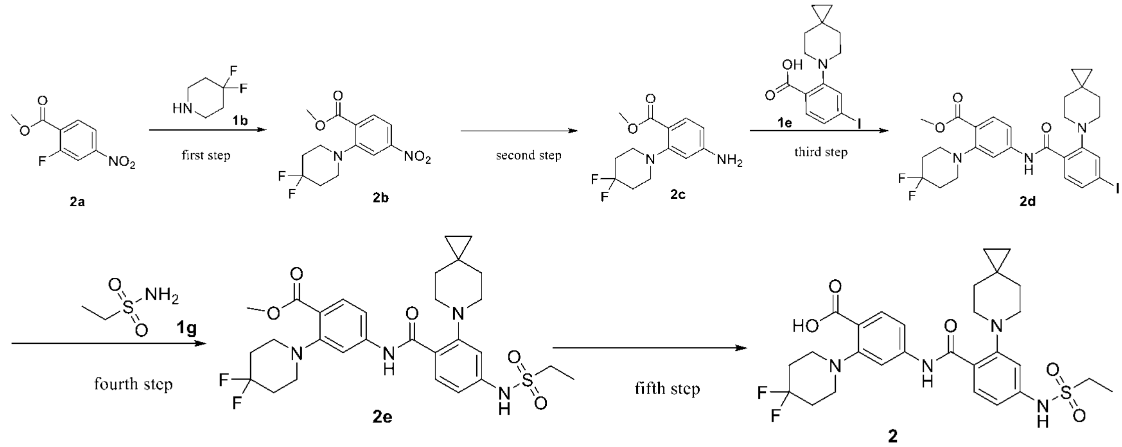


First step

methyl 2-(4,4-difluoropiperidin-1-yl)-4-nitrobenzoate

4,4-difluorpiperidine **1b** (500 mg, 4.13 mmol) and methyl 2-fluoro-4-nitrobenzoate 2a (822.00 mg, 4.13 mmol) were dissolved in dimethyl sulfoxide (6 mL), triethylamine (417.70 mg, 4.13 mmol) was added, and stirred at 120°C for 8 hours, until mass spectrometry showed that the reaction was complete. Water (10 mL) was added to the reaction solution and the system was extracted with ethyl acetate (30 mL×2), and a combined organic phase was washed with saturated sodium chloride (20 mL), dried with anhydrous sodium sulfate, filtered, and concentrated. The crude product was separated and purified by silica gel column chromatography (eluent: A system) to obtain methyl 2-(4,4-difluoropiperidin-1-yl)-4-nitrobenzoate **2b** (872 mg), with a yield of 70.36%.
MS m/z (ESI): 301.1 [M+1]

Second step

methyl 4-amino-2-(4,4-difluoropiperidin-1-yl)benzoate

At room temperature, methyl 2-(4,4-difluoropiperidin-1-yl)-4-nitrobenzoate **2b** (500 mg, 1.67 mmol) was dissolved in methanol (10 mL), palladium on carbon (606.73 mg, 5.00 mmol, 10%) was added, and the system was stirred under a hydrogen atmosphere for 16 hours, until mass spectrometry showed that the reaction was complete. The reaction solution was filtered, and the filtrate was spin-dried and directly used in the next step to obtain methyl 4-amino-2-(4,4-difluoropiperidin-1-yl)benzoate **2c** (400 mg), with a yield of 88.87%.
MS m/z (ESI): 271.2 [M+1]

Third step

methyl

2-(4,4-difluoropiperidin-1-yl)-4-(4-iodo-2-(6-azaspiro[2.5]octan-6-yl)benzamido)benzoate

2-(6-Azaspiro[2.5]octan-6-yl)-4-iodo-benzoic acid **1e** (500 mg, 1.35 mmol) was dissolved in dichloromethane (1 mL), then oxalyl chloride (170.97 mg, 1.35 mmol) was added dropwise, and the obtained system was stirred at room temperature for 15 minutes. The reaction system was concentrated under reduced pressure, and then a dichloromethane

solution (1 mL) of methyl 4-amino-2-(4,4-difluoropiperidin-1-yl)benzoate **2c** (364.04 mg, 1.35 mmol) was added into the reaction flask and the mixture was stirred at room temperature for 30 minutes, until mass spectrometry showed that the reaction was complete. Water (10 mL) was added to the reaction solution and the system was extracted with ethyl acetate (30 mL×2), and a combined organic phase was washed with saturated sodium chloride (20 mL), dried with anhydrous sodium sulfate, filtered, and concentrated. The crude product was separated and purified by silica gel column chromatography (eluent: A system) to obtain methyl 2-(4,4-difluoropiperidin-1-yl)-4-(4-iodo-2-(6-azaspiro[2.5]octan-6-yl)benzamido)benzoate **2d** (800 mg), with a yield of 97.46%.
MS m/z (ESI): 610.1 [M+1]

Fourth step

methyl 2-(4,4-difluoropiperidin-1-yl)-4-(4-(ethylsulfonamido)-2-(6-azaspiro[2.5]octan-6-yl)benzamido) benzoate

Methyl 2-(4,4-difluoropiperidin-1-yl)-4-(4-iodo-2-(6-azaspiro[2.5]octan-6-yl)benzamido)benzoate **2d** (800 mg, 1.31 mmol) and ethyl sulfonamide **1g** (214.91 mg, 1.97 mmol) were dissolved in N,N-dimethylformamide (10 mL), potassium phosphate (557.28 mg, 2.63 mmol), cuprous iodide (145.75 mg, 656.33 μmol) and sarcosine (116.95 mg, 1.31 mmol) were added, and the system was heated to 110°C under argon protection to react for three hours. Water (10 mL) was added to the reaction solution and the system was extracted with ethyl acetate (30 mL×2), and a combined organic phase was washed with saturated sodium chloride (20 mL), dried with anhydrous sodium sulfate, filtered, and concentrated. The residue was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain a product methyl 2-(4,4-difluoropiperidin-1-yl)-4-(4-(ethylsulfonamido)-2-(6-azaspiro[2.5]octan-6-yl)benzamido) benzoate 2e (160 mg), with a yield of 20.64%.
MS m/z (ESI): 591.1 [M+1]

Fifth step

2-(4,4-difluoropiperidin-1-yl)-4-(4-(ethylsulfonamido)-2-(6-azaspiro[2.5]octan-6-yl)benzamido) benzoic acid

Methyl 2-(4,4-difluoropiperidin-1-yl)-4-(4-(ethylsulfonamido)-2-(6-azaspiro[2.5]octan-6-yl)benzamido) benzoate **2e** (100 mg, 169.30 μmol) was dissolved in methanol (1 mL) and water (1 mL), sodium hydroxide (20.32 mg, 507.89 μmol) was added, and the system was stirred at room temperature for 16 hours, until mass spectrometry showed that the reaction was complete. The reaction solution was extracted with ethyl acetate (30 mL×2), and organic phases were combined, washed with saturated sodium chloride (20 mL), dried with anhydrous sodium sulfate, filtered, and concentrated. The residue was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4,4-difluoropiperidin-1-yl)-4-(4-(ethylsulfonamido)-2-(6-azaspiro[2.5]octan-6-yl)benzamido) benzoic acid 2 (80 mg), with a yield of 74.73%.
MS m/z (ESI): 576.9 [M+1]

Example 3

2-(4,4-difluoropiperidin-1-yl)-4-(4-(ethylsulfonamido)-2-(6-azaspiro[2.5]octan-6-yl)benzamido)-N-methylbenzamide

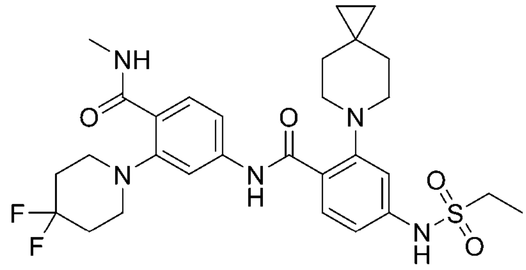

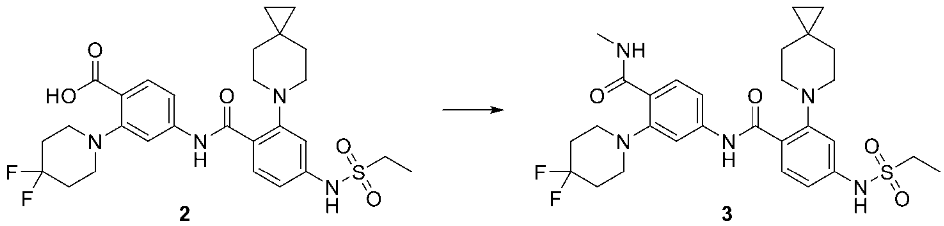

2 3

1-Hydroxybenzotriazole (3.51 mg, 26.01 μmol) and 1-(3-dimethylaminopropyl)-3-ethylcarbodiimide hydrochloride (4.99 mg, 26.01 μmol) were added to a 1,4-dioxane (1 mL) mixture of 2-(4,4-difluoropiperidin-1-yl)-4-(4-(ethylsulfonamido)-2-(6-azaspiro[2.5]octan-6-yl)benzamido) benzoic acid **2** (15 mg, 26.01 μmol) and methylamine (807.86 μg, 26.01 μmol), and th system was stirred at room temperature for 1 hour, until mass spectrometry showed that the reaction was complete. The reaction solution was concentrated. The residue was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4,4-difluoropiperidin-1-yl)-4-(4-(ethylsulfonamido)-2-(6-azaspiro[2.5]octan-6-yl) benzamido)-N-methylbenzamide 3 (10 mg), with a yield of 64.93%.
MS m/z (ESI): 590.0 [M+1]

Example 4

2-(4,4-difluoropiperidin-1-yl)-4-(4-(ethylsulfonamido)-2-(6-azaspiro[2.5]octan-6-yl)benzamido)-N,N-dimethylbenzamide

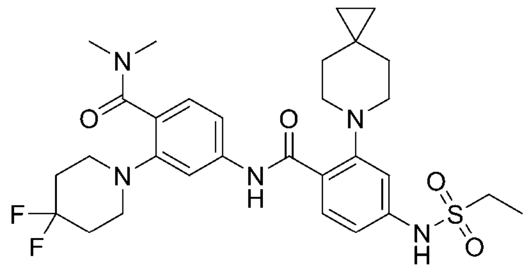

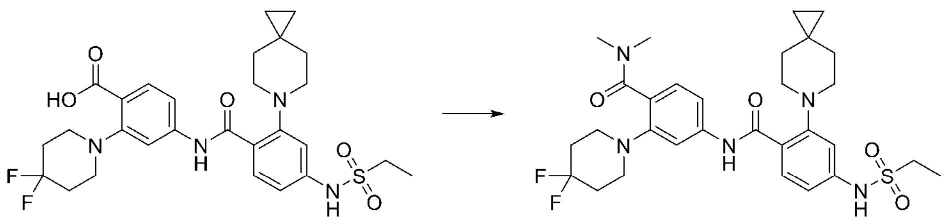

2 4

Tetramethylchlorouronium hexafluorophosphate (4.87 mg, 17.34 μmol) and N-methylimidazole (1.42 mg, 17.34 μmol) were added to a 1,4-dioxane (1 mL) mixture of 2-(4,4-difluoropiperidin-1-yl)-4-(4-(ethylsulfonamido)-2-(6-azaspiro [2.5]octan-6-yl)benzamido) benzoic acid **2** (15 mg, 26.01 μmol) and dimethylamine (781.81 μg, 17.34 μmol), and stirred at room temperature for 1 hour, until mass spectrometry showed that the reaction was complete. The reaction solution was concentrated. The residue was separated by preparative liquid chromatography (separation column AKZONOBEL

Kromasil; 250×21.2 mm I.D.; 5 μm, 20 mL/min; mobile phase A: 0.05% TFA+$H_2O$, mobile phase B: $CH_3CN$) to obtain 2-(4,4-difluoropiperidin-1-yl)-4-(4-(ethylsulfonamido)-2-(6-azaspiro[2.5]octan-6-yl)benzamido)-N,N-dimethylbenzamide **4** (8 mg), with a yield of 76.18%.

MS m/z (ESI): 604.2 [M+1]
1H NMR (400 MHz) δ 11.82 (s, 1H), 7.83 (d, J = 8.5 Hz, 1H), 7.58 (d, J = 1.9 Hz, 1H), 7.46 - 7.35 (m, 1H), 7.24 - 7.11 (m, 2H), 7.03 (dd, J = 8.5, 2.1 Hz, 1H), 3.18 (d, J = 7.3 Hz, 2H), 2.98 (d, J = 11.0 Hz, 8H), 2.87 (s, 3H), 2.79 (s, 3H), 2.12 - 1.97 (m, 4H), 1.55 (s, 3H), 1.25 - 1.17 (m, 4H), 0.35 (s, 4H).

Example 5

N-(4-acetyl-3-(4,4-difluoropiperidin-1-yl)phenyl)-4-(ethylsulfonamido)-2-(6-azaspiro[2.5]octan-6-yl)benzamide

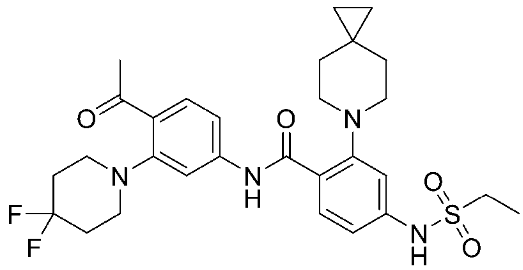

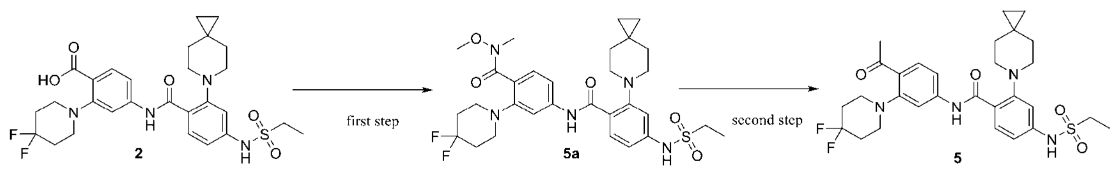


First step

2-(4,4-difluoropiperidin-1-yl)-4-(4-(ethylsulfonamido)-2-(6-azaspiro[2.5]octan-6-yl)benzamido)-N-methoxy-N-methylbenzamide

2-(4,4-Difluoropiperidin-1-yl)-4-(4-(ethylsulfonamido)-2-(6-azaspiro[2.5]octan-6-yl)benza mido)benzoic acid **2** (100 mg, 173.41 μmol) was dissolved in N,N-dimethylformamide (0.5 mL), tetramethylchlorouronium hexafluorophosphate (97.31 mg, 346.83 μmol) and N-methylimidazole (28.47 mg, 346.83 μmol) were added, stirred at room temperature for 1 hour, then free N-methoxymethylamine (16.92 mg, 173.41 μmol) was added, and stirred at room temperature for 1 hour, until mass spectrometry showed that the reaction was complete. Water (10 mL) was added to the reaction solution and the system was extracted with ethyl acetate (30 mL×2), and a combined organic phase was washed with saturated sodium chloride (20 mL), dried with anhydrous sodium sulfate, filtered, and concentrated. The crude product was separated and purified by silica gel column chromatography (eluent: A system) to obtain 2-(4,4-difluoropiperidin-1-yl)-4-(4-(ethylsulfonamido)-2-(6-azaspiro[2.5]octan-6-yl)benzamido)-N-methoxy-N-methylbenzamide **5a** (100 mg), with a yield of 93.05%.
MS m/z (ESI): 620.1 [M+1]

Second step

N-(4-acetyl-3-(4,4-difluoropiperidin-1-yl)phenyl)-4-(ethylsulfonamido)-2-(6-azaspiro[2.5]octan-6-yl)benzamide

2-(4,4-Difluoropiperidin-1-yl)-4-(4-(ethylsulfonamido)-2-(6-azaspiro[2.5]octan-6-yl)benza mido)-N-methoxy-N-methylbenzamide **5a** (100 mg, 161.36 μmol) was dissolved in tetrahydrofuran (2 mL), then methyl lithium (1.6 M ether solution) (3.55 mg, 161.36 μmol) was added at 0°C, and stirred at room temperature for 1 hour, until mass spectrometry showed that the reaction was complete. Water was added to the reaction solution for quenching, and separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain

N-(4-acetyl-3-(4,4-difluoropiperidin-1-yl)phenyl)-4-(ethylsulfonamido)-2-(6-azaspiro[2.5]octan-6-yl)benzamide **5** (8 mg), with a yield of 8.63%.
MS m/z (ESI): 574.9 [M+1]
1H NMR (400 MHz, DMSO-d6) δ 13.05 (s, 1H), 7.70 (d, J = 8.7 Hz, 1H), 7.57 (d, J = 8.1 Hz, 2H), 7.49 (d, J = 8.5 Hz, 1H), 6.83 (d, J = 2.2 Hz, 1H), 6.73 - 6.62 (m, 1H), 3.07 (d, J = 5.8 Hz, 4H), 2.92 (d, J = 5.5 Hz, 4H), 2.72 (q, J = 7.4 Hz, 2H), 2.57 (s, 3H), 2.18 (d, J = 16.3 Hz, 4H), 1.23 (s, 4H), 1.12 (t, J = 7.3 Hz, 3H), 0.39 (s, 4H).

Example 6

N-(3-(4,4-difluoropiperidin-1-yl)-4-(hydroxymethyl)phenyl)-4-(ethylsulfonamido)-2-(6-azaspiro [2.5]octan-6-yl)benzamide

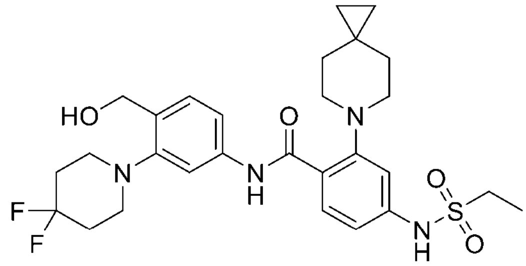

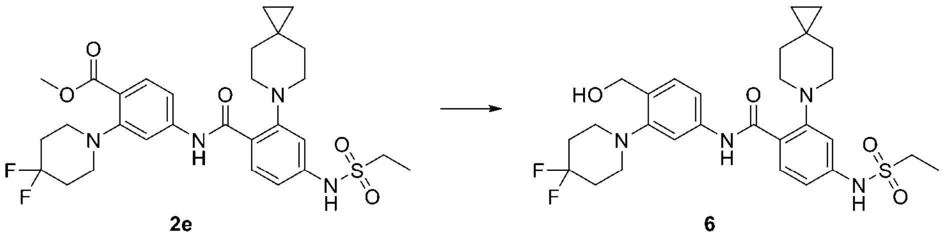

2e 6

Methyl 2-(4,4-difluoropiperidin-1-yl)-4-(4-(ethylsulfonamido)-2-(6-azaspiro[2.5]octan-6-yl)benzamido) benzoate **2e** (50 mg, 84.65 μmol) was dissolved in tetrahydrofuran (1 mL), lithium borohydride (1.84 mg, 84.65 μmol) was added dropwise, the system was heated to 75°C, and stirred for 6 hours. Water was added to the reaction solution for quenching, and then the system was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(hydroxymethyl)phenyl)-4-(ethylsulfonamido)-2-(6-azaspiro [2.5]octan-6-yl)benzamide **6** (5 mg), with a yield of 10.50%.
MS m/z (ESI): 563.2 [M+1]

Example 7

N-(3-(4,4-difluoropiperidin-1-yl)-4-(oxazol-2-yl)phenyl)-4-(ethylsulfonamido)-2-(6-azaspiro[2.5 ]octan-6-yl)benzamide

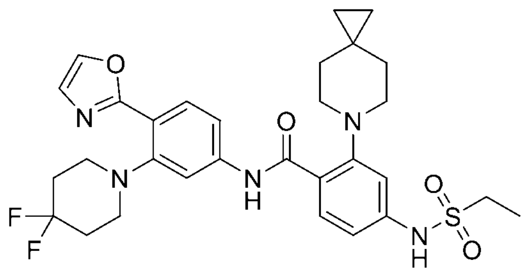

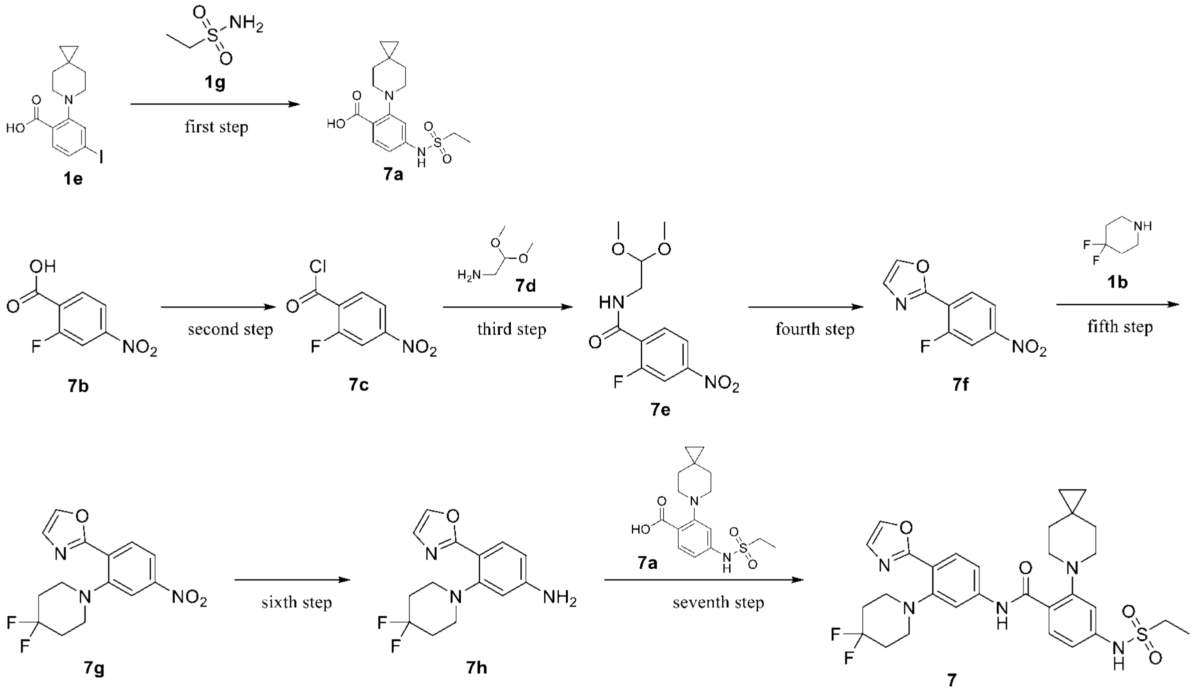


First step

4-(ethylsulfonamido)-2-(6-azaspiro[2.5]octan-6-yl)benzoic acid

2-(6-Azaspiro[2.5]octan-6-yl)-4-iodo-benzoic acid **1e** (1.0 g, 2.80 mmol) and ethyl sulfonamide **1g** (458.36 mg, 4.20 mmol) were dissolved in N,N-dimethylformamide (10 mL), cuprous iodide (266.60 mg, 1.40 mmol), sarcosine (249.43 mg, 2.80 mmol) and potassium phosphate (3.73 g, 14.00 mmol) were added, the system was degassed by argon sparging for 5 minutes, heated to 110°C, and then stirred for 6 hours. After cooling to room temperature, the reaction solution was poured into 200 mL of ice water, and a pH value was adjusted to 6 with dilute hydrochloric acid (2 M). The reaction solution was extracted with dichloromethane (200 mL×3), and a combined organic phase was washed with saturated sodium chloride solution (50 mL), dried with anhydrous sodium sulfate, filtered, and concentrated. The crude product was separated and purified by silica gel column chromatography (eluent: A system) to obtain 4-(ethylsulfonamido)-2-(6-azaspiro[2.5]octan-6-yl)benzoic acid **7a** (0.75 g), with a yield of 79.16%.
MS m/z (ESI): 339.4 [M+1]

Second step

2-fluoro-4-nitrobenzoyl chloride

2-Fluoro-4-nitrobenzoic acid **7b** (5.00 g, 27.01 mmol, commercially available) was dissolved in a dichloromethane solution (60 mL), a drop of N,N-dimethylformamide was added, oxalyl chloride (3.43 g, 27.01 mmol) was dropwise added in batches in an ice bath, and the system was subjected to nitrogen purging for three times. The reaction was carried out at 25°C for 1 hour to obtain a yellow suspension. After mass spectrometry showed that the reaction of starting materials was complete, the reaction solution was concentrated to dryness under reduced pressure to obtain 2-fluoro-4-nitrobenzoyl chloride **7c** (5.60 g). The crude product was directly used in the next step of the reaction.

MS m/z (ESI): 200.0 [M+1]

Third step

N-(2,2-dimethoxyethyl)-2-fluoro-4-nitrobenzamide

2-Fluoro-4-nitrobenzoyl chloride **7c** (5.60 g, 27.51 mmol, crude product) was dissolved in a tetrahydrofuran solution (60 mL), and sodium bicarbonate (6.93 g, 82.53 mmol) and 2,2-dimethoxyethane-1-amine **7d** (4.34 g, 41.27 mmol) were added. The system was subjected to nitrogen purging for three times. The reaction was carried out at 25°C for 18 hours to obtain a yellow suspension. After it was detected that the reaction of starting materials was complete, the reaction solution was poured into 200 mL of water and extracted with ethyl acetate (60 mL×2), and a combined organic phase was washed with saturated sodium chloride solution (60 mL), dried with anhydrous sodium sulfate, filtered, and concentrated under reduced pressure. The obtained crude product was separated and purified by silica gel column chromatography (eluent: A system) to obtain N-(2,2-dimethoxyethyl)-2-fluoro-4-nitrobenzamide **7e** (6.3 g), with a yield of 84.12%.
1H NMR (400 MHz, DMSO) δ 8.73 (s, 1H), 8.20 (dd, J = 9.8, 2.1 Hz, 1H), 8.14 (dd, J = 8.4, 2.1 Hz, 1H), 7.81 (dd, J = 8.4, 7.1 Hz, 1H), 4.51 (t, J = 5.8 Hz, 1H), 3.38 (t, J = 5.8 Hz, 2H), 3.31 (s, 6H).

Fourth step

2-(2-fluoro-4-nitrophenyl)oxazole

N-(2,2-dimethoxyethyl)-2-fluoro-4-nitrobenzamide **7e** (200.00 mg, 0.73 mmol) was added into an Eaton's Reagent (1.75 g, 7.35 mmol) at 0°C, and subjected to nitrogen purging for three times. The reaction was quickly transferred to 145°C and carried out for 2 hours to obtain a black suspension. After mass spectrometry showed that the reaction of starting materials was complete, the reaction solution was poured into water (100 mL) and extracted with ethyl acetate (50 mL×2), and a combined organic phase was washed with saturated sodium chloride solution (50 mL), dried with anhydrous sodium sulfate, filtered, and concentrated under reduced pressure. The obtained crude product was separated and purified by silica gel column chromatography (eluent: A system) to obtain 2-(2-fluoro-4-nitrophenyl)oxazole **7f** (105.00 mg), with a yield of 68.66%.
MS m/z (ESI): 209.0 [M+1]

Fifth step

2-(2-(4,4-difluoropiperidin-1-yl)-4-nitrophenyl)oxazole

At room temperature, 2-(2-fluoro-4-nitrophenyl)oxazole **7f** (125.00 mg, 0.60 mmol) was dissolved in dimethyl sulfoxide solution (2 mL), potassium carbonate (249.00 mg, 1.80 mmol) and 4,4-difluorpiperidine **1b** (145.28 mg, 1.20 mmol) were added, and the system was subjected to nitrogen purging for three times. The reaction was carried out at 120°C for 56 hours to obtain a yellow suspension. After mass spectrometry showed that the reaction of starting materials was complete, the reaction solution was poured into water (100 mL) and extracted with ethyl acetate (50 mL×2), and a combined organic phase was washed with saturated sodium chloride solution (50 mL), dried with anhydrous sodium sulfate, filtered, and concentrated under reduced pressure. The obtained crude product was separated and purified by silica gel column chromatography (eluent: A system) to obtain 2-(2-(4,4-difluoropiperidin-1-yl)-4-nitrophenyl)oxazole **7g** (92.00 mg), with a yield of 49.53%.
MS m/z (ESI): 310.2 [M+1]

Sixth step

3-(4,4-difluoropiperidin-1-yl)-4-(oxazol-2-yl)aniline

At room temperature, 2-(2-(4,4-difluoropiperidin-1-yl)-4-nitrophenyl)oxazole **7g** (50.00 mg, 0.16 mmol) was dissolved in ethyl acetate solution (1 mL), palladium on carbon (1.72 mg, 0.02 mmol) was added, and the system was subjected to hydrogen purging for three times. The reaction was carried out at room temperature under a hydrogen atmosphere for 18 hours to obtain a black suspension. After mass spectrometry showed that the reaction of starting materials was complete, the product was generated. The reaction solution was filtered and washed with ethyl acetate, and organic phases were combined and concentrated to dryness under reduced pressure to obtain 3-(4,4-difluoropiperidin-1-yl)-4-(oxazol-2-yl)aniline **7h** (44.00 mg). The crude product was directly used in the next step.

MS m/z (ESI): 280.2 [M+1]

Seventh step

N-(3-(4,4-difluoropiperidin-1-yl)-4-(oxazol-2-yl)phenyl)-4-(ethylsulfonamido)-2-(6-azaspiro[2.5 ]octan-6-yl)benzamide

At room temperature, 3-(4,4-difluoropiperidin-1-yl)-4-(oxazol-2-yl)aniline **7h** (40.00 mg,0.16 mmol) was dissolved in an N,N-dimethylformamide solution (1 mL), N,N-diisopropylethylamine (81.44 mg, 0.63 mmol), 4-(ethylsulfonamido)-2-(6-azaspiro[2.5]octan-6-yl)benzoic acid **7a** (53.32 mg, 0.16 mmol) and (7-azabenzotriazole-1-yloxy)tripyrrolidinophosphonium hexafluorophosphate (123.21 mg, 0.24 mmol) were sequentially added, and the system was subjected to nitrogen purging for three times. The reaction was carried out at 25°C for 18 hours to obtain a yellow suspension. After mass spectrometry showed that the reaction of starting materials was complete, the reaction solution was poured into water (100 mL) and extracted with ethyl acetate (40 mL×2), and a combined organic phase was washed with saturated sodium chloride solution (40 mL), dried with anhydrous sodium sulfate, filtered, and concentrated under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(oxazol-2-yl)phenyl)-4-(ethylsulfonamido)-2-(6-azaspiro[2.5 ]octan-6-yl)benzamide **7** (19.95 mg), with a yield of 21.12%.

MS m/z (ESI): 600.2 [M+1]
1H NMR (400 MHz, DMSO) δ 11.87 (s, 1H), 10.19 (s, 1H), 8.22 (d, J = 0.6 Hz, 1H), 7.85 (dd, J = 17.1, 8.4 Hz, 2H), 7.65 (d, J = 1.6 Hz, 1H), 7.58 (dd, J = 8.4, 1.8 Hz, 1H), 7.38 (d, J = 0.6 Hz, 1H), 7.17 (d, J = 1.9 Hz, 1H), 7.05 (dd, J = 8.5, 2.0 Hz, 1H), 3.20 (q, J = 7.3 Hz, 2H), 3.08 - 3.03 (m, 4H), 2.98 (t, J = 5.0 Hz, 4H), 2.15 (m, 4H), 1.55 (m, 4H), 1.21 (t, J = 7.2 Hz, 3H), 0.35 (s, 4H).

Example 8

N-(3-(4,4-difluoropiperidin-1-yl)-4-(pyridin-4-yl)phenyl)-4-(ethylsulfonamido)-2-(6-azaspiro[2. 5]octan-6-yl)benzamide

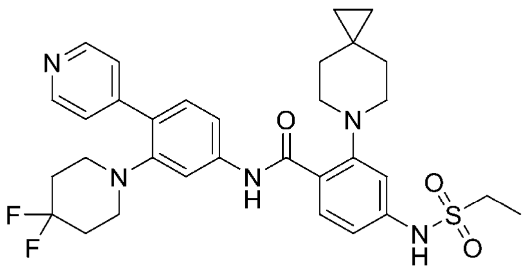

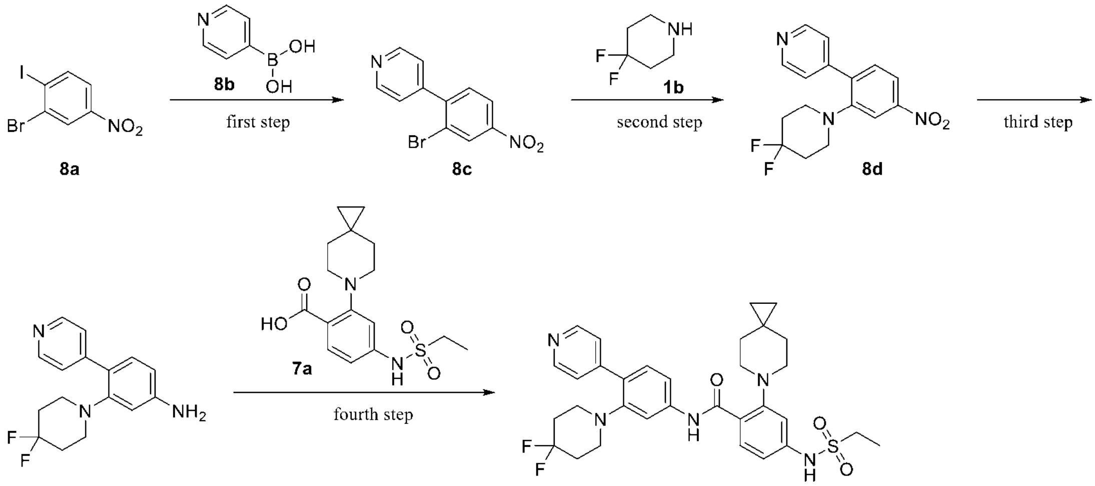

8b 1b 7a
8a 8c 8d
8e 8

First step

4-(2-bromo-4-nitrophenyl)pyridine

At room temperature, 2-bromo-1-iodo-4-nitrobenzene **8a** (500 mg, 1.52 mmol, commercially available) was added into toluene (5 mL), ethanol (1 mL) and water (2 mL), and pyridin-4-boronic acid **8b** (281 mg, 2.29 mmol, commercially available), tetrakis(triphenylphosphine)palladium (176 mg, 0.152 mmol) and sodium carbonate (323 mg, 3.05 mmol) were added into the above reaction solution, and subjected to nitrogen purging for three times. The reaction was carried out at 100°C for 18 hours. The mixture was filtered and concentrated to dryness under reduced pressure. The crude product was separated and purified by silica gel column chromatography (eluent: A system) to obtain 4-(2-bromo-4-nitrophenyl)pyridine **8c** (200 mg), with a yield of 47.0%.
MS m/z (ESI): 279.0 [M+1]

Second step

4-(2-(4,4-difluoropiperidin-1-yl)-4-nitrophenyl)pyridine

At room temperature, 4-(2-bromo-4-nitrophenyl)pyridine **8c** (100 mg, 0.358 mmol) and 4,4-difluorpiperidine **1b** (86.8 mg, 0.717 mmol) were added into 1,4-dioxane (3 mL), and palladium acetate (8.04 mg, 0.0358), cesium carbonate (233.49 mg, 0.717 mmol) and 4,5-bis(diphenylphosphino)-9,9-dimethylxanthene (41.5 mg, 0.0717 mmol) were added into the above reaction solution, and the system was subjected to nitrogen purging for three times. The reaction was carried out at 100°C for 18 hours. The mixture was concentrated to dryness under a reduced pressure. The crude product was separated and purified by silica gel column chromatography (eluent: A system) to obtain 4-(2-(4,4-difluoropiperidin-1-yl)-4-nitrophenyl)pyridine **8d** (50.0 mg), with a yield of 43.70%.
MS m/z (ESI): 320.2 [M+1]

Third step

3-(4,4-difluoropiperidin-1-yl)-4-(pyridin-4-yl)aniline

At room temperature, 4-(2-(4,4-difluoropiperidin-1-yl)-4-nitrophenyl)pyridine **8d** (50.0 mg, 0.157 mmol) was added into methanol (2 mL), then 10% palladium on carbon (16.7 mg, 0.157 mmol) was added, and the system was subjected to hydrogen purging for three times. The reaction was carried out at 25°C for 4 hours. The mixture was concentrated to dryness under reduced pressure to obtain 3-(4,4-difluoropiperidin-1-yl)-4-(pyridin-4-yl)aniline **8e** (45.0 mg), with a yield of 99.33%.
MS m/z (ESI): 290.2 [M+1]

Fourth step

N-(3-(4,4-difluoropiperidin-1-yl)-4-(pyridin-4-yl)phenyl)-4-(ethylsulfonamido)-2-(6-azaspiro[2. 5]octan-6-yl)benzamide

At room temperature, 3-(4,4-difluoropiperidin-1-yl)-4-(pyridin-4-yl)aniline **8e** (45.0 mg, 0.156 mmol) and 4-(ethylsulfonamido)-2-(6-azaspiro[2.5]octan-6-yl)benzoic acid **7a** (63.2 mg, 0.187 mmol) were added into N,N-dimethylformamide (2 mL), and (7-azabenzotriazole-1-yloxy)tripyrrolidinophosphonium hexafluorophosphate (122 mg, 0.233 mmol) and N,N-diisopropylethylamine (40.2 mg, 0.311 mmol) were added into the above reaction solution, and the system was subjected to nitrogen purging for three times. The reaction was carried out at 60°C for 3 hours. The mixture was poured into water (10 mL), the thus obtained mixture was extracted with ethyl acetate (10 mL×3), and a combined organic phase was washed with brine (10 mL), dried with anhydrous sodium sulfate, filtered, and concentrated to dryness under a reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(pyridin-4-yl)phenyl)-4-(ethylsulfonamido)-2-(6-azaspiro[2. 5]octan-6-yl)benzamide **8** (3.00 mg), with a yield of 4.75%.

MS m/z (ESI): 610.2 [M+1]
1H NMR (400 MHz, DMSO-d6) δ 11.84 (s, 1H), 10.19 (s, 1H), 8.67 (d, J = 5.2 Hz, 2H), 7.91 - 7.80 (m, 3H), 7.67 - 7.44 (m, 1H), 7.59 - 7.56 (m, 1H), 7.42 - 7.37 (m, 1H), 7.18 (d, J = 2.0 Hz, 1H), 7.07 - 7.03 (m, 1H), 3.23 - 3.18 (m, 2H), 3.00 - 2.96 (m, 4H), 2.94 - 2.89 (m, 4H), 1.61 - 1.52 (m, 4H), 1.24 - 1.22 (m, 4H), 1.22 - 1.19 (m, 3H), 0.36 (s, 4H).

Example 9

N-(3-(4,4-difluoropiperidin-1-yl)-4-(pyridin-3-yl)phenyl)-4-(ethylsulfonamido)-2-(6-azaspiro[2. 5]octan-6-yl)benzamide

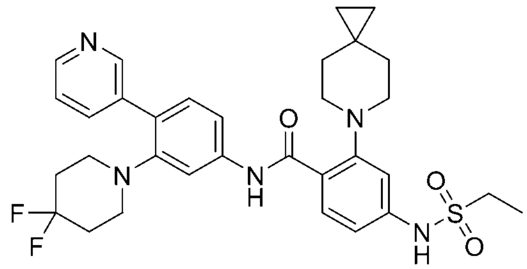

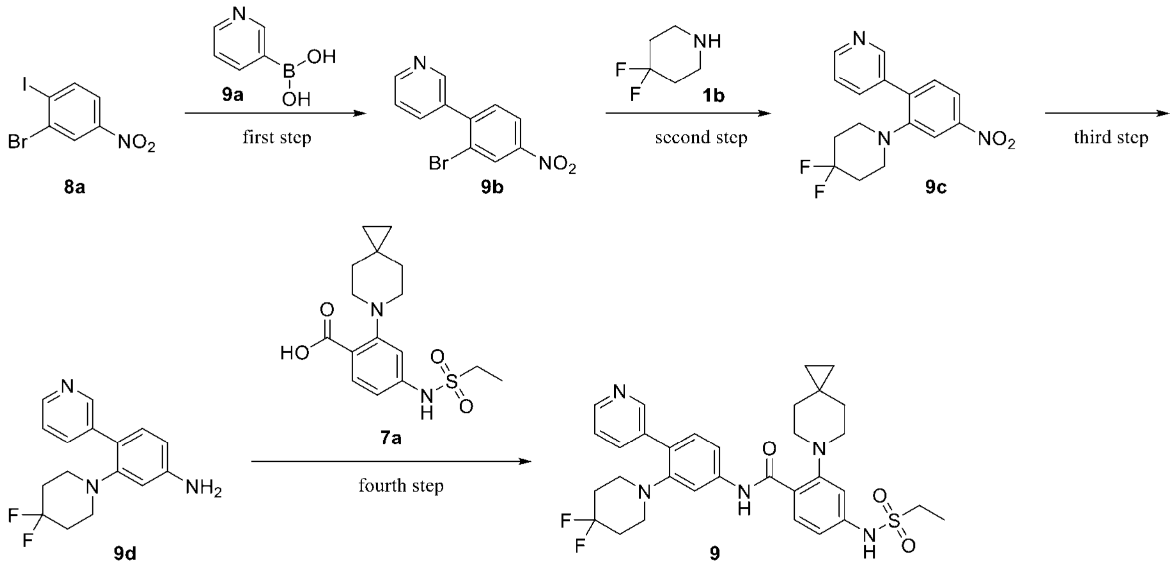


First step

3-(2-bromo-4-nitrophenyl)pyridine

At room temperature, 2-bromo-1-iodo-4-nitrobenzene **8a** (500 mg, 1.52 mmol, commercially available) was added into toluene (5 mL), ethanol (1 mL) and water (2 mL), then pyridin-3-boronic acid **9a** (469 mg, 2.29 mmol, commercially available), tetrakis(triphenylphosphine)palladium (176 mg, 0.152 mmol) and sodium carbonate (323 mg, 3.05 mmol) were added, and the system was subjected to nitrogen purging for three times. The reaction was carried out at 100°C for 18 hours. The mixture was filtered and concentrated to dryness under reduced pressure. The crude product was separated and purified by silica gel column chromatography (eluent: A system) to obtain 3-(2-bromo-4-nitrophenyl) pyridine **9b** (150 mg), with a yield of 35.25%.
MS m/z (ESI): 279.0 [M+1]

Second step

3-(2-(4,4-difluoropiperidin-1-yl)-4-nitrophenyl)pyridine

At room temperature, 3-(2-bromo-4-nitrophenyl)pyridine **9b** (150 mg, 0.537 mmol) and 4,4-difluorpiperidine **1b** (130.20 mg, 1.07 mmol) were added into 1,4-dioxane (3 mL), and palladium acetate (12.1 mg, 0.0538 mmol), cesium carbonate (350 mg, 1.07 mmol) and 4,5-bis(diphenylphosphino)-9,9-dimethylxanthene (62.2 mg, 0.107 mmol) were added into the above reaction solution, and the system was subjected to nitrogen purging for three times. The reaction was carried out at 100°C for 18 hours. The mixture was concentrated to dryness under reduced pressure. The crude product was separated and purified by silica gel column chromatography (eluent: A system) to obtain 3-(2-(4,4-difluoropiperidin-1-yl)-4-nitrophenyl)pyridine **9c** (50.0 mg), with a yield of 29.14%.
MS m/z (ESI): 320.0 [M+1]

Third step

3-(4,4-difluoropiperidin-1-yl)-4-(pyridin-3-yl)aniline

At room temperature, 3-(2-(4,4-difluoropiperidin-1-yl)-4-nitrophenyl)pyridine **9c** (50.0 mg, 0.157 mmol) was added into methanol (2 mL), then 10% palladium on carbon (16.7 mg, 0.157 mmol) was added, and the system was subjected to hydrogen purging for three times. The reaction was carried out at 25°C for 4 hours. The reaction solution was filtered, and the filtrate was concentrated to dryness under reduced pressure to obtain 3-(4,4-difluoropiperidin-1-yl)-4-(pyridin-3-yl)aniline **9d** (45.0 mg), with a yield of 99.33%.
MS m/z (ESI): 290.2 [M+1]

Fourth step

N-(3-(4,4-difluoropiperidin-1-yl)-4-(pyridin-3-yl)phenyl)-4-(ethylsulfonamido)-2-(6-azaspiro[2. 5]octan-6-yl)benzamide

At room temperature, 3-(4,4-difluoropiperidin-1-yl)-4-(pyridin-3-yl)aniline **9d** (45.0 mg, 0.156 mmol) and 4-(ethylsulfonamido)-2-(6-azaspiro[2.5]octan-6-yl)benzoic acid **7a** (63.2 mg, 0.187 mmol) were added into N,N-dimethylformamide (2 mL), then (7-azabenzotriazole-1-yloxy)tripyrrolidinophosphonium hexafluorophosphate (122 mg, 0.233 mmol) and N,N-diisopropylethylamine (40.2 mg, 0.311 mmol) were added, and the system was subjected to nitrogen purging for three times. The reaction was carried out at 60°C for 3 hours. The mixture was poured into water (10 mL), the thus obtained mixture was extracted with ethyl acetate (10 mL×3), and a combined organic phase was washed with saturated sodium chloride solution (10 mL), dried with anhydrous sodium sulfate, filtered, and concentrated to dryness under reduced pressure. The crude product was separated by a preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(pyridin-3-yl)phenyl)-4-(ethylsulfonamido)-2-(6-azaspiro[2. 5]octan-6-yl)benzamide **9** (5.00 mg), with a yield of 5.27%.

MS m/z (ESI): 610.2 [M+1]
1H NMR (400 MHz, DMSO-d6) δ 11.84 (s, 1H), 10.24 (s, 1H), 8.82 (d, J = 2.0 Hz, 1H), 8.54 - 8.50 (m, 1H), 8.14 - 8.17 (m, 1H), 7.85 (d, J = 8.4 Hz, 1H), 7.64 (d, J = 2.0 Hz, 1H), 7.58 - 7.54 (m, 1H), 7.48 - 7.44 (m, 1H), 7.32 (d, J = 8.0 Hz, 1H), 7.18 (d, J = 2.0 Hz, 1H), 7.07 - 7.02 (m, 1H), 3.22 - 3.17 (m, 2H), 3.02 - 2.96 (m, 4H), 2.91 - 2.85 (m, 4H), 1.95 - 1.84 (m, 4H), 1.63 - 1.51 (m, 4H), 1.21 (t, J = 7.2 Hz, 3H), 0.36 (s, 4H).

Example 10

N-(3-(4,4-difluoropiperidin-1-yl)-4-(pyridin-2-yl)phenyl)-4-(ethylsulfonamido)-2-(6-azaspiro[2. 5]octan-6-yl)benzamide

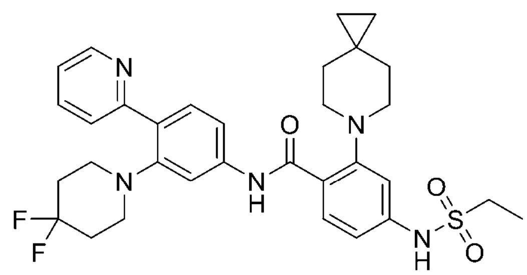

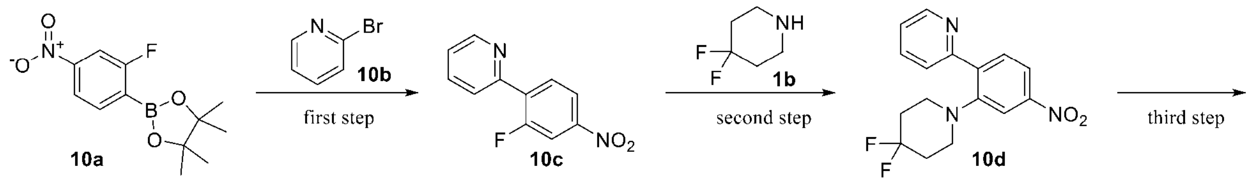

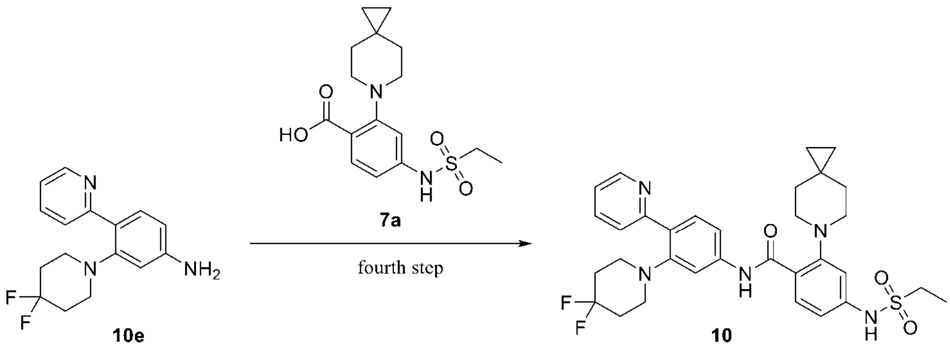


First step

2-(2-fluoro-4-nitrophenyl)pyridine

At room temperature, 2-(2-fluoro-4-nitrophenyl)-4,4,5,5-tetramethyl-1,3,2-dioxaborolane **10a** (800 mg, 3.00 mmol, commercially available) was added into 1,4-dioxane (10 mL) and water (1 mL), then 2-bromopyridine **10b** (710 mg, 4.49 mmol, commercially available), tetrakis(triphenylphosphine)palladium (346. mg, 0.300 mmol) and potassium carbonate (828 mg, 5.99 mmol) were added, and the system was subjected to nitrogen purging for three times. The reaction was carried out at 100°C for 18 hours. The mixture was filtered and concentrated to dryness under reduced pressure. The crude product was separated and purified by silica gel column chromatography (eluent: A system) to obtain 2-(2-fluoro-4-nitrophenyl)pyridine **10c** (500 mg), with a yield of 76.50%.
MS m/z (ESI): 280.0 [M+1]

Second step

2-(2-(4,4-difluoropiperidin-1-yl)-4-nitrophenyl)pyridine

At room temperature, 2-(2-fluoro-4-nitrophenyl)pyridine **10c** (300 mg, 1.37 mmol) was added into dimethyl sulfoxide (3 mL), then triethylamine (278 mg, 2.75 mmol) and 4,4-difluorpiperidine **1b** (666 mg, 5.50 mmol) were added, and the system was subjected to nitrogen purging for three times. The reaction was carried out at 100°C for 18 hours. The mixture was poured into water (10 mL) and extracted with ethyl acetate (10 mL×3), and a combined organic phase was washed with a saturated sodium chloride solution (10 mL), dried with anhydrous sodium sulfate, filtered, and concentrated to dryness under reduced pressure. The crude product was separated and purified by silica gel column chromatography (eluent: A system) to obtain 2-(2-(4,4-difluoropiperidin-1-yl)-4-nitrophenyl)pyridine **10d** (150 mg), with a yield of 34.17%.
MS m/z (ESI): 320.0 [M+1]

Third step

3-(4,4-difluoropiperidin-1-yl)-4-(pyridin-2-yl)aniline

At room temperature, 2-(2-(4,4-difluoropiperidin-1-yl)-4-nitrophenyl)pyridine **10d** (130 mg, 0.407 mmol) was added into anhydrous methanol (2 mL), then 10% palladium on carbon (43.3 mg, 0.407 mmol) was added, and the system was subjected to hydrogen purging for three times. The reaction was carried out at 25°C for 4 hours. The mixture was concentrated to dryness under reduced pressure to obtain 3-(4,4-difluoropiperidin-1-yl)-4-(pyridin-2-yl)aniline **10e** (110 mg), with a yield of 93.38%.
MS m/z (ESI): 290.2 [M+1]

Fourth step

N-(3-(4,4-difluoropiperidin-1-yl)-4-(pyridin-2-yl)phenyl)-4-(ethylsulfonamido)-2-(6-azaspiro[2. 5]octan-6-yl)benzamide

At room temperature, 3-(4,4-difluoropiperidin-1-yl)-4-(pyridin-2-yl)aniline **10e** (110 mg, 0.380 mmol) and 4-(ethylsulfonamido)-2-(6-azaspiro[2.5]octan-6-yl)benzoic acid **7a** (154 mg, 0.456 mmol) were added into N,N-dimethylformamide (2 mL), then (7-azabenzotriazole-1-yloxy)tripyrrolidinophosphonium hexafluorophosphate (297 mg, 0.570 mmol) and N,N-diisopropylethylamine (98.3 mg, 0.760 mmol) were added, and the system was subjected to nitrogen purging for three times. The reaction was carried out at 60°C for 3 hours. The mixture was poured into water (10 mL) and

extracted with ethyl acetate (10 mL×3), and a combined organic phase was washed with saturated sodium chloride solution (10 mL), dried with anhydrous sodium sulfate, filtered, and concentrated to dryness under reduced pressure. The crude product was separated by a preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(pyridin-2-yl)phenyl)-4-(ethylsulfonamido)-2-(6-azaspiro[2. 5]octan-6-yl)benzamide **10** (5.00 mg), with a yield of 2.16%.

MS m/z (ESI): 610.4 [M+1]

1H NMR (400 MHz, DMSO-d6) δ 11.89 (s, 1H), 8.67 - 8.64 (m, 1H), 8.11 (d, J = 8.0 Hz, 1H), 7.86 - 7.81 (m, 2H), 7.62 - 7.59 (m, 1H), 7.58 - 7.54 (m, 2H), 7.31 - 7.28 (m, 1H), 7.16 (d, J = 2.0 Hz, 1H), 7.06 - 7.01 (m, 1H), 3.21 - 3.15 (m, 2H), 3.01 - 2.96 (m, 4H), 2.95 - 2.89 (m, 4H), 1.58 (s, 4H), 1.28 - 1.23 (m, 4H), 1.20 - 1.18 (m, 3H), 0.36 (s, 4H).

Examples 11-19 were synthesized according to the synthesis methods in Examples 9-10 of the present invention, and structures and characterization data were as shown in the following table:

| Serial number and structure of example | MS m/z (ESI) | $^1$H NMR |
|---|---|---|
| 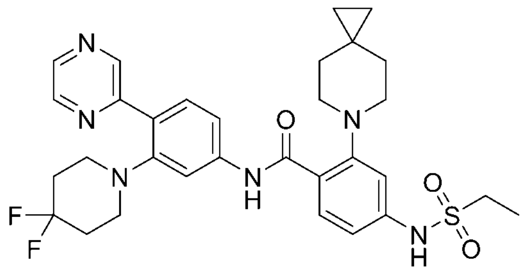 **11** | 611.4 [M+1] | $^1$H NMR (400 MHz, DMSO-$d_6$) δ 11.85 (s, 1H), 10.15 (s, 1H), 9.30 (d, *J* = 1.6 Hz, 1H), 8.74 - 8.71 (m, 1H), 8.53 (d, *J* = 2.8 Hz, 1H), 7.84 (d, *J* = 8.4 Hz, 1H), 7.69 (d, *J* = 1.6 Hz, 1H), 7.64 - 7.61 (m, 1H), 7.60 - 7.57 (m, 1H), 7.18 (d, *J* = 2.0 Hz, 1H), 7.07 - 7.03 (m, 1H), 3.23 - 3.17 (m, 2H), 3.01 - 2.97 (m, 4H), 2.95 - 2.91 (m, 4H), 2.02 - 1.92 (m, 4H), 1.62 - 1.54 (m, 4H), 1.21 (t, *J* = 7.6 Hz, 3H), 0.36 (s, 4H). |
| 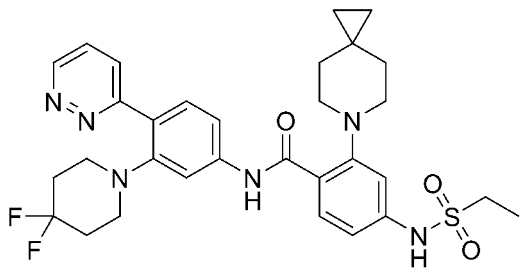 **12** | 611.2 [M+1] | $^1$H NMR (400 MHz, DMSO-$d_6$) δ 11.89 (s, 1H), 10.39 (s, 1H), 9.16 (d, *J* = 4.8, 1.6 Hz, 1H), 8.32 (d, *J* = 8.4, 1.5 Hz, 1H), 7.85 (d, *J* = 8.4 Hz, 1H), 7.74 - 7.68 (m, 2H), 7.67 - 7.58 (m, 2H), 7.18 (s, 1H), 7.04 (d, *J* = 8.4, 2.0 Hz, 1H), 3.24 - 3.12 (m, 2H), 3.04 - 2.96 (m, 4H), 2.94 - 2.86 (m, 4H), 2.03 - 1.85 (m, 4H), 1.58 (s, 4H), 1.21 (t, *J* = 7.2 Hz, 3H), 0.36 (s, 4H). |
| 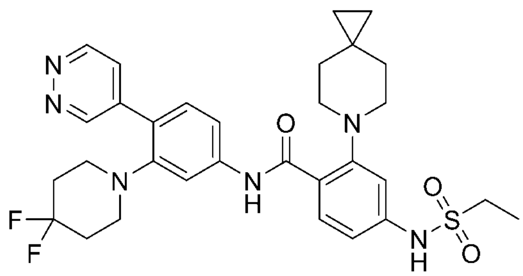 **13** | 611.2 [M+1] | $^1$H NMR (400 MHz, DMSO-$d_6$) δ 11.89 (s, 1H), 10.36 (s, 1H), 9.58 (s, 1H), 9.25 (d, *J* = 5.6, 1.1 Hz, 1H), 8.04 - 7.95 (m, 1H), 7.84 (d, *J* = 8.4 Hz, 1H), 7.72 (s, 1H), 7.60 (d, *J* = 8.4, 1.7 Hz, 1H), 7.46 (d, *J* = 8.4 Hz, 1H), 7.17 (s, 1H), 7.04 (d, *J* = 8.4, 2.0 Hz, 1H), 3.24 - 3.13 (m, 2H), 3.02 - 2.94 (m, 4H), 2.94 - 2.84 (m, 4H), 2.05 - 1.84 (m, 4H), 1.57 (s, 4H), 1.21 (t, *J* = 7.2 Hz, 3H), 0.36 (s, 4H). |

(continued)

| Serial number and structure of example | MS m/z (ESI) | $^1$H NMR |
|---|---|---|
| 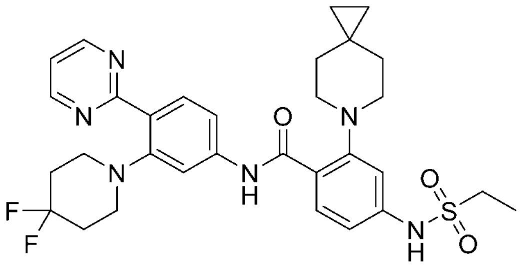 14 | 611.2 [M+1] | $^1$H NMR (400 MHz, DMSO-$d_6$) δ 11.84 (s, 1H), 10.28 (s, 1H), 8.91 (d, *J* = 4.8 Hz, 2H), 7.85 (d, *J* = 8.4 Hz, 1H), 7.67 (d, *J* = 8.4 Hz, 1H), 7.62 - 7.53 (m, 2H), 7.41 (t, *J* = 4.8 Hz, 1H), 7.18 (s, 1H), 7.06 (d, *J* = 8.4, 1.9 Hz, 1H), 3.26 - 3.14 (m, 2H), 3.09 - 2.90 (m, 8H), 2.04 - 1.87 (m, 4H), 1.58 (s, 4H), 1.22 (t, *J*= 7.2 Hz, 3H), 0.36 (s, 4H). |
| 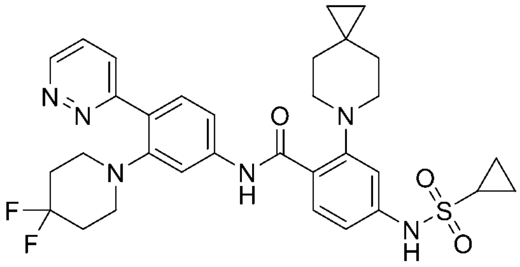 15 | 623.4 [M+1] | $^1$H NMR (400 MHz, DMSO-$d_6$) δ 11.92 (s, 1H), 10.15 (s, 1H), 9.16 (dd, *J* = 4.8, 1.6 Hz, 1H), 8.32 (dd, *J* = 8.6, 1.6 Hz, 1H), 7.86 (d, *J* = 8.6 Hz, 1H), 7.75-7.67 (m, 2H), 7.67 - 7.60 (m, 2H), 7.22 (d, *J* = 2.0 Hz, 1H), 7.08 (dd, *J* = 8.6, 2.0 Hz, 1H), 3.02 - 2.88 (m, 8H), 2.80-2.70 (m, 1H), 2.03-1.90 (m, 4H), 1.64-1.50 (m, 4H), 1.02 - 0.97 (m, 4H), 0.37 (s, 4H). |
| 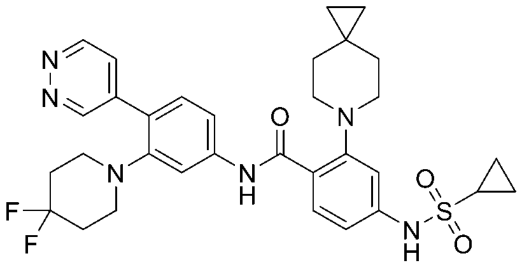 16 | 623.4 [M+1] | $^1$H NMR (400 MHz, DMSO-$d_6$) δ 11.95 (s, 1H), 10.22 (s, 1H), 9.60 - 9.55 (m, 1H), 9.29 - 9.20 (m, 1H), 8.01 - 7.97 (m, 1H), 7.85 (d, *J* = 8.4 Hz, 1H), 7.71 (d, *J* = 2.0 Hz, 1H), 7.68 - 7.58 (m, 1H), 7.46 (d, *J* = 8.4 Hz, 1H), 7.21 (d, *J* = 2.0 Hz, 1H), 7.09 - 7.03 (m, 1H), 3.02 - 2.96 (m, 4H), 2.94 - 2.88 (m, 4H), 2.77 - 2.71 (m, 1H), 2.01 - 1.90 (m, 4H), 1.66 - 1.50 (m, 4H), 1.03 - 0.97 (m, 4H), 0.36 (s, 4H). |
| 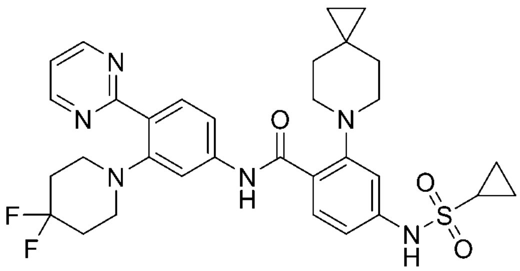 17 | 623.4 [M+1] | $^1$H NMR (400 MHz, DMSO-$d_6$) δ 11.91 (s, 1H), 10.15 (s, 1H), 8.94 (d, *J* = 4.8 Hz, 2H), 7.87 (d, *J* = 8.4 Hz, 1H), 7.76 (d, *J* = 8.4 Hz, 1H), 7.66 (s, 1H), 7.61 (d, *J* = 8.4, 1.7 Hz, 1H), 7.44 (t, *J* = 4.8 Hz, 1H), 7.22 (d, *J* = 2.0 Hz, 1H), 7.12 - 7.01 (m, 1H), 3.12 - 3.04 (m, 4H), 3.04 - 2.95 (m, 4H), 2.84 - 2.71 (m, 1H), 2.11 - 1.89 (m, 4H), 1.59 (s, 4H), 1.08 - 0.93 (m, 4H), 0.37 (s, 4H). |
| 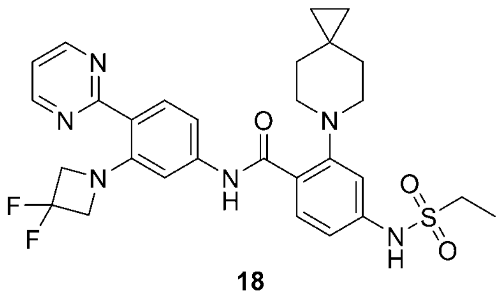 18 | 583.3 [M+1] | |

(continued)

| Serial number and structure of example | MS m/z (ESI) | $^1$H NMR |
|---|---|---|
| 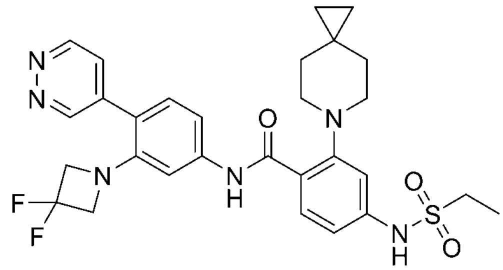 19 | 583.4 [M+1] | $^1$H NMR (400 MHz, DMSO-$d_6$) δ 11.81 (s, 1H), 9.33 - 9.28 (m, 1H), 9.24 (d, $J$ = 5.2 Hz, 1H), 7.82 (d, $J$ = 8.4 Hz, 1H), 7.73 - 7.63 (m, 1H), 7.43 (s, 1H), 7.38 - 7.30 (m, 2H), 7.17 - 7.12 (m, 1H), 7.05 - 6.99 (m, 1H), 4.02 - 3.91 (m, 4H), 3.20 - 3.13 (m, 2H), 3.02 - 2.95 (m, 4H), 1.63 - 1.50 (m, 4H), 1.20 (t, $J$ = 7.2 Hz, 3H), 0.36 (s, 4H). |

Example 20

N-(3-(4,4-difluoropiperidin-1-yl)-4-(1H-imidazol-1-yl)phenyl)-4-(ethylsulfonamido)-2-(6-azaspi ro[2.5]octan-6-yl)benzamide

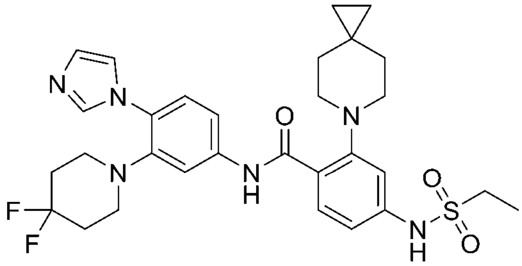

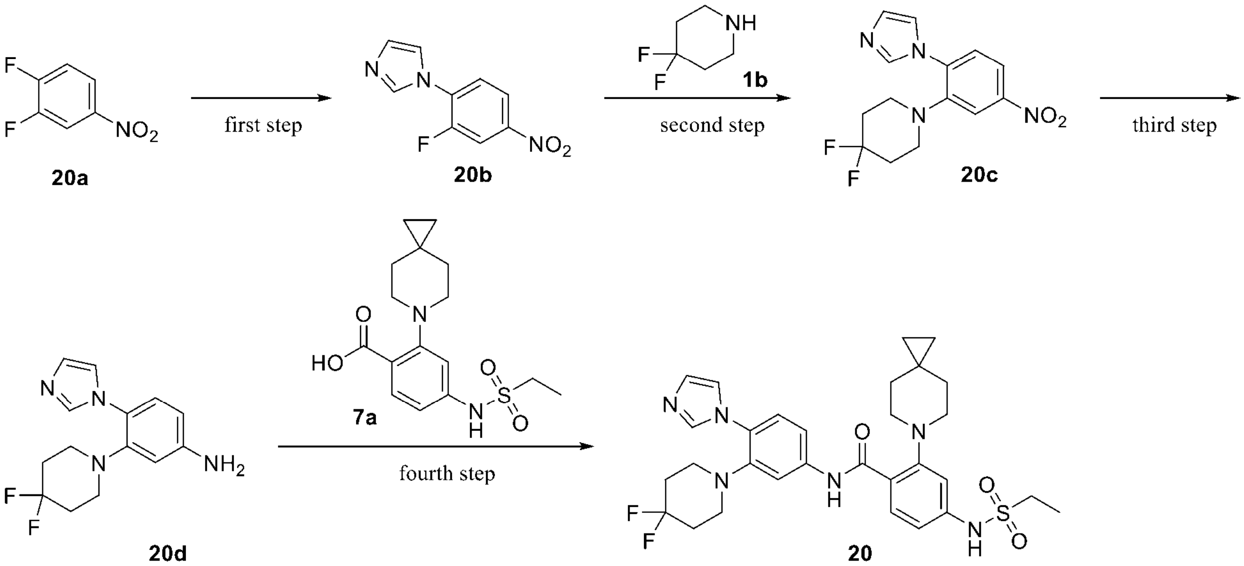


First step

1-(2-fluoro-4-nitrophenyl)-1H-imidazole

At 25°C, imidazole (1.18 g, 17.3 mmol) and potassium carbonate (6.5 g, 47.1 mmol) were added to an N,N-dimethylformamide solution (20 mL) of 1,2-difluoro-4-nitrobenzene **20a** (2.5 g, 15.7 mmol, commercially available) to react

at 70°C for 18 hours. The mixture was poured into water (100 mL) and extracted with ethyl acetate (100 mL×3), and a combined organic phase was washed with saturated sodium chloride solution (50 mL), dried with anhydrous sodium sulfate, filtered, and concentrated under reduced pressure to obtain 1-(2-fluoro-4-nitrophenyl)-1H-imidazole **20b** (2.00 g), with a yield of 61.4%. The crude product was directly used in the next step of reaction.
MS m/z (ESI): 208.0 [M+1]

Second step

1-(2-(1H-imidazol-1-yl)-5-nitrophenyl)-4,4-difluoropiperidine

At 25°C, potassium carbonate (4.00 g, 29.0 mmol) and 4,4-difluorpiperidine **1b** (3.51 g, 29.0 mmol) were added to a dimethyl sulfoxide solution (20 mL) of 1-(2-fluoro-4-nitrophenyl)-1H-imidazole **20b** (2.00 g, 9.65 mmol) , and the system was reacted at 130°C for 18 hours. The mixture was poured into water (200 mL) and extracted with ethyl acetate (200 mL×3), and a combined organic phase was washed with saturated sodium chloride solution (50 mL), dried with anhydrous sodium sulfate, filtered, and concentrated under reduced pressure. The crude product was separated and purified by silica gel column chromatography (eluent: A system) to obtain 1-(2-(1H-imidazol-1-yl)-5-nitrophenyl)-4,4-difluoropiperidine **20c** (800 mg), with a yield of 26.9%.
MS m/z (ESI): 309.2 [M+1]

Third step

3-(4,4-difluoropiperidin-1-yl)-4-(1H-imidazol-1-yl)aniline

At 25°C, 10% wet palladium on carbon (30.0 mg, 0.282 mmol) was added to a methanol solution (3 mL) of 1-(2-(1H-imidazol-1-yl)-5-nitrophenyl)-4,4-difluoropiperidine **20c** (100 mg, 0.324 mmol), and the system was subjected to hydrogen purging for three times. The reaction was carried out at 25°C for 4 hours. The mixture was filtered and then concentrated under reduced pressure to obtain 3-(4,4-difluoropiperidin-1-yl)-4-(1H-imidazol-1-yl)aniline **20d** (90.0 mg). The crude product was directly used in the next step of reaction.
MS m/z (ESI): 279.1 [M+1]

Fourth step

N-(3-(4,4-difluoropiperidin-1-yl)-4-(1H-imidazol-1-yl)phenyl)-4-(ethylsulfonamido)-2-(6-azaspi ro[2.5]octan-6-yl)benzamide

At 25°C, 3-(4,4-difluoropiperidin-1-yl)-4-(1H-imidazol-1-yl)aniline **20d** (80.0 mg, 0.287 mmol) and 4-(ethylsulfonamido)-2-(6-azaspiro[2.5]octan-6-yl)benzoic acid **7a** (146 mg, 0.431 mmol) were added into N,N-dimethylformamide (5 mL), and then (7-azabenzotriazole-1-yloxy)tripyrrolidinophosphonium hexafluorophosphate (300 mg, 0.575 mmol) and N,N-diisopropylethylamine (149 mg, 1.15 mmol) were added to react at 65°C for 2 hours. The mixture was poured into water (50 mL) and the thus obtained mixture was extracted with ethyl acetate (100 mL×3), and a combined organic phase was washed with saturated sodium chloride solution (50 mL), dried with anhydrous sodium sulfate, filtered, and concentrated under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(1H-imidazol-1-yl)phenyl)-4-(ethylsulfonamido)-2-(6-azaspi ro[2.5]octan-6-yl)benzamide **20** (50.0 mg), with a yield of 29%.

MS m/z (ESI): 599.4 [M+1]
$^1$H NMR (400 MHz, DMSO-$d_6$) δ 11.79 (s, 1H), 10.17 (s, 1H), 8.02 (s, 1H), 7.83 (d, *J* = 8.4 Hz, 1H), 7.70 (d, *J* = 2.4 Hz, 1H), 7.60 - 7.50 (m, 2H), 7.34 (d, *J* = 8.4 Hz, 1H), 7.18 (d, *J* = 2.0 Hz, 1H), 7.10 (s, 1H), 7.05 (dd, *J* = 8.4, 2.0 Hz, 1H), 3.20 (q, *J* = 7.2 Hz, 2H), 2.98 (t, *J* = 5.2 Hz, 4H), 2.79 (t, *J* = 5.6 Hz, 4H), 2.05 - 1.91 (m, 4H), 1.55 (d, *J* = 5.2 Hz, 4H), 1.21 (t, *J* = 7.2 Hz, 3H), 0.35 (s, 4H).

Example 21

N-(3-(4,4-difluorocyclohexyl)-4-(1H-imidazol-1-yl)phenyl)-4-(ethylsulfonamido)-2-(6-azaspiro[ 2.5]octan-6-yl)benzamide

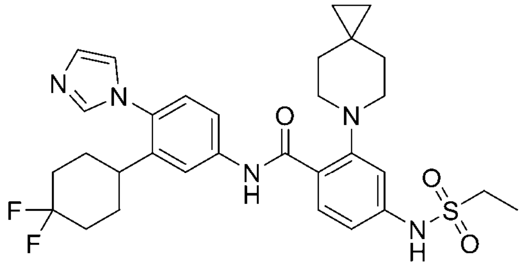

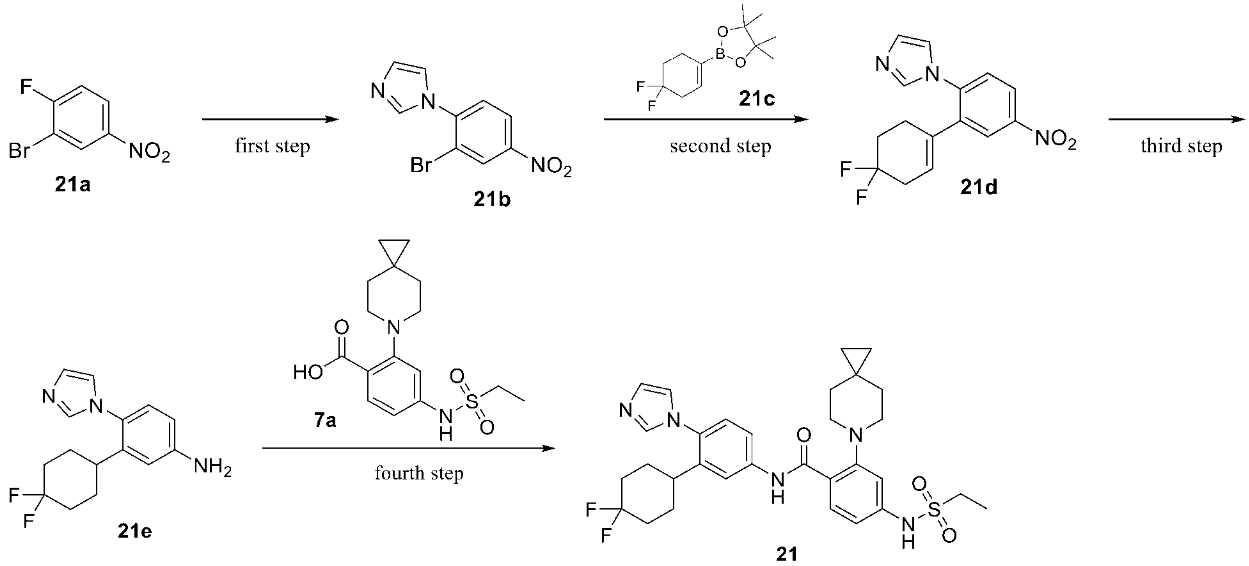


First step

1-(2-bromo-4-nitrophenyl)-1H-imidazole

At room temperature, 2-bromo-1-fluoro-4-nitrobenzene **21a** (1.00 g, 4.55 mmol, commercially available) was added into N,N-dimethylformamide (10 mL), then imidazole (619 mg, 9.09 mmol) and potassium carbonate (1.26 g, 9.09 mmol) were added, and the system was subjected to nitrogen purging for three times. The reaction was carried out at 100°C for 18 hours. The mixture was filtered and concentrated to dryness under reduced pressure. The crude product was separated and purified by silica gel column chromatography (eluent: A system) to obtain 1-(2-bromo-4-nitrophenyl)-1H-imidazole **21b** (300 mg), with a yield of 24.6%.
MS m/z (ESI): 269.0 [M+1]

Second step

1-(4',4'-difluoro-5-nitro-2',3',4',5'-tetrahydro-[1,1'-biphenyl]-2-yl)-1H-imidazole

At room temperature, 1-(2-bromo-4-nitrophenyl)-1H-imidazole **21b** (200 mg, 746.08 μmol) and 2-(4,4-difluorocyclohex-1-en-1-yl)-4,4,5,5-tetramethyl-1,3,2-dioxaborolane **21c** (273 mg, 1.12 mmol, commercially available) were added into 1,4-dioxane (3 mL) and water (1 mL), then [1,1'-bis(diphenylphosphino)ferrocene]dichloropalladium(II) dichloromethane complex (54.6 mg, 0.0746 mmol) and sodium carbonate (158 mg, 1.49 mmol) were added, and the system was subjected to nitrogen purging for three times. The reaction was carried out at 100°C for 18 hours. The mixture was poured into water (10 mL), the thus obtained mixture was extracted with ethyl acetate (10 mL×3), and a combined organic phase was washed with saturated sodium chloride solution (10 mL), dried with anhydrous sodium sulfate, filtered, and concentrated to dryness under reduced pressure to obtain 1-(4',4'-difluoro-5-nitro-2',3',4',5'-tetrahydro-[1,1'-biphenyl]-2-yl)-1H-imidazole **21d** (130 mg), with a yield of 57.1%.
MS m/z (ESI): 306.0 [M+1]

Third step

3-(4,4-difluorocyclohexyl)-4-(1H-imidazol-1-yl)aniline

At room temperature, 1-(4',4'-difluoro-5-nitro-2',3',4',5'-tetrahydro-[1,1'-biphenyl]-2-yl)-1H-imidazole **21d** (150 mg, 0.491 mmol) was added into anhydrous methanol (3 mL) and acetic acid (0.5 mL), then 10% palladium on carbon (26.1 mg, 0.246 mmol) was added, and the system was subjected to hydrogen purging for three times. The reaction was carried out at 25°C for 4 hours. The mixture was concentrated to dryness under reduced pressure to obtain 3-(4,4-difluorocyclohexyl)-4-(1H-imidazol-1-yl)aniline **21e** (50.0 mg), with a yield of 36.9%.
MS m/z (ESI): 278.2 [M+1]

Fourth step

N-(3-(4,4-difluorocyclohexyl)-4-(1H-imidazol-1-yl)phenyl)-4-(ethylsulfonamido)-2-(6-azaspiro[ 2.5]octan-6-yl)benzamide

At room temperature, 3-(4,4-difluorocyclohexyl)-4-(1H-imidazol-1-yl)aniline **21e** (30 mg, 0.108 mmol) and 4-(ethylsulfonamido)-2-(6-azaspiro[2.5]octan-6-yl)benzoic acid **7a** (43.9 mg, 0.130 mmol) were added into N,N-dimethylformamide (2 mL), then (7-azabenzotriazole-1-yloxy)tripyrrolidinophosphonium hexafluorophosphate (84.6 mg, 0.162 mmol) and N,N-diisopropylethylamine (28.0 mg, 0.216 mmol) were added, and the system was subjected to nitrogen purging for three times. The reaction was carried out at 60°C for 3 hours. The mixture was poured into water (10 mL) and extracted with ethyl acetate (10 mL×3), and a combined organic phase was washed with anhydrous sodium chloride solution (10 mL), dried with anhydrous sodium sulfate, filtered, and concentrated to dryness under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluorocyclohexyl)-4-(1H-imidazol-1-yl)phenyl)-4-(ethylsulfonamido)-2-(6-azaspiro[ 2.5]octan-6-yl)benzamide **21** (2 mg), with a yield of 3.1%.

MS m/z (ESI): 598.3 [M+1]
$^1$H NMR (400 MHz, DMSO-$d_6$) δ 11.90 (s, 1H), 10.17 (s, 1H), 7.90 - 7.83 (m, 2H), 7.82 - 7.80 (m, 1H), 7.77 - 7.75 (m, 1H), 7.40 - 7.36 (m, 1H), 7.31 (d, *J* = 8.8 Hz, 1H), 7.19 (d, *J* = 2.0 Hz, 1H), 7.11 (t, *J* = 1.1 Hz, 1H), 7.07 - 7.04 (m, 1H), 3.29 (s, 2H), 3.23 - 3.17 (m, 2H), 3.00 - 2.96 (m, 4H), 2.11 - 2.01 (m, 2H), 1.86 - 1.80 (m, 2H), 1.71 - 1.66 (m, 2H), 1.59 - 1.52 (m, 4H), 1.22-1.20 (m, 4H), 0.35 (s, 4H).

Example 22 was synthesized according to the synthesis method in Example 21 of the present invention, and a structure and characterization data were as shown in the following table:

| Serial number of structure of example | MS m/z (ESI) | $^1$H NMR |
|---|---|---|
| 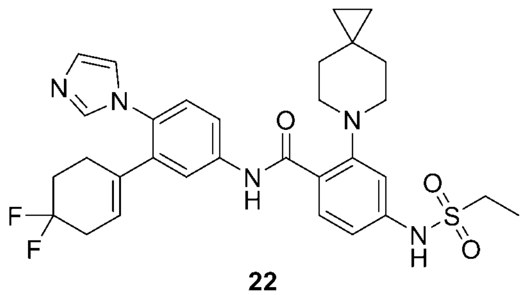 **22** | 596.2 [M+1] | $^1$H NMR (400 MHz, DMSO-$d_6$) δ 11.67 (s, 1H), 10.14 (s, 1H), 7.95 (d, *J* = 2.4 Hz, 1H), 7.81 - 7.76 (m, 2H), 7.68 - 7.62 (m, 1H), 7.41 (d, *J* = 8.8 Hz, 1H), 7.33 - 7.31 (m, 1H), 7.16 (d, *J* = 2.0 Hz, 1H), 7.06 - 7.01 (m, 2H), 5.58 (s, 1H), 3.22 - 3.16 (m, 2H), 3.01 - 2.96 (m, 4H), 2.68 - 2.58 (m, 2H), 1.98 - 1.94 (m, 4H), 1.57 - 1.50 (m, 4H), 1.21 (t, *J* = 7.6 Hz, 3H), 0.35 (s, 4H). |

Example 23

N-(3-(3,3-difluorocyclobutoxy)-4-(oxazol-2-yl)phenyl)-4-(ethylsulfonamido)-2-(6-azaspiro[2.5] octan-6-yl)benzamide

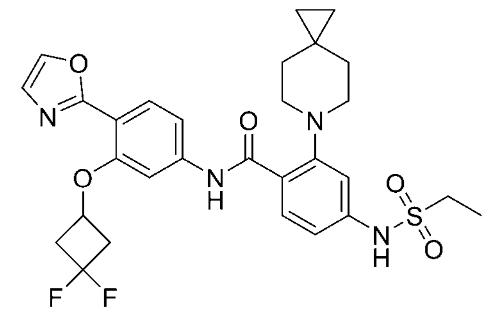

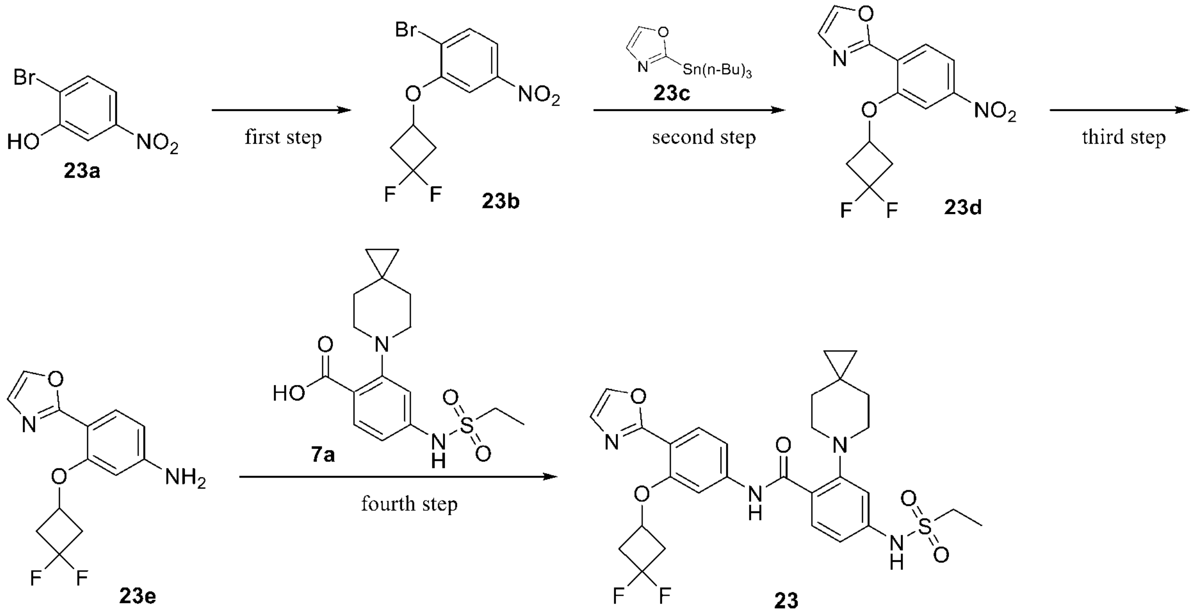

23a 23b 23d 23e 23

First step

1-bromo-2-(3,3-difluorocyclobutoxy)-4-nitrobenzene

2-bromo-5-nitrophenol **23a** (2.00 g, 9.17 mmol) was dissolved in a tetrahydrofuran solution (15 mL), triphenylphosphine (2.78 g, 13.76 mmol) was added, the system was subjected to nitrogen purging for three times, then **1a** (1.49 g, 13.76 mmol) and diisoethyl azodicarboxylate (2.78 g, 13.76 mmol) were added dropwise to react at 25°C for 18 hours. After mass spectrometry showed that the reaction of the starting materials was complete, the product was generated. The reaction solution was diluted with water (150 mL) and extracted with ethyl acetate (60 mL×3), and a combined organic phase was washed with saturated sodium chloride solution (60 mL), dried with anhydrous sodium sulfate, filtered, and concentrated under reduced pressure. The crude product was separated and purified by silica gel column chromatography (eluent: A system) to obtain 1-bromo-2-(3,3-difluorocyclobutoxy)-4-nitrobenzene **23b** (2.56 g), with a yield of 72.4%.
MS m/z (ESI): 308.0/310.0 [M+1]

Second step

2-(2-(3,3-difluorocyclobutoxy)-4-nitrophenyl)oxazole

At room temperature, 1-bromo-2-(3,3-difluorocyclobutoxy)-4-nitrobenzene **23b** (500.00 mg, 1.62 mmol) and 2-(tributylstannyl)oxazole **23c** (871.80 mg, 2.43 mmol) were dissolved in an N,N-dimethylformamide solution (5 mL), 1,1'-bis(diphenylphosphino)ferrocene dichloropalladium(II) (252.56 mg, 0.32 mmol) and cuprous iodide (30.91 mg, 0.16 mmol) were sequentially added, and the system was subjected to nitrogen purging for three times. The reaction was carried out at 100°C for 18 hours. The reaction solution was poured into water (100 mL) and extracted with ethyl acetate (40 mL×3), and a combined organic phase was washed with saturated sodium chloride solution (40 mL), dried with anhydrous sodium sulfate, filtered, and concentrated under reduced pressure. The crude product was separated and purified by silica gel column chromatography (eluent: A system) to obtain 2-(2-(3,3-difluorocyclobutoxy)-4-nitrophenyl)oxazole **23d**

(196.00 mg), with a yield of 40.8%.
MS m/z (ESI): 297.0 [M+1]

Third step

3-(3,3-difluorocyclobutoxy)-4-(oxazol-2-yl)aniline

The 2-(2-(3,3-difluorocyclobutoxy)-4-nitrophenyl)oxazole **23d** (100.00 mg, 0.34 mmol) was dissolved in a mixed solution of ethanol (1 mL) and water (0.5 mL), ammonium chloride (18.06 mg, 0.34 mmol) and iron powder (18.85 mg, 0.34 mmol) were added sequentially, and the system was subjected to nitrogen purging for three times. After the reaction was carried out during stirring at 100°C for 3 hours, the reaction solution was cooled to room temperature and filtered, the filter cake was washed with ethyl acetate (20 mL×3), and a combined organic phase was concentrated under reduced pressure to obtain 3-(3,3-difluorocyclobutoxy)-4-(oxazol-2-yl)aniline **23e** (62.00 mg), with a yield of 68.9%.
MS m/z (ESI): 267.0 [M+1]

Fourth step

N-(3-(3,3-difluorocyclobutoxy)-4-(oxazol-2-yl)phenyl)-4-(ethylsulfonamido)-2-(6-azaspiro[2.5] octan-6-yl)benzamide

At room temperature, 3-(3,3-difluorocyclobutoxy)-4-(oxazol-2-yl)aniline **23e** (62.00 mg,0.23 mmol) was dissolved in an N,N-dimethylformamide solution (1 mL), N,N-diisopropylethylamine (120.39 mg, 0.93 mmol), 4-(ethylsulfonamido)-2-(6-azaspiro[2.5]octan-6-yl)benzoic acid **7a** (78.81 mg, 0.23 mmol) and (7-azabenzotriazole-1-yloxy)tripyrrolidinophosphonium hexafluorophosphate (182.12 mg, 0.39 mmol) were added sequentially, and the system was subjected to nitrogen purging for three times. The reaction was carried out at 25°C for 18 hours. The reaction solution was poured into water (80 mL) and extracted with ethyl acetate (30 mL×3), and a combined organic phase was washed with saturated sodium chloride solution (30 mL), dried with anhydrous sodium sulfate, filtered, and concentrated under reduced pressure. The crude product was separated by a preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(3,3-difluorocyclobutoxy)-4-(oxazol-2-yl)phenyl)-4-(ethylsulfonamido)-2-(6-azaspiro[2.5] octan-6-yl)benzamide **23** (41.89 mg), with a yield of 30.7%.

MS m/z (ESI): 587.4 [M+1]
$^1$H NMR (400 MHz, DMSO) δ 11.73 (s, 1H), 10.30 (s, 1H), 8.17 (s, 1H), 7.90 (d, *J* = 8.4 Hz, 1H), 7.82 (d, *J* = 8.4 Hz, 1H), 7.64 (dd, *J* = 8.4, 1.5 Hz, 1H), 7.42 (d, *J* = 1.2 Hz, 1H), 7.35 (s, 1H), 7.16 (d, *J* = 1.6 Hz, 1H), 7.03 (dd, *J* = 8.4, 1.9 Hz, 1H), 4.84 (s, 1H), 3.27 - 3.16 (m, 4H), 3.01 - 2.94 (m, 4H), 2.88-2.74 (m, 2H), 1.54 (s, 4H), 1.21 (t, *J* = 7.2 Hz, 3H), 0.35 (s, 4H).

Example 24

N-(3-(3,3-difluorocyclobutoxy)-4-(1H-imidazol-1-yl)phenyl)-4-(ethylsulfonamido)-2-(6-azaspir o[2.5]octan-6-yl)benzamide

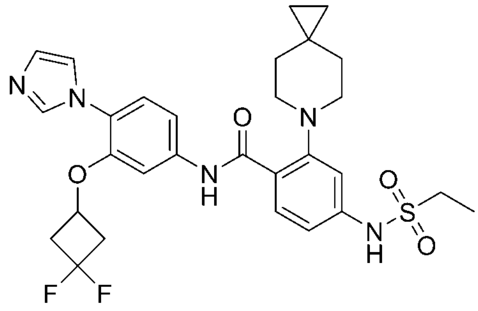

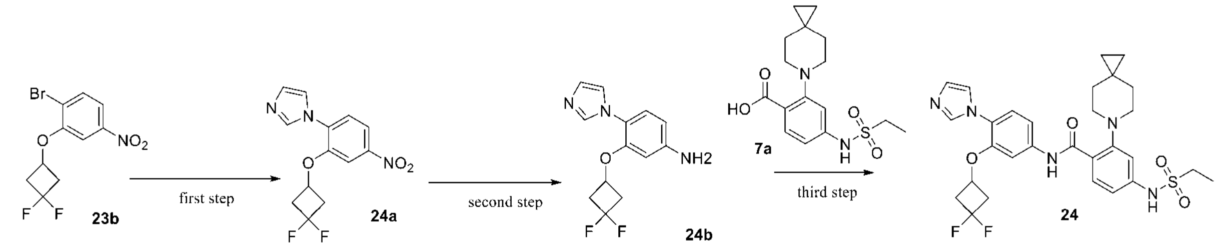


First step

1-(2-(3,3-difluorocyclobutoxy)-4-nitrophenyl)-1H-imidazole

1-bromo-2-(3,3-difluorocyclobutoxy)-4-nitrobenzene **23b** (200.00 mg, 0.65 mmol) was dissolved in an N,N-dimethylformamide solution (20 mL), potassium phosphate (413.40 mg, 1.95 mmol), trans-(1R,2R)-N,N'-dimethyl-1,2-cyclohexanediamine (46.17 mg, 0.32 mmol), imidazole (44.20 mg, 0.65 mmol) and cuprous iodide (123.64 mg, 0.65 mmol) were added sequentially, and the system was subjected to nitrogen purging for three times. The reaction was carried out during refluxing at 100°C for 8 hours. The reaction solution was filtered, the liquid phase was diluted with water (100 mL) and extracted with ethyl acetate (40 mL×3), and a combined organic phase was washed with saturated sodium chloride solution (40 mL), dried with anhydrous sodium sulfate, filtered, and concentrated under reduced pressure. The crude product was separated and purified by silica gel column chromatography (eluent: A system) to obtain 1-(2-(3,3-difluorocyclobutoxy)-4-nitrophenyl)-1H-imidazole **24a** (119.00 mg), with a yield of 62.1%.
MS m/z (ESI): 296.0 [M+1]

Second step

3-(3,3-difluorocyclobutoxy)-4-(1H-imidazol-1-yl)aniline

The 1-(2-(3,3-difluorocyclobutoxy)-4-nitrophenyl)-1H-imidazole **24a** (119.00 mg, 0.40 mmol) was dissolved in methanol (5 mL), palladium on carbon (8.58 mg, 0.08 mmol) was added, and the system was subjected to hydrogen purging for three times. The reaction was carried out during stirring under a hydrogen atmosphere at 25°C for 18 hours. The reaction solution was filtered, and the filtrate was concentrated under reduced pressure to obtain 3-(3,3-difluorocyclobutoxy)-4-(1H-imidazol-1-yl)aniline **24b** (105 mg). The crude product was directly used in the next step, with a yield of 98.2%.
MS m/z (ESI): 266.0 [M+1]

Third step

N-(3-(3,3-difluorocyclobutoxy)-4-(1H-imidazol-1-yl)phenyl)-4-(ethylsulfonamido)-2-(6-azaspir o[2.5]octan-6-yl)benzamide

At room temperature, 3-(3,3-difluorocyclobutoxy)-4-(1H-imidazol-1-yl)aniline **24b** (55.00 mg, 0.20 mmol) was dissolved in N,N-dimethylformamide (1 mL), N,N-diisopropylethylamine (107.19 mg, 0.89 mmol), 4-(ethylsulfonamido)-2-(6-azaspiro[2.5]octan-6-yl)benzoic acid **7a** (70.17 mg, 0.20 mmol) and (7-azabenzotriazole-1-yloxy)tripyrrolidinophosphonium hexafluorophosphate (162.16 mg, 0.31 mmol) were added sequentially, and the system was subjected to nitrogen purging for three times. The reaction was carried out at 20°C for 18 hours. The mixture was poured into water (100 mL) and extracted with ethyl acetate (50 mL×3), and a combined organic phase was washed with saturated sodium

chloride solution (50 mL), dried with anhydrous sodium sulfate, filtered, and concentrated under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(3,3-difluorocyclobutoxy)-4-(1H-imidazol-1-yl)phenyl)-4-(ethylsulfonamido)-2-(6-azaspir o[2.5]octan-6-yl)benzamide **24** (55.03 mg), with a yield of 45.3%.

MS m/z (ESI): 587.2 [M+1]
$^1$H NMR (400 MHz, DMSO-*d*$_6$) δ 11.69 (s, 1H), 10.30 (s, 1H), 7.94 (s, 1H), 7.82 (d, *J* = 8.4 Hz, 1H), 7.58 (dd, *J* = 8.6, 1.9 Hz, 1H), 7.51 - 7.43 (m, 3H), 7.16 (d, *J* = 1.7 Hz, 1H), 7.05 (s, 2H), 4.79 (s, 1H), 3.18 (dd, *J* = 14.6, 7.2 Hz, 4H), 2.97 (d, *J* = 5.0 Hz, 4H), 2.79 (dd, *J* = 14.0, 4.6 Hz, 2H), 1.55 (s, 4H), 1.21 (t, *J* = 7.2 Hz, 3H), 0.35 (s, 4H).

Example 25

N-(3-(4,4-difluoropiperidin-1-yl)-4-(1H-imidazol-2-yl)phenyl)-4-(ethylsulfonamido)-2-(6-azaspi ro[2.5]octan-6-yl)benzamide

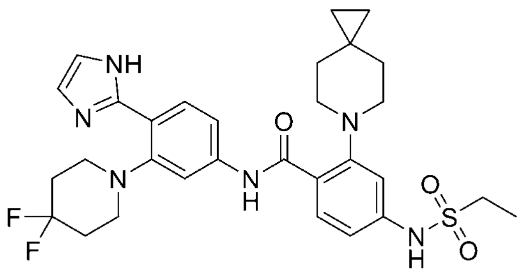

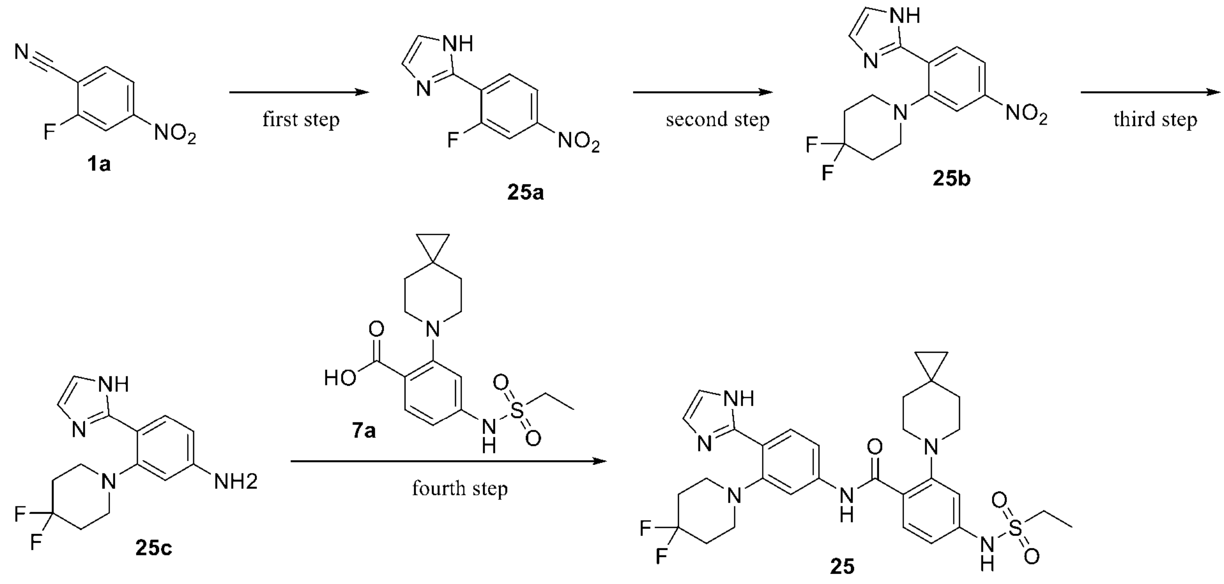


First step

2-(2-fluoro-4-nitrophenyl)-1H-imidazole

2-Fluoro-4-nitrobenzonitrile **1a** (1.50 g, 9.03 mmol) was dissolved in methanol (20 mL), sodium methoxide (243.92 mg, 4.52 mmol) was added, and the system was stirred at 25°C for 4 hours, and then acetic acid (1.08 g, 18.06 mmol) and imidazole (1.14 g, 10.84 mmol) were added, the mixture was stirred at 50°C for 0.5 hour, then cooled, and finally hydrochloric acid (6 M, 10 mL) was added to reflux at 80°C for 2 hours. The reaction solution was cooled, then diluted with water (100 mL), and extracted with dichloromethane (40 mL×3), and a combined organic phase was washed with saturated sodium chloride solution (50 mL), dried with anhydrous sodium sulfate, filtered, and concentrated under reduced pressure. The crude product was separated and purified by silica gel column chromatography (eluent: A system) to obtain

2-(2-fluoro-4-nitrophenyl)-1H-imidazole **25a** (500.00 mg), with a yield of 26.7%.
MS m/z (ESI): 208.0 [M+1]

Second step

1-(2-(1H-imidazol-2-yl)-5-nitrophenyl)-4,4-difluoropiperidine

2-(2-fluoro-4-nitrophenyl)-1H-imidazole **25a** (200.00 mg, 0.97 mmol) was dissolved in dimethyl sulfoxide (2 mL), triethylamine (488.46 mg, 4.83 mmol) and 4,4-difluorpiperidine **1b** (584.71 mg, 4.83 mmol) were added, and the system was subjected to nitrogen purging for three times. The reaction was carried out at 125°C for 18 hours. The reaction solution was poured into water (50 mL) and extracted with ethyl acetate (20 mL×3), and a combined organic phase was washed with saturated sodium chloride solution (20 mL), dried with anhydrous sodium sulfate, filtered, and concentrated under reduced pressure. The crude product was separated and purified by silica gel column chromatography (eluent: A system) to obtain 1-(2-(1H-imidazol-2-yl)-5-nitrophenyl)-4,4-difluoropiperidine **25b** (157.00 mg), with a yield of 52.7%.
MS m/z (ESI): 309.0 [M+1]

Third step

3-(4,4-difluoropiperidin-1-yl)-4-(1H-imidazol-2-yl)aniline

At room temperature, 1-(2-(1H-imidazol-2-yl)-5-nitrophenyl)-4,4-difluoropiperidine **25b** (150.00 mg, 0.49 mmol) was dissolved in amethanol solution (2 mL), palladium on carbon (10.36 mg, 0.10 mmol) was added, and the system was subjected to hydrogen purging for three times. The reaction was carried out at 25°C under a hydrogen atmosphere for 5 hours. The reaction solution was filtered and washed with methanol (20 mL×3), and a combined organic phase was concentrated to dryness under reduced pressure to obtain 3-(4,4-difluoropiperidin-1-yl)-4-(1H-imidazol-2-yl)aniline **25c** (111.00 mg). The crude product was directly used in the next step of reaction.
MS m/z (ESI): 279.2 [M+1]

Fourth step

N-(3-(4,4-difluoropiperidin-1-yl)-4-(1H-imidazol-2-yl)phenyl)-4-(ethylsulfonamido)-2-(6-azaspi ro[2.5]octan-6-yl)benzamide

At room temperature, 3-(4,4-difluoropiperidin-1-yl)-4-(1H-imidazol-2-yl)aniline **25c** (50.00 mg, 0.18 mmol) was dissolved in an N,N-dimethylformamide solution (1 mL), N,N-diisopropylethylamine (92.88 mg, 0.72 mmol), 4-(ethylsulfonamido)-2-(6-azaspiro[2.5]octan-6-yl)benzoic acid **7a** (60.80 mg, 0.18 mmol) and (7-azabenzotriazole-1-yloxy)tripyrrolidinophosphonium hexafluorophosphate (140.51 mg, 0.27 mmol) were sequentially added, and the system was subjected to nitrogen purging for three times. The reaction was carried out at 25°C for 18 hours. The reaction solution was poured into water (80 mL) and extracted with ethyl acetate (30 mL×3), and a combined organic phase was washed with saturated sodium chloride solution (30 mL), dried with anhydrous sodium sulfate, filtered, and concentrated under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(1H-imidazol-2-yl)phenyl)-4-(ethylsulfonamido)-2-(6-azaspi ro[2.5]octan-6-yl) benzamide **25** (14.30 mg), with a yield of 13.3%.

MS m/z (ESI): 600.4 [M+1]
$^1$H NMR (400 MHz, DMSO-$d_6$) δ 11.82 (s, 1H), 10. 32 (s, 1H), 7.84 (d, $J$ = 8.4 Hz, 2H), 7.68 (d, $J$ = 1.8 Hz, 1H), 7.56 (dd, $J$ = 8.4, 1.8 Hz, 1H), 7.29 - 6.94 (m, 4H), 3.19 (dd, $J$ = 14.6, 7.2 Hz, 2H), 3.00 - 2.88 (m, 8H), 2.28-2.14(m, 4H), 1.56 (s, 4H), 1.21 (t, $J$ = 7.3 Hz, 3H), 0.36 (s, 4H).

Example 26 was synthesized according to the synthesis method in Example 25 of the present invention, and a structure and characterization data were as shown in the following table:

| Serial number and structure of example | MS m/z (ESI) | $^1$H NMR |
|---|---|---|
| 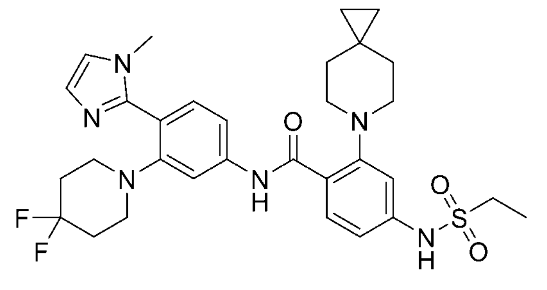 **26** | 613.4 [M+1] | $^1$H NMR (400 MHz, DMSO-$d_6$) δ 11.87 (s, 1H), 10.43 (s, 1H), 7.85 (d, *J* = 8.4 Hz, 1H), 7.64 (d, *J* = 1.6 Hz, 1H), 7.49 (dd, *J* = 8.4, 1.6 Hz, 1H), 7.31 - 7.16 (m, 3H), 7.05 (dd, *J* = 8.4, 2.0 Hz, 1H), 7.00 (d, *J* = 1.0 Hz, 1H), 3.49 (s, 3H), 3.20 (q, *J* = 7.2 Hz, 2H), 2.97 (dd, *J* = 21.8, 16.8 Hz, 8H), 1.96-1.82 (m, 4H), 1.64-1.51 (m, 4H), 1.21 (t, *J* = 7.3 Hz, 3H), 0.36 (s, 4H). |

Example 27

N-(3-(4,4-difluoropiperidin-1-yl)-4-(4H-1,2,4-triazol-4-yl)phenyl)-4-(ethylsulfonamido)-2-(6-az aspiro[2.5]octan-6-yl) benzamide

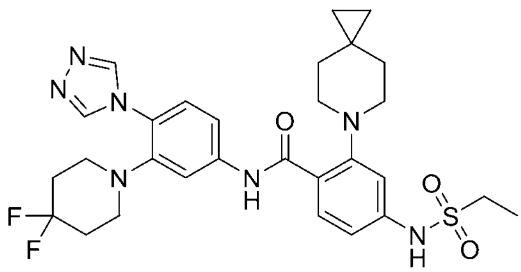

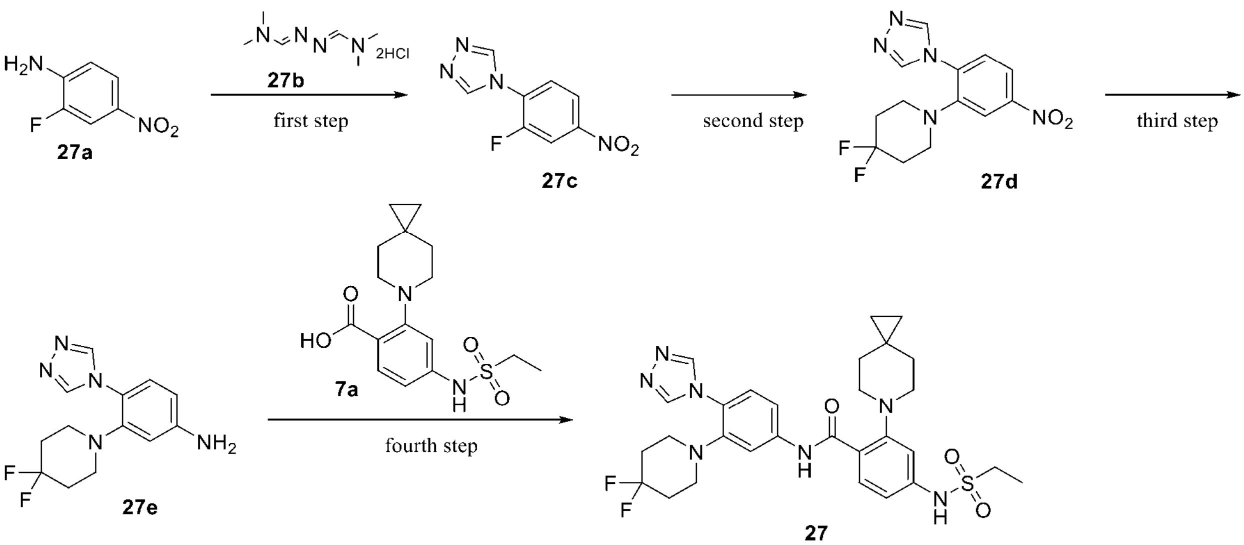


First step

4-(2-fluoro-4-nitrophenyl)-4H-1,2,4-triazole

At room temperature, N,N'-bis(dimethylaminomethylene)hydrazine dihydrochloride **27b** (2.76 g, 12.8 mmol, commercially available) and p-toluenesulfonic acid (110 mg, 0.64 mmol) were added into a toluene solution (10 mL) of 2-fluoro-4-nitroaniline **27a** (1 g, 6.41 mmol, commercially available). The reaction solution was reacted at 110°C for 16 hours.

The reaction solution was concentrated under reduced pressure. The crude product was separated by a preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 4-(2-fluoro-4-nitrophenyl)-4H-1,2,4-triazole **27c** (600 mg), with a yield of 45%.
MS m/z (ESI): 209.0 [M+1]

Second step

4,4-difluoro-1-(5-nitro-2-(4H-1,2,4-triazol-4-yl)phenyl)piperidine

At room temperature, 4-(2-fluoro-4-nitrophenyl)-4H-1,2,4-triazole **27c** (300 mg, 1.44 mmol) was added into a mixed solution of dimethyl sulfoxide (2.5 mL) and 4,4-difluoropiperidine (2.5 mL). The reaction solution was reacted at 120°C for 16 hours. The reaction solution was added into water and the system was extracted with ethyl acetate (10 mL×3), and a combined organic phase was washed with saturated sodium chloride solution, dried with anhydrous sodium sulfate, and concentrated under reduced pressure. The crude product was purified by silica gel column chromatography (eluent: A system) to obtain 4,4-difluoro-1-(5-nitro-2-(4H-1,2,4-triazol-4-yl)phenyl)piperidine **27d** (260 mg), with a yield of 58%.
MS m/z (ESI): 310.0 [M+1]

Third step

3-(4,4-difluoropiperidin-1-yl)-4-(4H-1,2,4-triazol-4-yl)aniline

At room temperature, palladium on carbon (50.0 mg, 10%) was added into a methanol solution (20 mL) of 4,4-difluoro-1-(5-nitro-2-(4H-1,2,4-triazol-4-yl)phenyl)piperidine **27d** (260 mg, 0.84 mmol), and the system was subjected to hydrogen purging for three times. Then, the reaction was carried out at room temperature for 2 hours. The reaction solution was filtered, the filter cake was washed with methanol (50 mL), and the filtrate was concentrated under reduced pressure to obtain 3-(4,4-difluoropiperidin-1-yl)-4-(4H-1,2,4-triazol-4-yl)aniline **27e** (220 mg), with a yield of 94%.
MS m/z (ESI): 280.0 [M+1]

Fourth step

N-(3-(4,4-difluoropiperidin-1-yl)-4-(4H-1,2,4-triazol-4-yl)phenyl)-4-(ethylsulfonamido)-2-(6-az aspiro[2.5]octan-6-yl) benzamide

At room temperature, O-(7-azabenzotriazol-1-yl)-N,N,N',N'-tetramethyluronium hexafluorophosphate (70.8 mg, 0.18 mmol) and N,N-diisopropylethylamine (27.7 mg, 0.21 mmol) were added into an N,N-dimethylformamide solution (1 mL) of 3-(4,4-difluoropiperidin-1-yl)-4-(4H-1,2,4-triazol-4-yl)aniline **27e** (40.0 mg, 0.14 mmol) and 4-(ethylsulfonamido)-2-(6-azaspiro[2.5]octan-6-yl)benzoic acid **7a** (50 mg, 0.15 mmol). The reaction solution was reacted at 25°C for 16 hours. The reaction solution was added into water (5 mL) and extracted with ethyl acetate (5 mL×3), and a combined organic phase was washed with saturated sodium chloride solution, dried with anhydrous sodium sulfate, and concentrated under reduced pressure. The crude product was separated by a preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(4H-1,2,4-triazol-4-yl)phenyl)-4-(ethylsulfonamido)-2-(6-az aspiro [2.5]octan-6-yl)benzamide **27** (18.6 mg), with a yield of 21%.

MS m/z (ESI): 601.2 [M+1]
$^1$H NMR (400 MHz, DMSO-$d_6$) δ 11.80 (s, 1H), 10.22 (s, 1H), 8.93 (s, 2H), 7.87 - 7.72 (m, 2H), 7.56 (d, *J* = 8.4, 2.0 Hz, 1H), 7.44 (d, *J* = 8.4 Hz, 1H), 7.16 (d, *J* = 2.0 Hz, 1H), 7.03 (d, *J* = 8.4, 2.0 Hz, 1H), 3.24 - 3.11 (m, 2H), 3.06 - 2.91 (m, 4H), 2.86 - 2.72 (m, 4H), 2.04 - 1.86 (m, 4H), 1.55 (s, 4H), 1.21 (t, *J* = 7.2 Hz, 3H), 0.35 (s, 4H).

Examples 28-32 were synthesized according to the synthesis methods in Examples 20-25 of the present invention, and structures and characterization data were as shown in the following table:

| Serial number and structure of example | MS m/z (ESI) | $^1$H NMR |
|---|---|---|
| 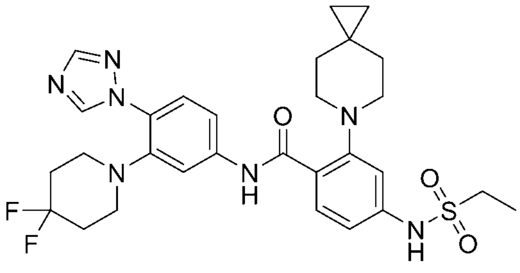 **28** | 600.2 [M+1] | $^1$H NMR (400 MHz, DMSO-$d_6$) δ 11.80 (s, 1H), 10.24 (s, 1H), 9.06 (s, 1H), 8.24 (s, 1H), 7.82 (d, *J* = 8.4 Hz, 1H), 7.77 (s, 1H), 7.59 (d, *J* = 8.4, 1.6 Hz, 1H), 7.47 (d, *J* = 8.4 Hz, 1H), 7.17 (s, 1H), 7.04 (d, *J* = 8.4, 1.6 Hz, 1H), 3.20 (d, *J* = 7.2 Hz, 2H), 3.06 - 2.93 (m, 4H), 2.90 - 2.74 (m, 4H), 2.03 - 1.86 (m, 4H), 1.55 (s, 4H), 1.21 (t, *J* = 7.2 Hz, 3H), 0.35 (s, 4H). |
| 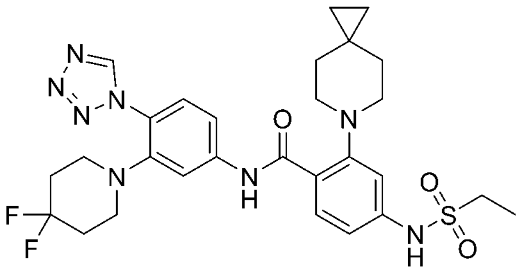 **29** | 601.3 [M+1] | $^1$H NMR (400 MHz, DMSO-$d_6$) δ 11.837 (s, 1H), 10.293 (s, 1H), 9.951 (s, 1H), 7.887 (d, *J* = 2.0 Hz, 1H), 7.811 (d, *J* = 8.4 Hz, 1H), 7.679 - 7.573 (m, 2H), 7.160 (s, 1H), 7.034 (d, *J* = 10.4 Hz, 1H), 3.190 (q, *J* = 7.2 Hz, 2H), 3.041 - 2.930 (m, 4H), 2.834 - 2.744 (m, 4H), 1.985 - 1.861 (dt, *J* = 14.1, 8.6 Hz, 4H), 1.547 (s, 4H), 1.211 (t, *J* = 7.2 Hz, 3H), 0.350 (s, 4H). |
| 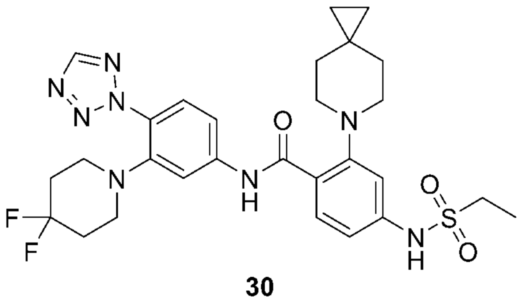 **30** | 601.4 [M+1] | $^1$H NMR (400 MHz, DMSO-$d_6$) δ 11.852 (s, 1H), 10.235 (s, 1H), 9.261 (s, 1H), 7.872 (d, *J* = 2.0 Hz, 1H), 7.821 (d, *J* = 8.4Hz, 1H), 7.672 - 7.544 (m, 2H), 7.172 (d, *J* = 2.0 Hz, 1H), 7.045 (dd, *J* = 8.8, 2.0 Hz, 1H), 3.202 (q, *J* = 7.2 Hz, 2H), 3.047 - 2.952 (m, 4H), 2.934 - 2.834 (m, 4H), 1900 - 1.790 (m, 4H), 1.554 (s, 4H), 1.215 (t, *J* = 7.2 Hz, 3H), 0.353 (s, 4H). |
| 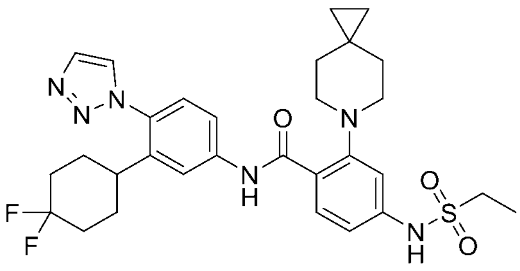 **31** | 599.2 [M+1] | $^1$H NMR (400 MHz, DMSO-$d_6$) δ 11.92 (s, 1H), 10.18 (s, 1H), 8.48 (d, *J* = 0.8 Hz, 1H), 7.97 (d, *J* = 0.8 Hz, 1H), 7.95 - 7.91 (m, 1H), 7.87 - 7.83 (m, 2H), 7.41 (d, *J* = 8.8 Hz, 1H), 7.19 (d, *J* = 2.0 Hz, 1H), 7.08 - 7.04 (m, 1H), 3.42 (s, 2H), 3.24 - 3.17 (m, 2H), 3.03 - 2.95 (m, 4H), 2.39 - 2.31 (m, 1H), 2.10 - 2.00 (m, 2H), 1.87 - 1.80 (m, 2H), 1.70 - 1.66 (m, 2H), 1.61 - 1.50 (m, 4H), 1.22 (t, *J* = 7.2 Hz, 3H), 0.35 (s, 4H). |
| 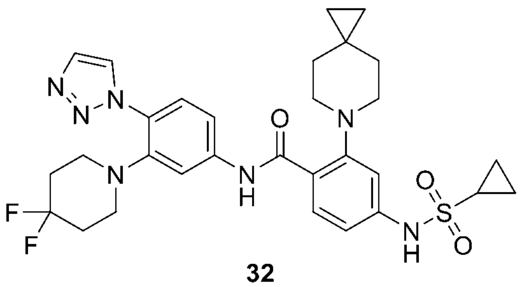 **32** | 612.2 [M+1] | $^1$H NMR (400 MHz, DMSO-$d_6$) δ 11.86 (s, 1H), 10.15 (s, 1H), 8.65 (d, *J* = 1.2 Hz, 1H), 7.96 (d, *J* = 1.2 Hz, 1H), 7.84 (d, *J* = 8.4 Hz, 1H), 7.79 (d, *J* = 2.4 Hz, 1H), 7.63 - 7.59 (m, 1H), 7.52 (d, *J* = 8.4 Hz, 1H), 7.21 (d, *J* = 2.0 Hz, 1H), 7.09 - 7.05 (m, 1H), 3.02 - 2.97 (m, 4H), 2.80 - 2.73 (m, 5H), 1.99 - 1.89 (m, 4H), 1.61 - 1.52 (m, 4H), 1.02 - 0.98 (m, 4H), 0.36 (s, 4H). |

(continued)

| Serial number and structure of example | MS m/z (ESI) | $^1H$ NMR |
|---|---|---|
| 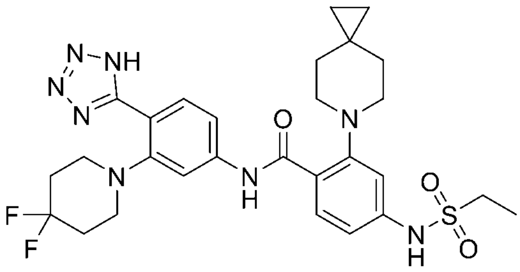 33 | 601.2 [M+1] | $^1H$ NMR (400 MHz, DMSO-$d_6$) δ 11.84 (s, 1H), 10.18 (s, 1H), 7.83 (d, *J* = 8.4 Hz, 1H), 7.80 - 7.73 (m, 2H), 7.63 (d, *J* = 8.4, 1.7 Hz, 1H), 7.18 (s, 1H), 7.05 (d, *J* = 8.4, 2.0 Hz, 1H), 3.25 - 3.16 (m, 2H), 3.02 - 2.95 (m, 4H), 2.95 - 2.84 (m, 4H), 2.21 - 2.01 (m, 4H), 1.55 (s, 4H), 1.21 (t, *J* = 7.2 Hz, 3H), 0.35 (s, 4H). |
| 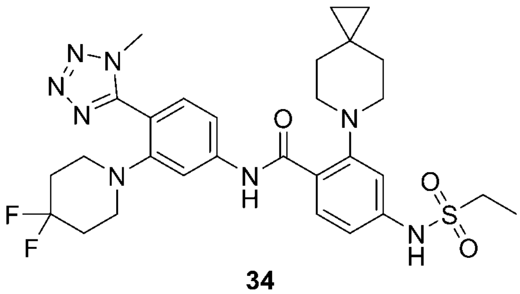 34 | 615.4 [M+1] | $^1H$ NMR (400 MHz, DMSO-$d_6$) δ 11.92 (s, 1H), 10.19 (s, 1H), 7.91 - 7.79 (m, 2H), 7.60 (d, *J* = 8.4 Hz, 1H), 7.45 (d, *J* = 8.4 Hz, 1H), 7.19 (s, 1H), 7.06 (d, *J* = 8.4, 1.9 Hz, 1H), 3.96 (s, 3H), 3.27 - 3.17 (m, 2H), 3.07 - 2.95 (m, 4H), 2.95 - 2.80 (m, 4H), 1.95 - 1.77 (m, 4H), 1.57 (s, 4H), 1.22 (t, *J* = 7.2 Hz, 3H), 0.36 (s, 4H). |
| 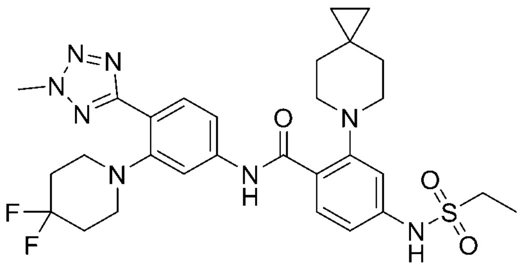 35 | 615.4 [M+1] | $^1H$ NMR (400 MHz, DMSO-$d_6$) δ 11.88 (s, 1H), 10.18 (s, 1H), 7.92 (d, *J* = 8.4 Hz, 1H), 7.85 (d, *J* = 8.4 Hz, 1H), 7.70 (s, 1H), 7.64 (d, *J* = 8.4, 1.8 Hz, 1H), 7.19 (s, 1H), 7.06 (d, *J* = 8.4, 2.0 Hz, 1H), 4.43 (s, 3H), 3.27 - 3.15 (m, 2H), 3.12 - 3.03 (m, 4H), 3.03 - 2.92 (m, 4H), 2.21 - 2.04 (m, 4H), 1.57 (s, 4H), 1.22 (t, *J* = 7.2 Hz, 3H), 0.36 (s, 4H). |

Example 36

N-(3-(4,4-difluoropiperidin-1-yl)-4-(isoxazol-3-yl)phenyl)-4-(ethylsulfonamido)-2-(6-azaspiro[2 .5]octan-6-yl)benzamide

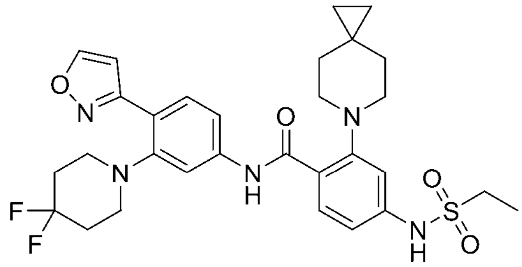

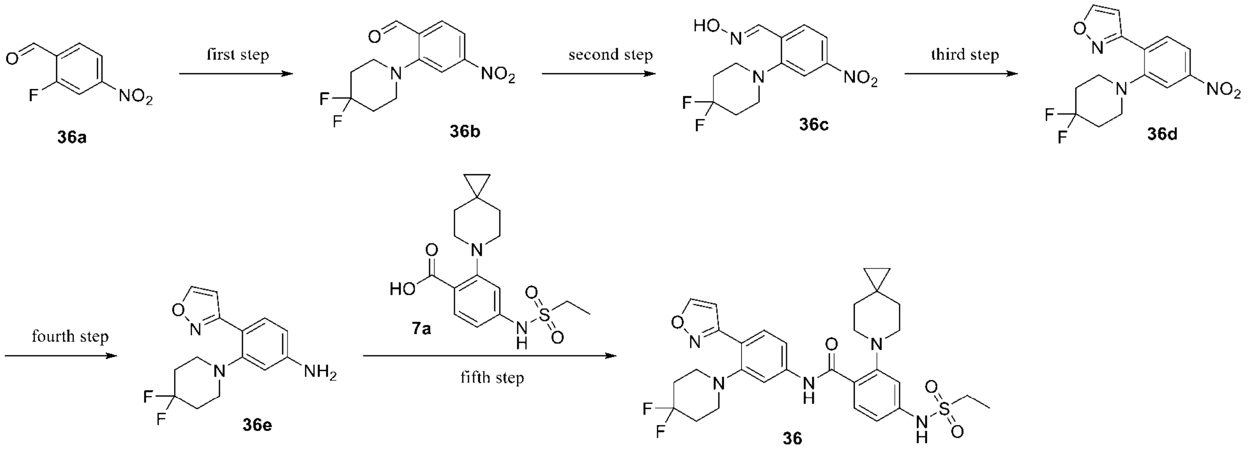


First step

2-(4,4-difluoropiperidin-1-yl)-4-nitrobenzaldehyde

Triethylamine (5.39 g, 53.22 mmol) was added to a dimethyl sulfoxide solution (10 mL) of 2-fluoro-4-nitrobenzaldehyde **36a** (3 g, 17.74 mmol, commercially available) and 4,4-difluorpiperidine **1b** (4.30 g, 35.48 mmol), and the mixture was heated to 100°C under nitrogen protection and stirred for 18 hours. The reaction solution was added into water and extracted with ethyl acetate (30 mL×3), and a combined organic phase was washed with saturated sodium chloride solution (30 mL), dried with anhydrous sodium sulfate, and concentrated under reduced pressure. The crude product was separated and purified by silica gel column chromatography (eluent: A system) to obtain 2-(4,4-difluoropiperidin-1-yl)-4-nitrobenzaldehyde **36b** (2 g), with a yield of 41.7%.
MS m/z (ESI): 271.2 [M+1]

Second step

(E)-2-(4,4-difluoropiperidin-1-yl)-4-nitrobenzaldehyde oxime

Pyridine (293 mg, 3.70 mmol) and hydroxylamine hydrochloride (257 mg, 3.70 mmol) were added to a tetrahydrofuran solution (8 mL) of 2-(4,4-difluoropiperidin-1-yl)-4-nitrobenzaldehyde **36b** (500 mg, 1.85 mmol). The reaction mixture was stirred at 0-25°C under nitrogen protection for 18 hours, the mixture was poured into water (60 mL) and extracted with ethyl acetate (60 mL×3), and a combined organic phase was washed with saturated sodium chloride solution (100 mL) and dried with anhydrous sodium sulfate. The crude product was separated and purified by silica gel column chromatography (eluent: A system) to obtain (E)-2-(4,4-difluoropiperidin-1-yl)-4-nitrobenzaldehyde oxime **36c** (484 mg), with a yield of 91%.
MS m/z (ESI): 286.2 [M+1]

Third step

3-(2-(4,4-difluoropiperidin-1-yl)-4-nitrophenyl)isoxazole

Water (254 mg, 14.1 mmol) was added to a carbon tetrachloride suspension (1.05 mL) of the (E)-2-(4,4-difluoropiperidin-1-yl)-4-nitrobenzaldehyde oxime **36c** (310 mg, 1.09 mmol), calcium carbide (453 mg, 7.06 mmol) and N-chlorosuccinimide (189 mg, 1.41 mmol), and then the system was sealed quickly. The reaction solution was stirred at 25°C

for 16 hours. The mixture was concentrated to dryness under reduced pressure. The crude product was separated and purified by silica gel column chromatography (eluent: A system) to obtain 3-(2-(4,4-difluoropiperidin-1-yl)-4-nitrophenyl) isoxazole **36d** (124 mg), with a yield of 36%.
MS m/z (ESI): 310.2 [M+1]

Fourth step

3-(4,4-difluoropiperidin-1-yl)-4-(isoxazol-3-yl)aniline

Wet palladium on carbon (31 mg, 0.029 mmol, 10% purity) was added to a tetrahydrofuran suspension (5 mL) of 3-(2-(4,4-difluoropiperidin-1-yl)-4-nitrophenyl)isoxazole **36d** (60 mg, 0.194 mmol), the system was subjected to hydrogen purging for 3 times, and stirred at 25°C for 5 hours. The mixture was filtered, the solid was washed with ethyl acetate (20 mL×3), and the filtrate was concentrated to dryness under reduced pressure to obtain 3-(4,4-difluoropiperidin-1-yl)-4-(isoxazol-3-yl)aniline **36e** (50 mg). The crude product was directly used in the next step of reaction.
MS m/z (ESI): 280.2 [M+1]

Fifth step

N-(3-(4,4-difluoropiperidin-1-yl)-4-(isoxazol-3-yl)phenyl)-4-(ethylsulfonamido)-2-(6-azaspiro[2 .5]octan-6-yl)benzamide

O-(7-azabenzotriazol-1-yl)-N,N,N',N'-tetramethyluronium hexafluorophosphate (102 mg, 0.269 mmol) was added to an N,N-dimethylformamide solution (1.5 mL) of 3-(4,4-difluoropiperidin-1-yl)-4-(isoxazol-3-yl)aniline **36e** (50 mg, 0.179 mmol), 4-(ethylsulfonamido)-2-(6-azaspiro[2.5]octan-6-yl)benzoic acid **7a** (61 mg, 0.179 mmol) and N,N-diisopropylethylamine (58 mg, 0.448 mmol). The reaction solution was stirred at 50°C for 3 hours. The mixture was dissolved in water (20 mL) and extracted with ethyl acetate (30 mL×3). A combined organic phase was washed with saturated sodium chloride solution (30 mL), dried with anhydrous sodium sulfate, filtered, and concentrated to dryness under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(isoxazol-3-yl)phenyl)-4-(ethylsulfonamido)-2-(6-azaspiro[2 .5]octan-6-yl) benzamide 36 (27.1 mg), with a yield of 25%.

MS m/z (ESI): 600.4 [M+1]
$^1$H NMR (400 MHz, DMSO-$d_6$) δ 11.83 (s, 1H), 10.17 (s, 1H), 8.96 (d, *J* = 1.6 Hz, 1H), 7.84 (d, *J* = 8.8 Hz, 1H), 7.70 (d, *J* = 1.6 Hz, 1H), 7.65 - 7.53(m, 2H), 7.22 - 7.13 (m, 2H), 7.05 (dd, *J* = 8.4, 2.0 Hz, 1H), 3.20 (q, *J* = 7.2 Hz, 2H), 3.02 - 2.89 (m, 8H), 2.15 - 2.01(m, 4H), 1.56 (s, 4H), 1.21 (t, *J* = 7.2 Hz, 3H), 0.35 (s, 4H).

Example 37

N-(3-(4,4-difluoropiperidin-1-yl)-4-(4H-1,2,4-triazol-3-yl)phenyl)-4-(ethylsulfonamido)-2-(6-az aspiro[2.5]octan-6-yl) benzamide

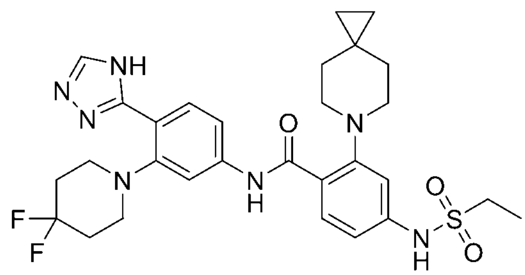

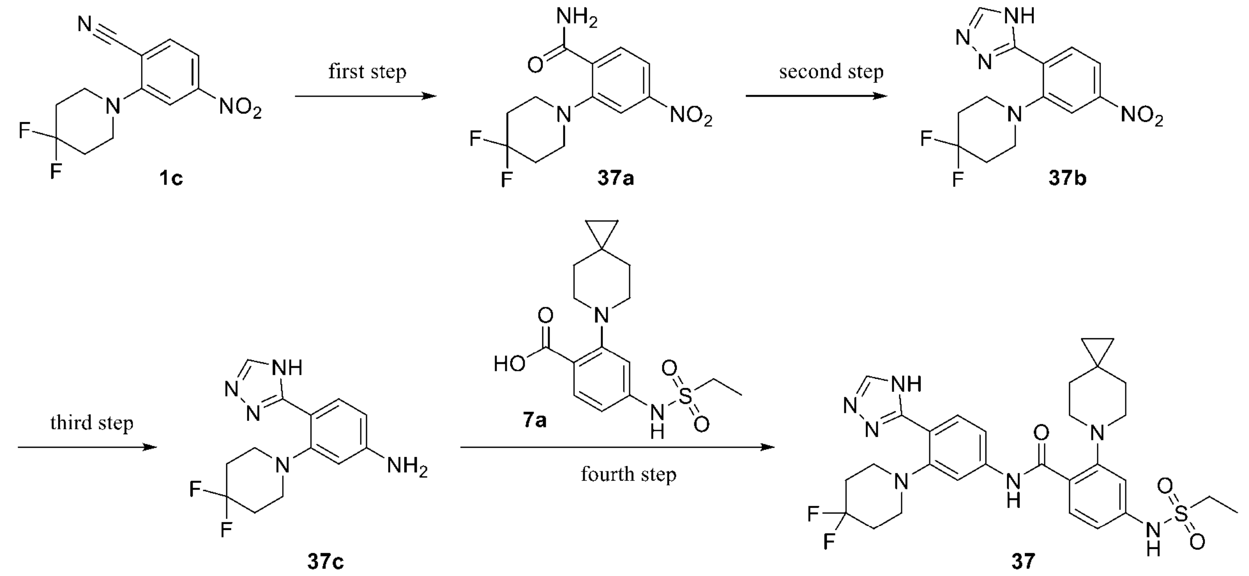


First step

2-(4,4-difluoropiperidin-1-yl)-4-nitrobenzamide

2-(4,4-difluoropiperidin-1-yl)-4-nitrobenzonitrile **1c** (500.00 mg, 1.87 mmol) was dissolved in dimethyl sulfoxide (5 mL), potassium carbonate (129.29 mg, 0.94 mmol) and hydrogen peroxide (318.21 mg, 2.81 mmol) were added, and the system was subjected to nitrogen purging for three times. The reaction was carried out at 25°C for 5 hours. The reaction solution was poured into water (80 mL) and extracted with ethyl acetate (30 mL×3), and a combined organic phase was washed with saturated sodium chloride solution (30 mL), dried with anhydrous sodium sulfate, filtered, and concentrated under reduced pressure to obtain 2-(4,4-difluoropiperidin-1-yl)-4-nitrobenzamide **37a** (424.00 mg). The crude product was directly used in the next step.
MS m/z (ESI): 286.0 [M+1]

Second step

4,4-difluoro-1-(5-nitro-2-(4H-1,2,4-triazol-3-yl)phenyl)piperidine

At room temperature, N,N-dimethylformamide dimethylacetal (2.66 g, 22.30 mmol) was added to 2-(4,4-difluoropiperidin-1-yl)-4-nitrobenzamide **37a** (424.00 mg, 1.49 mmol), and the system was subjected to nitrogen purging for three times. The reaction was carried out during stirring at 120°C for 1 hour, and then the reaction solution was cooled, and concentrated under reduced pressure and dried by rotary evaporation. Subsequently, hydrazine hydrate (9.53 mg, 0.30 mmol) and acetic acid (4 mL) were added to the system, the thus obtained system was heated to 90°C, and stirred for 1 hour. The reaction solution was concentrated to dryness under reduced pressure. The crude product was separated and purified by silica gel column chromatography (eluent: A system) to obtain 4,4-difluoro-1-(5-nitro-2-(4H-1,2,4-triazol-3-yl)phenyl)piperidine **37b** (218.00 mg), with a yield of 47.4%.
MS m/z (ESI): 310.0 [M+1]

Third step

3-(4,4-difluoropiperidin-1-yl)-4-(4H-1,2,4-triazol-3-yl)aniline

At room temperature, 4,4-difluoro-1-(5-nitro-2-(4H-1,2,4-triazol-3-yl)phenyl)piperidine **37b** (60.00 mg, 0.19 mmol) was dissolved in a methanol solution (3 mL), palladium on carbon (4.13 mg, 0.04 mmol) was added, and the system was subjected to hydrogen purging for three times. The reaction was carried out during stirring at 25°C under a hydrogen atmosphere for 18 hours. The reaction solution was filtered, the filter cake was washed with methanol (20 mL×3), and the obtained filtrate was concentrated under reduced pressure to obtain 3-(4,4-difluoropiperidin-1-yl)-4-(4H-1,2,4-triazol-3-yl)aniline **37c** (52.00 mg). The crude product was directly used in the next step.
MS m/z (ESI): 280.2 [M+1]

Fourth step

N-(3-(4,4-difluoropiperidin-1-yl)-4-(4H-1,2,4-triazol-3-yl)phenyl)-4-(ethylsulfonamido)-2-(6-az aspiro[2.5]octan-6-yl) benzamide

At room temperature, 3-(4,4-difluoropiperidin-1-yl)-4-(4H-1,2,4-triazol-3-yl)aniline **37c** (52.00 mg, 0.19 mmol) was dissolved in an N,N-dimethylformamide solution (1 mL), N,N-diisopropylethylamine (96.25 mg, 0.74 mmol), 4-(ethylsulfonamido)-2-(6-azaspiro[2.5]octan-6-yl)benzoic acid **7a** (63.01 mg, 0.19 mmol) and (7-azabenzotriazole-1-yloxy)tripyrrolidinophosphonium hexafluorophosphate (145.61 mg, 0.28 mmol) were added sequentially, and the system was subjected to nitrogen purging for three times. The reaction was carried out at 25°C for 18 hours. The reaction solution was poured into water (50 mL) and the obtained system was extracted with ethyl acetate (20 mL×3), and a combined organic phase was washed with saturated sodium chloride solution (20 mL), dried with anhydrous sodium sulfate, filtered, and concentrated under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(4H-1,2,4-triazol-3-yl)phenyl)-4-(ethylsulfonamido)-2-(6-az aspiro[2.5]octan-6-yl)benzamide **37** (10.35 mg), with a yield of 9.3%.

MS m/z (ESI): 600.4 [M+1]

$^1$H NMR (400 MHz, DMSO-$d_6$) δ 13.84 (s, 1H), 11.87 (s, 1H), 10.40 (s, 1H), 8.11 (s, 1H), 7.81 (dd, *J* = 19.7, 8.5 Hz, 2H), 7.69 (s, 1H), 7.59 (d, *J* = 8.4 Hz, 1H), 7.16 (d, *J* = 2.0 Hz, 1H), 7.04 (dd, *J* = 8.4, 2.0 Hz, 1H), 3.19 (q, *J* = 7.3 Hz, 2H), 3.04-2.87 (m, 8H), 2.24-2.09 (m, 4H), 1.56 (s, 4H), 1.21 (t, *J* = 7.3 Hz, 3H), 0.35 (s, 4H).

Examples 38-39 were synthesized according to the synthesis method in Example 37 of the present invention, and structures and characterization data were as shown in the following table:

| Serial number and structure of example | MS m/z (ESI) | $^1$H NMR |
|---|---|---|
| 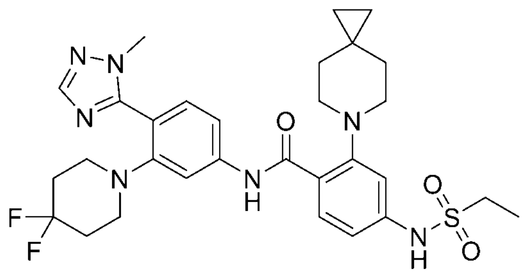 38 | 614.4 [M+1] | $^1$H NMR (400 MHz, DMSO-$d_6$) δ 11.95 (s, 1H), 8.04 (s, 1H), 7.84 (d, *J* = 8.5 Hz, 1H), 7.74 (d, *J* = 1.7 Hz, 1H), 7.54 (dd, *J* = 8.3, 1.7 Hz, 1H), 7.36 (d, *J* = 8.3 Hz, 1H), 7.17 (d, *J* = 1.9 Hz, 1H), 7.04 (dd, *J* = 8.5, 2.0 Hz, 1H), 3.71 (s, 3H), 3.18 (q, *J* = 7.3 Hz, 2H), 3.01 - 2.88 (m, 8H), 1.96-1.82 (m, 4H), 1.64-1.51 (m, 4H), 1.21 (t, *J* = 7.2 Hz, 3H), 0.36 (s, 4H). |
| 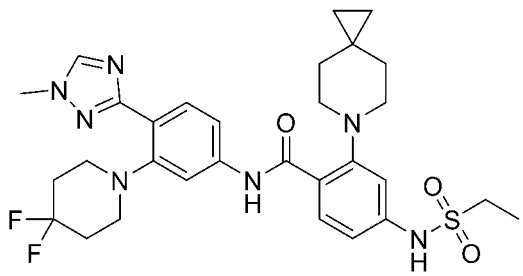 39 | 614.4 [M+1] | $^1$H NMR (400 MHz, DMSO-$d_6$) δ 11.84 (s, 1H), 10.17 (s, 1H), 8.51 (s, 1H), 7.85 (dd, *J* = 8.4, 3.3 Hz, 2H), 7.56 (dd, *J* = 11.7, 3.2 Hz, 2H), 7.18 (d, *J* = 1.9 Hz, 1H), 7.06 (dd, *J* = 8.5, 2.0 Hz, 1H), 3.93 (s, 3H), 3.20 (q, *J* = 7.2 Hz, 2H), 3.02 (dt, *J* = 29.8, 5.2 Hz, 8H), 2.18-2.04 (m, 4H), 1.57 (s, 4H), 1.21 (t, *J* = 7.2 Hz, 3H), 0.36 (s, 4H). |

Example 40

N-(3-(4,4-difluoropiperidin-1-yl)-4-(1,3,4-oxadiazol-2-yl)phenyl)-4-(ethylsulfonamido)-2-(6-aza spiro[2.5]octan-6-yl) benzamide

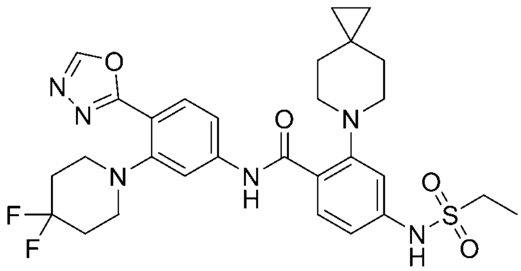

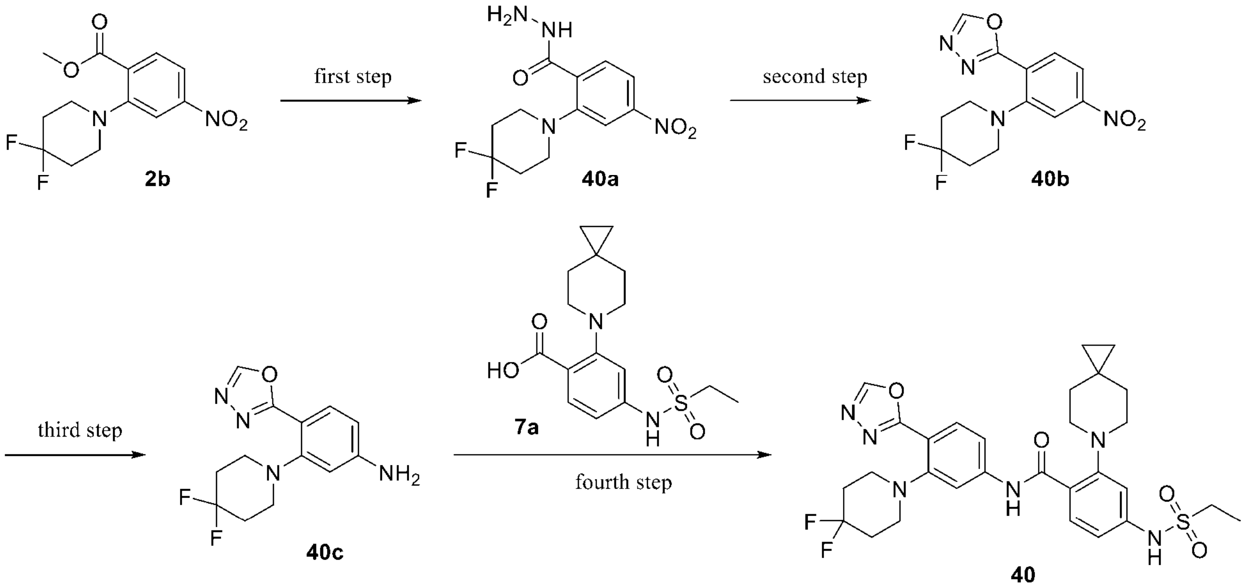


2b 40a 40b 40c 7a 40

First step

2-(4,4-difluoropiperidin-1-yl)-4-nitrobenzohydrazide

Hydrazine hydrate (5.15 g, 82.3 mmol, 80% purity) was added to an ethanol suspension (20 mL) of methyl 2-(4,4-difluoropiperidin-1-yl)-4-nitrobenzoate **2b** (2.00 g, 6.66 mmol), the mixture was heated to 70°C, and stirred for 6 hours. The mixture was concentrated to dryness under reduced pressure to obtain a residue. The crude product was separated and purified by silica gel column chromatography (eluent: A system) to obtain 2-(4,4-difluoropiperidin-1-yl)-4-nitrobenzohydrazide **40a** (1.40 g), with a yield of 70%.
MS m/z (ESI): 301.2 [M+1]

Second step

2-(2-(4,4-difluoropiperidin-1-yl)-4-nitrophenyl)-1,3,4-oxadiazole

Triethyl orthoformate (148 mg, 0.999 mmol) was added to an acetic acid solution (1 mL) of 2-(4,4-difluoropiperidin-1-yl)-4-nitrobenzohydrazide **40a** (200 mg, 0.666 mmol), and the obtained mixture was stirred at 100°C for 2 hours. The mixture was concentrated to dryness under reduced pressure. The crude product was separated and purified by silica gel column chromatography (eluent: A system) to obtain 2-(2-(4,4-difluoropiperidin-1-yl)-4-nitrophenyl)-1,3,4-oxadiazole **40b** (107 mg), with a yield of 51%.
MS m/z (ESI): 311.2 [M+1]

Third step

3-(4,4-difluoropiperidin-1-yl)-4-(1,3,4-oxadiazol-2-yl)aniline

Wet palladium on carbon (34 mg, 0.032 mmol, 10% purity) was added to a methanol suspension (10 mL) of 2-(2-(4,4-difluoropiperidin-1-yl)-4-nitrophenyl)-1,3,4-oxadiazole **40b** (50 mg, 0.161 mmol), the system was subjected to

hydrogen purging for 3 times, and stirred at 25°C for 3 hours. The mixture was filtered, the solid was washed with ethyl acetate (20 mL×3), and the filtrate was concentrated to dryness under reduced pressure to obtain 3-(4,4-difluoropiperidin-1-yl)-4-(1,3,4-oxadiazol-2-yl)aniline **40c** (40 mg), with a yield of 88%.
MS m/z (ESI): 281.2 [M+1]

Fourth step

N-(3-(4,4-difluoropiperidin-1-yl)-4-(1,3,4-oxadiazol-2-yl)phenyl)-4-(ethylsulfonamido)-2-(6-aza spiro[2.5]octan-6-yl) benzamide

O-(7-azabenzotriazol-1-yl)-N,N,N',N'-tetramethyluronium hexafluorophosphate (71 mg, 0.186 mmol) was added to an N,N-dimethylformamide solution (1 mL) of 3-(4,4-difluoropiperidin-1-yl)-4-(1,3,4-oxadiazol-2-yl)aniline **40c** (40 mg, 0.143 mmol), 4-(ethylsulfonamido)-2-(6-azaspiro[2.5]octan-6-yl)benzoic acid **7a** (48 mg, 0.143 mmol) and N,N-diisopropylethylamine (46 mg, 0.357 mmol). The mixture was stirred at 60°C for 16 hours. The mixture was dissolved in water (20 mL) and extracted with ethyl acetate (30 mL×3), and a combined organic phase was washed withsaturated sodium chloride solution (30 mL), dried with anhydrous sodium sulfate, filtered, and concentrated to dryness under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(1,3,4-oxadiazol-2-yl)phenyl)-4-(ethylsulfonamido)-2-(6-aza spiro[2.5]octan-6-yl)benzamide **40** (25 mg), with a yield of 29%.

MS m/z (ESI): 601.4 [M+1]
$^1$H NMR (400 MHz, DMSO-$d_6$) δ 11.90 (s, 1H), 10.40 - 10.05 (s, 1H), 9.31 (s, 1H), 7.92 (d, *J* = 8.4 Hz, 1H), 7.83 (d, *J* = 8.4 Hz, 1H), 7.74 (s, 1H), 7.63 (d, *J* = 7.2 Hz, 1H), 7.17 (s, 1H), 7.05 (d, *J* = 8.4 Hz, 1H), 6.82 - 6.77 (m, 1H), 3.24 - 3.16 (m, 2H), 3.12 - 3.03 (m, 4H), 3.02 - 2.93 (m, 4H), 2.23 - 2.10(m, 4H), 1.62 - 1.45 (s, 4H), 1.21 (t, *J* = 7.2 Hz, 3H), 0.35 (s, 4H).

Examples 41-43 were synthesized according to the synthesis method in Example 40 of the present invention, and structures and characterization data were as shown in the following table:

| Serial number and structure of example | MS m/z (ESI) | $^1$H NMR |
|---|---|---|
| 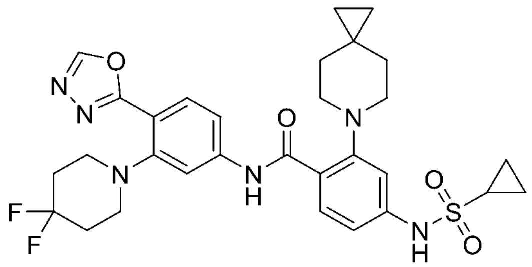 **41** | 613.4 [M+1] | $^1$H NMR (400 MHz, DMSO-$d_6$) δ 11.97 (s, 1H), 10.16 (s, 1H), 9.31 (s, 1H), 7.92 (d, *J* = 8.4 Hz, 1H), 7.86 - 7.83 (m, 1H), 7.74 (d, *J* = 2.0 Hz, 1H), 7.66 - 7.63 (m, 1H), 7.21 (d, *J* = 2.4 Hz, 1H), 7.09 - 7.05 (m, 1H), 3.10 - 3.06 (m, 4H), 3.00 - 2.97 (m, 4H), 2.78 - 2.74 (m, 1H), 2.20 - 2.12 (m, 4H), 1.59 - 1.53 (m, 4H), 1.02 - 0.99 (m, 4H), 0.36 (s, 4H). |
| 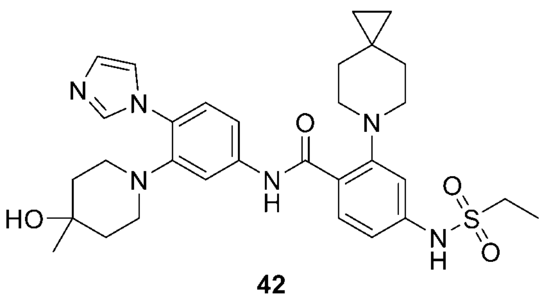 **42** | 593.4 [M+1] | $^1$H NMR (400 MHz, DMSO-$d_6$) δ 11.84 (s, 1H), 7.97 (s, 1H), 7.85 (d, *J* = 8.4 Hz, 1H), 7.66 (d, *J* = 2.0 Hz, 1H), 7.54 - 7.47 (m, 2H), 7.28 (d, *J* = 8.4 Hz, 1H), 7.19 (d, *J* = 2.0 Hz, 1H), 7.11 - 7.02 (m, 2H), 3.20 (q, *J* = 7.2 Hz, 2H), 2.99 (t, *J* = 5.2 Hz, 4H), 2.87 - 2.76 (m, 2H), 2.60 - 2.53 (m, 2H), 1.56 (s, 4H), 1.53 - 1.42 (m, 4H), 1.22 (t, *J* = 7.2 Hz, 3H), 1.12 (s, 3H), 0.37 (s, 4H). |

(continued)

| Serial number and structure of example | MS m/z (ESI) | $^{1}$H NMR |
|---|---|---|
| 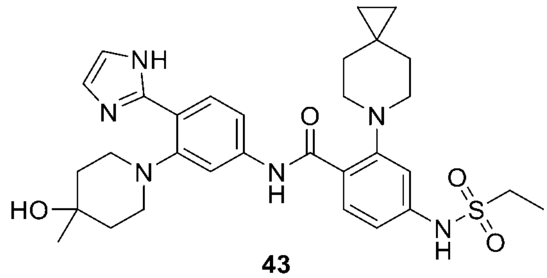 43 | 593.4 [M+1] | $^{1}$H NMR (400 MHz, DMSO-$d_6$) δ 11.84 (d, $J$ = 14.2 Hz, 2H), 10.17 (s, 1H), 7.85 (d, $J$ = 8.4 Hz, 2H), 7.71 (s, 1H), 7.53 (d, $J$ = 7.0 Hz, 1H), 7.18 (s, 2H), 7.05 (d, $J$ = 8.6 Hz, 1H), 6.99 (s, 1H), 4.26 (s, 1H), 3.20 (q, $J$ = 7.2 Hz, 2H), 3.04-2.96 (m, 6H), 2.75-2.66 (m, 2H), 1.84 - 1.70 (m, 4H), 1.61-1.53 (m, 4H), 1.25-1.17 (m, 6H), 0.37 (s, 4H). |

Example 44

N-(3-(4,4-difluoro-1-hydroxycyclohexyl)-4-(1H-imidazol-1-yl)phenyl)-4-(ethylsulfonamido)-2-( 6-azaspiro[2.5]octan-6-yl)benzamide

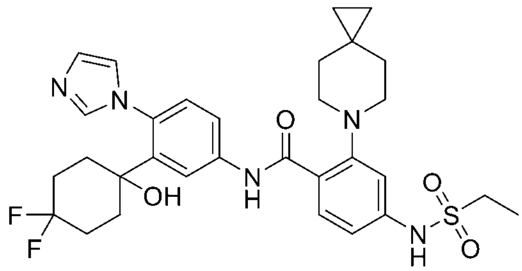

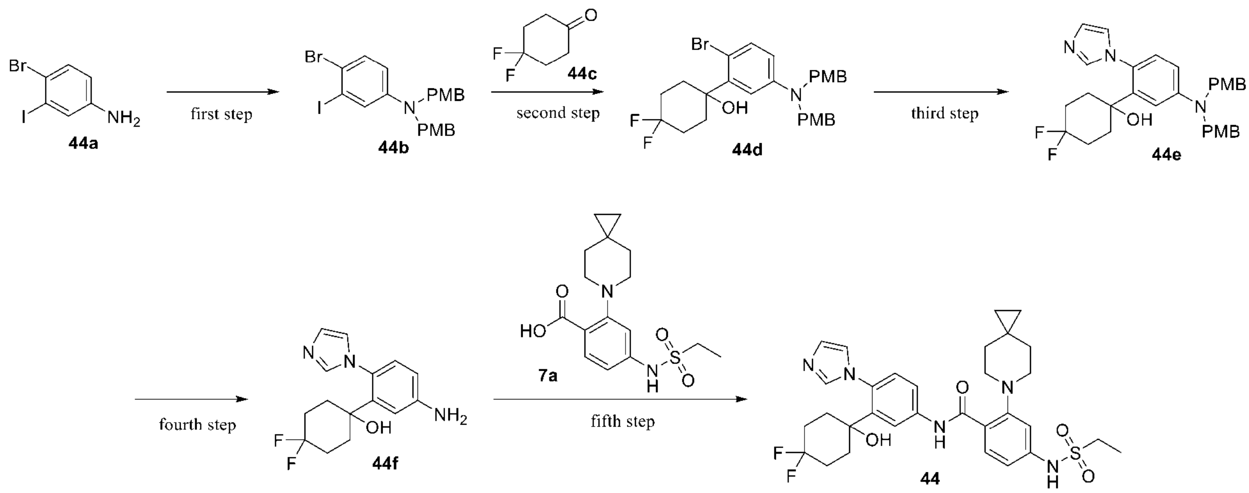


First step

4-bromo-3-iodo-N,N-bis(4-methoxybenzyl)aniline

At 0°C, sodium hydride (144.99 mg, 6.04 mmol) was added to an N,N-dimethylformamide solution (10 mL) of 4-bromo-3-iodoaniline **44a** (900.00 mg, 3.02 mmol, commercially available), stirred at room temperature for 0.5 hours, then 4-methoxybenzyl chloride (709.67 mg, 4.53 mmol) was added, the system was stirred at 25°C for 1 hour. The reaction solution was diluted with water (100 mL) and extracted with ethyl acetate (45 mL×3), and a combined organic phase was washed with saturated sodium chloride solution (40 mL), dried with anhydrous sodium sulfate, filtered, and concentrated

under reduced pressure. The crude product was separated and purified by silica gel column chromatography (eluent: A system) to obtain 4-bromo-3-iodo-N,N-bis(4-methoxybenzyl)aniline **44b** (1.4 g), with a yield of 86.1%.
MS m/z (ESI): 538/540 [M+1]

Second step

1-(5-(bis(4-methoxybenzyl)amino)-2-bromophenyl)-4,4-difluorocyclohexan-1-ol

Under nitrogen protection, 4-bromo-3-iodo-N,N-bis(4-methoxybenzyl)aniline **44b** (1.40 g, 2.60 mmol) was dissolved in a tetrahydrofuran solution (20 mL), and the system was cooled to -78°C, an isopropylmagnesium chloride-lithium chloride solution (1.13 g, 7.80 mmol) was added dropwise, the mixture was stirred for 0.5 hour, and then a tetrahydrofuran solution (5 mL) of 4,4-difluorocyclohexan-1-one **44c** (523.32 mg, 3.90 mmol, commercially available) was added. The reaction solution was concentrated under reduced pressure. The obtained crude product was separated and purified by silica gel column chromatography (eluent: A system) to obtain 1-(5-(bis(4-methoxybenzyl)amino)-2-bromophenyl)-4,4-difluorocyclohexan-1-ol **44d** (983.00 mg), with a yield of 69.2%.
MS m/z (ESI): 546.2/548.2 [M+1]

Third step

1-(5-(bis(4-methoxybenzyl)amino)-2-(1H-imidazol-1-yl)phenyl)-4,4-difluorocyclohexan-1-ol

At room temperature, 1-(5-(bis(4-methoxybenzyl)amino)-2-bromophenyl)-4,4-difluorocyclohexan-1-ol **44d** (983.00 mg, 1.80 mmol) was dissolved in N,N-dimethylformamide (10 mL), potassium phosphate (1.15 g, 5.40 mmol), imidazole (122.46 mg, 1.80 mmol), trans-(1R,2R)-N,N'-dimethyl-1,2-cyclohexanediamine (127.94 mg, 0.90 mmol) and cuprous iodide (342.60 mg, 1.80 mmol) were sequentially added, and the system was subjected to nitrogen purging for three times. The reaction was carried out at 100°C for 4 hours. The mixture was poured into water (100 mL), allowed to stand for ten minutes and then filtered, the filter cake was washed with ethyl acetate (20 mL×3) and extracted with ethyl acetate (30 mL ×3), and a combined organic phase was washed with saturated sodium chloride solution (50 mL), dried with anhydrous sodium sulfate, filtered, and concentrated under reduced pressure. The obtained crude product was separated and purified by silica gel column chromatography (eluent: A system) to obtain 1-(5-(bis(4-methoxybenzyl)amino)-2-(1H-imidazol-1-yl)phenyl)-4,4-difluorocyclohexan-1-ol **44e** (628.00 mg), with a yield of 65.4%.
MS m/z (ESI): 534.4 [M+1]

Fourth step

1-(5-amino-2-(1H-imidazol-1-yl)phenyl)-4,4-difluorocyclohexan-1-ol

At room temperature, 1-(5-(bis(4-methoxybenzyl)amino)-2-(1H-imidazol-1-yl)phenyl)-4,4-difluorocyclohexan-1-ol **44e** (628.00 mg, 1.18 mmol) was dissolved in ethanol (10 mL), trifluoroacetic acid (5 mL) was added dropwise, and the system was subjected to nitrogen purging for three times. The reaction was carried out at 70°C for 18 hours. The mixture was concentrated under reduced pressure to obtain 1-(5-amino-2-(1H-imidazol-1-yl)phenyl)-4,4-difluorocyclohexan-1-ol **44f** (290.00 mg). The crude product was directly used in the next step.
MS m/z (ESI): 294.2 [M+1]

Fifth step

N-(3-(4,4-difluoro-1-hydroxycyclohexyl)-4-(1H-imidazol-1-yl)phenyl)-4-(ethylsulfonamido)-2-( 6-azaspiro[2.5]octan-6-yl)benzamide

At room temperature, 1-(5-amino-2-(1H-imidazol-1-yl)phenyl)-4,4-difluorocyclohexan-1-ol **44f** (290.00 mg, 0.98 mmol) was dissolved in N,N-dimethylformamide (3 mL), N,N-diisopropylethylamine (511.12 mg, 3.95 mmol), 4-(ethylsulfonamido)-2-(6-azaspiro[2.5]octan-6-yl)benzoic acid **7a** (334.60 mg, 0.98 mmol) and O-(7-azabenzotriazol-1-yl)-N,N,N',N'-tetramethyluronium hexafluorophosphate (563.91 mg, 1.48 mmol) were added sequentially, and the system was subjected to nitrogen purging for three times. The reaction was carried out at 20°C for 18 hours. The mixture was poured into water (40 mL) and extracted with ethyl acetate (15 mL×3), and a combined organic phase was washed with saturated sodium chloride solution (50 mL), dried with anhydrous sodium sulfate, filtered, and concentrated under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain

N-(3-(4,4-difluoro-1-hydroxycyclohexyl)-4-(1H-imidazol-1-yl)phenyl)-4-(ethylsulfonamido)-2-( 6-azaspiro[2.5]octan-6-yl)benzamide **44** (35.57 mg), with a yield of 5.9%.

MS m/z (ESI): 614.3 [M+1]

$^1$H NMR (400 MHz, DMSO-$d_6$) δ 11.93 (s, 1H), 10.18 (s, 1H), 8.20 (d, *J* = 2.4 Hz, 1H), 7.88 - 7.82 (m, 2H), 7.75 (s, 1H), 7.32 (s, 1H), 7.20 (d, *J* = 2.0 Hz, 1H), 7.15 (d, *J* = 8.4 Hz, 1H), 7.06 (dd, *J* = 8.4, 2.0 Hz, 1H), 7.02 (s, 1H), 5.36 (s, 1H), 3.20 (q, *J* = 7.2 Hz, 2H), 3.03 - 2.97 (m, 4H), 2.19 - 2.01 (m, 2H), 1.81 - 1.55 (m, 10H), 1.22 (t, *J* = 7.3 Hz, 3H), 0.37 (s, 4H).

Example 45

N-(3-(3,3-difluorocyclobutoxy)-4-(1-methyl-1H-1,2,4-triazol-3-yl)phenyl)-4-(ethylsulfonamido) -2-(6-azaspiro[2.5]octan-6-yl)benzamide

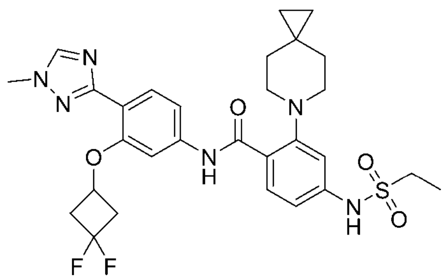

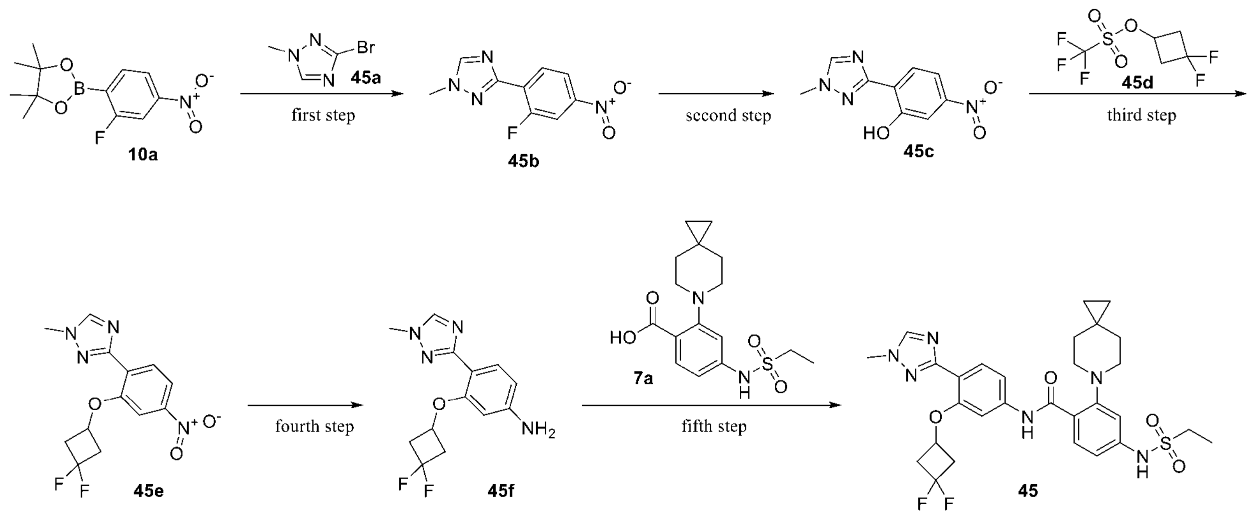


First step

3-(2-fluoro-4-nitrophenyl)-1-methyl-1H-1,2,4-triazole

At room temperature, 2-(2-fluoro-4-nitrophenyl)-4,4,5,5-tetramethyl-1,3,2-dioxaborolane **10a** (1.4 g, 5.24 mmol) and 3-bromo-1-methyl-1,2,4-triazole **45a** (1.02 g, 6.29 mmol, commercially available) were dissolved in dioxane (15 mL) and water (5 mL),[1,1'-bis(diphenylphosphino)ferrocene]dichloropalladium(II) (191.79 mg, 262.11 μmol) and potassium carbonate (1.45 g, 10.48 mmol) were added, the system was subjected to argon purging for 5 minutes, heated to 90°C, and stirred to react for 3 hours. The reaction solution was cooled to room temperature, water (20 mL) was added and the system was extracted with ethyl acetate (30 mL×3), and a combined organic phase was washed with saturated sodium chloride solution (20 mL), dried with anhydrous sodium sulfate, filtered, and concentrated under reduced pressure. The crude product was separated and purified by silica gel column chromatography (eluent: A system) to obtain 3-(2-fluoro-4-nitrophenyl)-1-methyl-1,2,4-triazole 45b (0.6 g), with a yield of 51.5%.
MS m/z (ESI): 223.1 [M+1]

Second step

2-(1-methyl-1H-1,2,4-triazol-3-yl)-5-nitrophenol

At room temperature, an aqueous solution (1 mL) of sodium hydroxide (270.04 mg, 6.75 mmol) was added to a dimethyl sulfoxide solution (5 mL) of 3-(2-fluoro-4-nitro-phenyl)-1-methyl-1,2,4-triazole **45b** (300 mg, 1.35 mmol) , the mixture was heated to 100°C, and stirred to react for 18 hours. Water (20 mL) was added to the reaction solution , a pH value of the reaction solution was adjusted to 3-4 with 1 M hydrochloric acid solution, the reaction solution was extracted with ethyl acetate (30 mL×2), and a combined organic phase was washed with saturated sodium chloride solution (20 mL), dried with anhydrous sodium sulfate, filtered, and concentrated under reduced pressure to obtain 2-(1-methyl-1H-1,2,4-triazol-3-yl)-5-nitrophenol **45c** (0.28 g), with a yield of 94.2%.
MS m/z (ESI): 221.1 [M+1]

Third step

3-(2-(3,3-difluorocyclobutoxy)-4-nitrophenyl)-1-methyl-1H-1,2,4-triazole

At room temperature, cesium carbonate (1.33 g, 4.09 mmol) and 3,3-difluorocyclobutyl trifluoromethanesulfonate **45d** (325 mg, 1.36 mmol, prepared according to the published patent "WO2020176765") were added to an N,N-dimethyldimethylamine solution (10 mL) of 2-(1-methyl-1H-1,2,4-triazol-3-yl)-5-nitrophenol **45c** (300 mg, 1.36 mmol), and stirred at room temperature for 2 hours. Water (30 mL) wsas added to the reaction solutionand the system was extracted with ethyl acetate (30 mL×3), and a combined organic phase was washed with saturated sodium chloride solution (20 mL), dried with anhydrous sodium sulfate, filtered, and concentrated under reduced pressure to obtain 3-(2-(3,3-difluorocyclobutoxy)-4-nitrophenyl)-1-methyl-1H-1,2,4-triazole **45e** (0.4 g). The crude product was directly used in the next step of reaction.
MS m/z (ESI): 311.1 [M+1]

Fourth step

3-(3,3-difluorocyclobutoxy)-4-(1-methyl-1H-1,2,4-triazol-3-yl)aniline

At room temperature, an aqueous solution (2 mL) of ammonium chloride (482.75 mg, 9.02 mmol) was added to an ethanol solution (10 mL) of 3-(2-(3,3-difluorocyclobutoxy)-4-nitrophenyl)-1-methyl-1H-1,2,4-triazole **45e** (400 mg, 1.29 mmol), then iron powder (359.99 mg, 6.45 mmol) was added, the system was subjected to argon purging for 3 times, heated to 80°C, and stirred to react for 2 hours. The reaction solution was cooled to room temperature, tetrahydrofuran (30 mL) was added, the mixture was stirred for 30 minutes, filtered and concentrated under reduced pressure. Water (30 mL) was added to the residue, a pH value of the mixture was adjusted to 7-8 with saturated sodium bicarbonate solution, the mixture was extracted with ethyl acetate (30 mL×3), and a combined organic phase was washed with saturated sodium chloride solution (10 mL), dried with anhydrous sodium sulfate, filtered, and concentrated under reduced pressure to obtain 3-(3,3-difluorocyclobutoxy)-4-(1-methyl-1H-1,2,4-triazol-3-yl)aniline **45f** (0.35 g), with a yield of 96.8%.
MS m/z (ESI): 281.1 [M+1]

Fifth step

N-(3-(3,3-difluorocyclobutoxy)-4-(1-methyl-1H-1,2,4-triazol-3-yl)phenyl)-4-(ethylsulfonamido) -2-(6-azaspiro[2.5]octan-6-yl)benzamide

At room temperature, 1-methylimidazole (72.78 mg, 886.47μmol) and N,N,N',N'-tetramethylchloroformamidinium hexafluorophosphate (248.72 mg, 886.47 μmol) were added to an acetonitrile solution (5 mL) of 4-(ethylsulfonamido)-2-(6-azaspiro[2.5]octan-6-yl)benzoic acid **7a** (100 mg, 295.49 μmol) , the system was stirred at room temperature for 1 hour, 3-(3,3-difluorocyclobutoxy)-4-(1-methyl-1H-1,2,4-triazol-3-yl)aniline **45f** (107.66 mg, 384.14 μmol) was added, and the thus obtained mixture was heated to 70°C to react for 1 hour. The reaction solution was concentrated. The crude product was separated by preparative liquid chromatography (separation column AKZONOBEL Kromasil; 250×21.2 mm I.D.; 5 μm, 20 mL/min; mobile phase A: 0.05% $NH_4HCO_3$+$H_2O$, mobile phase B: $CH_3CN$) to obtain N-(3-(3,3-difluorocyclobutoxy)-4-(1-methyl-1H-1,2,4-triazol-3-yl)phenyl)-4-(ethylsulfonamido) -2-(6-azaspiro[2.5]octan-6-yl)benzamide **45** (100.6 mg), with a yield of 53.9%.

MS m/z (ESI): 601.3 [M+1]

1H NMR (400 MHz, DMSO-d6) δ 11.65 (s, 1H), 10.17 (s, 1H), 8.64 (s, 1H), 7.83 (d, J = 8.5 Hz, 1H), 7.80 (d, J = 8.4 Hz, 1H), 7.61 (dd, J = 8.5, 1.9 Hz, 1H), 7.37 (d, J = 1.9 Hz, 1H), 7.18 (d, J = 2.1 Hz, 1H), 7.05 (dd, J = 8.5, 2.1 Hz, 1H), 4.78 (s, 1H), 3.93 (s, 3H), 3.21 (t, J = 7.7 Hz, 4H), 2.99 (t, J = 5.2 Hz, 4H), 2.87 - 2.72 (m, 2H), 1.55 (s, 4H), 1.21 (t, J = 7.3 Hz, 3H), 0.35 (s, 4H).

Example 46 was synthesized according to the synthesis method in Example 45 of the present invention, and a structure and characterization data were as shown in the following table:

| Serial number and structure of example | MS m/z (ESI) | $^1$H NMR |
|---|---|---|
| 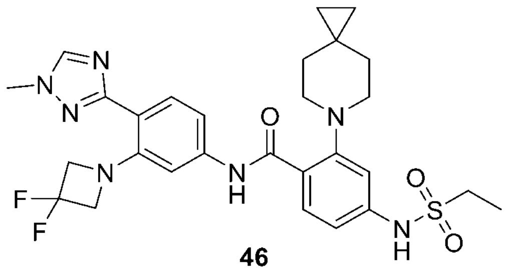 **46** | 586.3 [M+1] | 1H NMR (400 MHz, DMSO-d6) δ 11.66 (s, 1H), 10.19 (s, 1H), 8.58 (s, 1H), 7.84 (d, J = 8.5 Hz, 1H), 7.51 (d, J = 8.3 Hz, 1H), 7.28 (dd, J = 8.3, 1.9 Hz, 1H), 7.20 (dd, J = 12.0, 2.0 Hz, 2H), 7.06 (dd, J = 8.5, 2.0 Hz, 1H), 4.07 (t, J = 12.4 Hz, 4H), 3.93 (s, 3H), 3.21 (q, J = 7.3 Hz, 2H), 3.00 (t, J = 5.2 Hz, 4H), 1.63 - 1.44 (m, 4H), 1.21 (t, J = 7.3 Hz, 3H), 0.36 (s, 4H). |

Example 47

4-(ethylsulfonamido)-N-(4-(1-methyl-1H-1,2,4-triazol-3-yl)-3-(2,2,2-trifluoroethoxy)phenyl)-2-( 6-azaspiro[2.5]octan-6-yl)benzamide

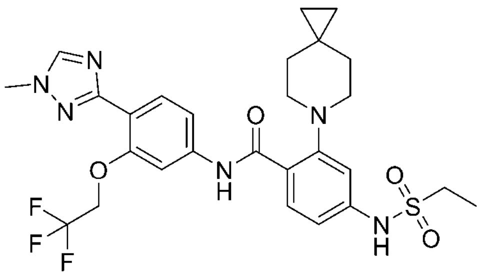

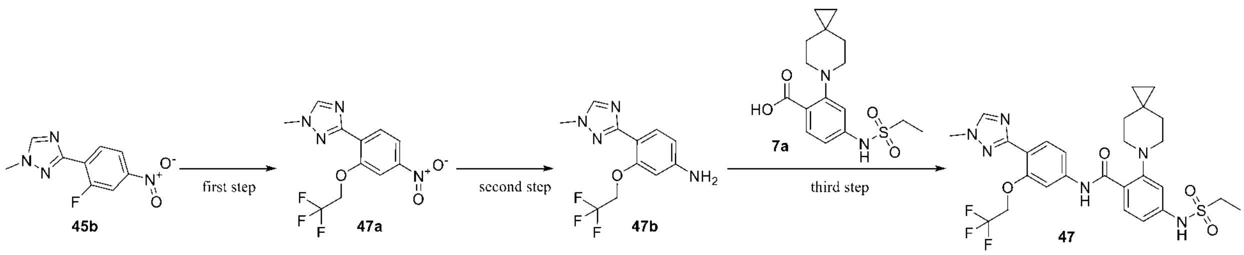


First step

1-methyl-3-(4-nitro-2-(2,2,2-trifluoroethoxy)phenyl)-1H-1,2,4-triazole

At room temperature, 2,2,2-trifluoroethanol (175.61 mg, 1.76 mmol) was dissolved in N,N-dimethylformamide (10 mL), cooled to 0°C, sodium hydride (108.01 mg, 2.70 mmol, 60% purity) was added, the mixture was stirred at 0°C for 30 minutes, 3-(2-fluoro-4-nitro-phenyl)-1-methyl-1,2,4-triazole 45b (300 mg, 1.35 mmol) was added, and the system was stirred at room temperature for 1 hour. Water (20 mL) was added to the reaction solution for quenching and the solution was extracted with dichloromethane (30 mL×2), and a combined organic phase was washed with saturated sodium chloride solution (10 mL), dried with anhydrous sodium sulfate, filtered, and concentrated under reduced pressure to obtain 1-methyl-3-(4-nitro-2-(2,2,2-trifluoroethoxy)phenyl)-1H-1,2,4-triazole **47a** (0.38 g), with a yield of 93.1%.
MS m/z (ESI): 303.1 [M+1]

Second step

4-(1-methyl-1H-1,2,4-triazol-3-yl)-3-(2,2,2-trifluoroethoxy)aniline

At room temperature, 1-methyl-3-(4-nitro-2-(2,2,2-trifluoroethoxy)phenyl)-1H-1,2,4-triazole **47a** (350 mg, 1.16 mmol) was dissolved in ethanol (10 mL), an aqueous solution (2 mL) of ammonium chloride (433.65 mg, 8.11 mmol) was added, then iron powder (323.38 mg, 5.79 mmol) was added, the system was subjected to argon purging, heated to 80°C, and stirred to react for 3 hours. The reaction solution was cooled to room temperature, tetrahydrofuran (30 mL) was added to the reaction solution, the mixture was stirred for 30 minutes, filtered, concentrated under reduced pressure, and then water (20 mL) was added, a pH value of the reaction solution was adjusted to 7-8 with saturated sodium bicarbonate solution, the reaction solution was extracted with ethyl acetate (30 mL×3), and a combined organic phase was washed with saturated sodium chloride solution (10 mL), dried with anhydrous sodium sulfate, filtered, and concentrated under reduced pressure to obtain 4-(1-methyl-1H-1,2,4-triazol-3-yl)-3-(2,2,2-trifluoroethoxy)aniline **47b** (0.3g), with a yield of 95.1%.
MS m/z (ESI): 273.1 [M+1]

Third step

4-(ethylsulfonamido)-N-(4-(1-methyl-1H-1,2,4-triazol-3-yl)-3-(2,2,2-trifluoroethoxy)phenyl)-2-( 6-azaspiro[2.5]octan-6-yl)benzamide

At room temperature, 1-methylimidazole (203.79 mg, 2.48 mmol) and N,N,N',N'-tetramethylchloroformamidinium hexafluorophosphate (696.43 mg, 2.48 mmol) were added to an acetonitrile solution (5 mL) of 4-(ethylsulfonamido)-2-(6-azaspiro[2.5]octan-6-yl)benzoic acid **7a** (280 mg, 827.37 μmol) , the system was stirred at room temperature for 1 hour, 4-(1-methyl-1H-1,2,4-triazol-3-yl)-3-(2,2,2-trifluoroethoxy)aniline **47b** (292.80 mg, 1.08 mmol) was added, the mixture was heated to 80°C, and stirred to react for 2 hours. The reaction solution was concentrated. The crude product was separated by preparative liquid chromatography (separation column AKZONOBEL Kromasil; 250×21.2 mm I.D.; 5 μm, 20 mL/min; mobile phase A: 0.05% $NH_4HCO_3$+$H_2O$, mobile phase B: $CH_3CN$) to obtain 4-(ethylsulfonamido)-N-(4-(1-methyl-1H-1,2,4-triazol-3-yl)-3-(2,2,2-trifluoroethoxy)phenyl)-2-( 6-azaspiro[2.5]octan-6-yl)benzamide **47** (288 mg), with a yield of 56.1%.

MS m/z (ESI): 593.2 [M+1]
1H NMR (400 MHz, DMSO-d6) δ 11.72 (s, 1H), 10.18 (s, 1H), 8.51 (s, 1H), 7.89-7.80 (m, 2H), 7.77 (d, J = 2.0 Hz, 1H), 7.51 (dd, J = 8.6, 1.9 Hz, 1H), 7.18 (d, J = 2.1 Hz, 1H), 7.05 (dd, J = 8.5, 2.0 Hz, 1H), 4.72 (q, J = 9.0 Hz, 2H), 3.91 (s, 3H), 3.21 (q, J = 7.3 Hz, 2H), 2.98 (t, J = 5.1 Hz, 4H), 1.54 (s, 4H), 1.21 (t, J = 7.3 Hz, 3H), 0.35 (s, 4H).

Examples 48-54 were synthesized according to the synthesis methods in Examples 45 and 47 of the present invention, and structures and characterization data were as shown in the following table:

| Serial number and structure of example | MS m/z (ESI) | $^1$H NMR |
|---|---|---|
| 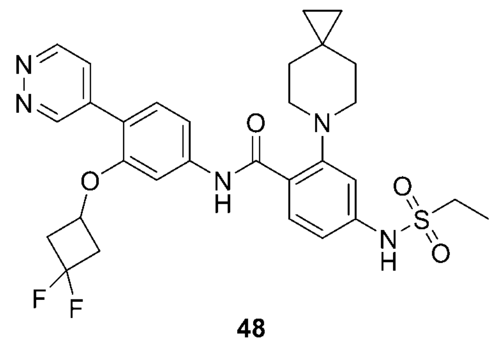 **48** | 598.0 [M+1] | $^1$H NMR (500 MHz, DMSO-$d_6$) δ 11.71 (s, 1H), 10.18 (s, 1H), 9.51 - 9.46 (m, 1H), 9.28 - 9.15 (m, 1H), 7.91 - 7.87 (m, 1H), 7.83 (d, *J* = 6.8 Hz, 1H), 7.67 - 7.62 (m, 2H), 7.45 (d, *J* = 1.2 Hz, 1H), 7.18 (d, *J* = 2.0 Hz, 1H), 7.12 - 6.99 (m, 1H), 4.87 - 4.77 (m, 1H), 3.24 - 3.17 (m, 4H), 3.01 - 2.96 (m, 4H), 2.89 - 2.80 (m, 2H), 1.60 - 1.50 (m, 4H), 1.21 (t, *J* = 6.0 Hz, 3H), 0.35 (s, 4H). |

(continued)

| Serial number and structure of example | MS m/z (ESI) | $^1$H NMR |
|---|---|---|
| 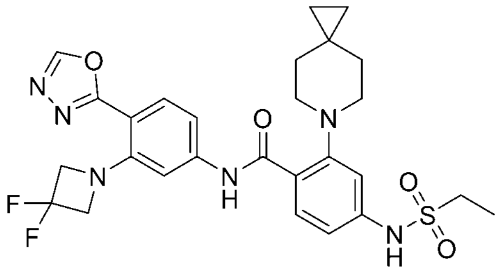 49 | 573.2 [M+1] | |
| 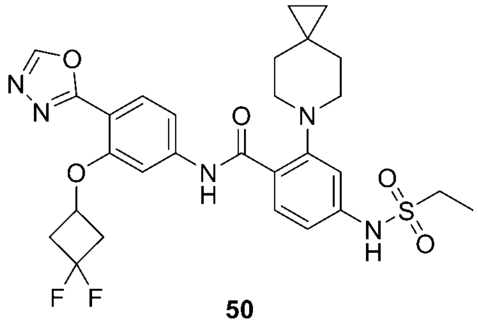 50 | 588.4 [M+1] | 1H NMR (400 MHz, ) δ 11.74 (s, 1H), 10.17 (s, 1H), 7.93 (d, J = 8.6 Hz, 1H), 7.83 - 7.77 (m, 3H), 7.67 (dd, J = 8.5, 1.8 Hz, 1H), 7.17 (d, J = 2.1 Hz, 1H), 7.04 (dd, J = 8.5, 2.1 Hz, 1H), 4.91-4.88 (m, 1H), 3.21 (d, J = 7.4 Hz, 2H), 2.42 (s, 4H), 1.54 (s, 4H), 1.24 - 1.20 (m, 4H), 1.18 (d, J = 7.1 Hz, 3H), 0.35 (s, 4H). |
| 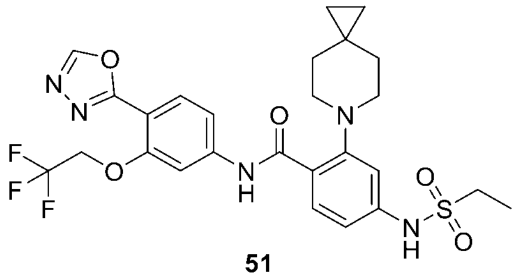 51 | 580.2 [M+1] | 1H NMR (400 MHz, DMSO-d6) δ 12.75 (s, 1H), 9.33 (s, 1H), 7.98 - 7.91 (m, 2H), 7.74 (d, J = 8.7 Hz, 1H), 7.46 (dd, J = 8.5, 1.8 Hz, 1H), 6.92 (d, J = 2.1 Hz, 1H), 6.77 (dd, J = 8.7, 2.1 Hz, 1H), 4.90 (q, J = 8.7 Hz, 2H), 2.96 (t, J = 5.3 Hz, 4H), 2.84 (q, J = 7.3 Hz, 2H), 1.59 (s, 4H), 1.15 (t, J = 7.3 Hz, 3H), 0.38 (s, 4H). |
| 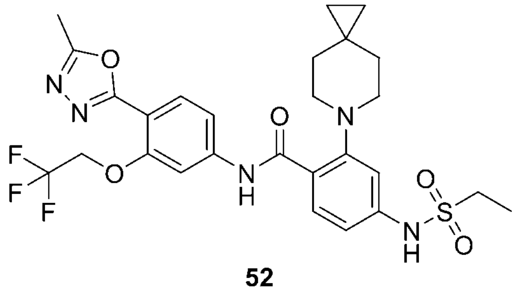 52 | 594.0 [M+1] | $^1$H NMR (400 MHz, DMSO-d6) δ 12.91 (s, 1H), 7.95 - 7.84 (m, 2H), 7.71 (d, J = 8.8 Hz, 1H), 7.44 (dd, J = 8.6, 1.9 Hz, 1H), 6.85 (d, J = 2.1 Hz, 1H), 6.70 (dd, J = 8.7, 2.0 Hz, 1H), 4.87 (q, J = 8.7 Hz, 2H), 2.94 (t, J = 5.4 Hz, 4H), 2.75 (q, J = 7.3 Hz, 2H), 1.77 (s, 3H), 1.59 (s, 4H), 1.12 (t, J = 7.3 Hz, 3H), 0.38 (s, 4H). |
| 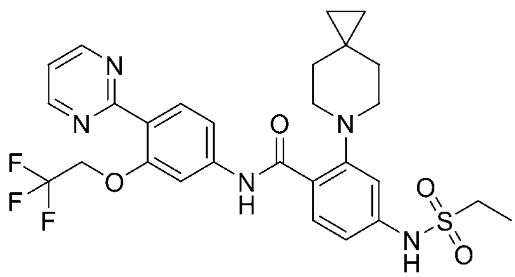 53 | 590.1 [M+1] | 1H NMR (400 MHz, DMSO-d6) δ 12.87 (s, 1H), 8.88 (d, J = 4.8 Hz, 2H), 7.84 - 7.76 (m, 2H), 7.71 (d, J = 8.7 Hz, 1H), 7.49 (dd, J = 8.5, 1.9 Hz, 1H), 7.41 (t, J = 4.9 Hz, 1H), 6.84 (d, J = 2.1 Hz, 1H), 6.68 (dd, J = 8.7, 2.1 Hz, 1H), 4.73 (q, J = 8.9 Hz, 2H), 2.95 (t, J = 5.3 Hz, 4H), 2.72 (q, J = 7.4 Hz, 2H), 1.65 (s, 4H), 1.12 (t, J = 7.4 Hz, 3H), 0.39 (s, 4H). |

(continued)

| Serial number and structure of example | MS m/z (ESI) | $^1$H NMR |
|---|---|---|
| 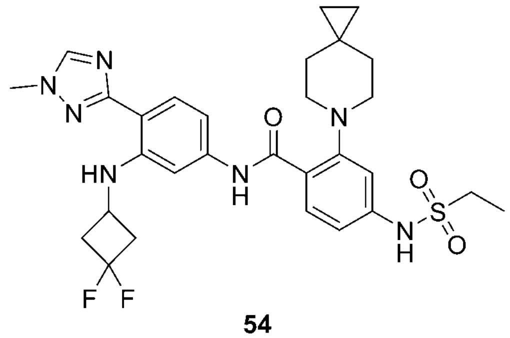<br>**54** | 600.1 [M+1] | 1H NMR (400 MHz, DMSO-d6) δ 11.75 (s, 1H), 10.18 (s, 1H), 8.56 (s, 1H), 7.98 (d, J = 8.5 Hz, 1H), 7.92 (d, J = 5.0 Hz, 1H), 7.87 (d, J = 8.5 Hz, 1H), 7.37 (dd, J = 8.5, 1.9 Hz, 1H), 7.18 (d, J = 2.2 Hz, 1H), 7.05 (dd, J = 8.5, 2.1 Hz, 1H), 6.89 (d, J = 2.0 Hz, 1H), 3.95 (s, 3H), 3.93-3.86 (m, 1H), 3.24-3.14 (m, 4H), 2.98 (t, J = 5.2 Hz, 4H), 2.65-2.52 (m, 2H), 1.59 (t, J = 5.1 Hz, 4H), 1.21 (t, J = 7.3 Hz, 3H), 0.37 (s, 4H). |

Example 55

N-(3-(4,4-difluoropiperidin-1-yl)-4-(pyridin-2-yloxy)phenyl)-4-(ethylsulfonamido)-2-(6-azaspiro [2.5]octan-6-yl)benzamide

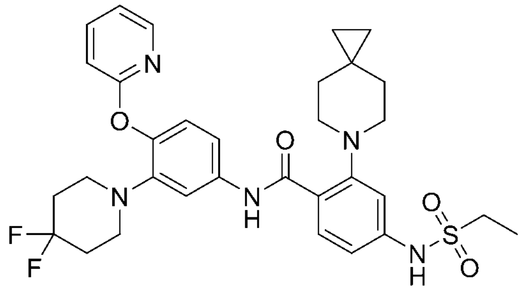

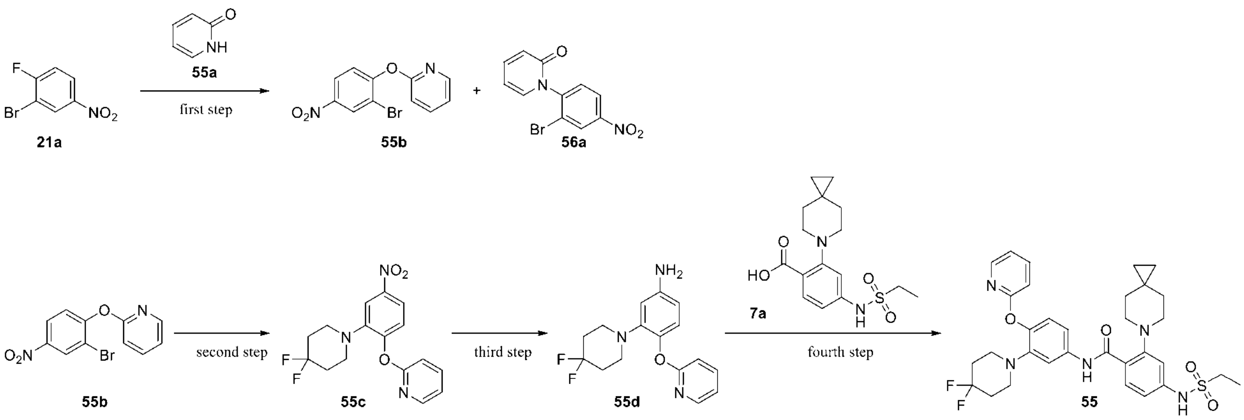


First step

2-(2-bromo-4-nitrophenoxy)pyridine

At 0°C, 1H-pyridin-2-one **55a** (358.66 mg, 3.77 mmol, commercially available) was added into an N,N-dimethylformamide solution (10 mL), potassium tert-butoxide (529.00 mg, 4.71 mmol) and 2-bromo-1-fluoro-4-nitrobenzene **21a** (500 mg, 3.14 mmol) were added, the system was subjected to nitrogen purging for three times, and slowly heated to room temperature to react for 18 hours. The mixture was poured into water (10 mL), the mixture was extracted with ethyl acetate (10 mL×3), and a combined organic phase was washed with saturated sodium chloride solution (20 mL),

dried with anhydrous sodium sulfate, filtered, and concentrated under reduced pressure. The crude product was separated and purified by silica gel column chromatography (eluent: A system) to obtain 1-(2-bromo-4-nitrophenyl) pyridin-2(1H)-one **56a** (200 mg), with a yield of 27.2%; and 2-(2-bromo-4-nitrophenoxy)pyridine **55b** (300 mg, 1.28 mmol, 40.76% yield), with a yield of 40.8%.

MS m/z (ESI): 297.0 [M+1]
HNMR of **56a**
$^1$H NMR (400 MHz, DMSO-$d_6$) δ 8.64 (d, *J* = 2.8 Hz, 1H), 8.41 - 8.33 (m, 1H), 7.83 (d, *J* = 8.4 Hz, 1H), 7.62 - 7.56 (m, 2H), 6.58 - 6.50 (m, 1H), 6.43 - 6.38 (m, 1H).
HNMR of **55b**
$^1$H NMR (400 MHz, DMSO-$d_6$) δ 8.57 (d, *J* = 2.8 Hz, 1H), 8.32 - 8.26 (m, 1H), 8.18 - 8.13 (m, 1H), 7.99 - 7.94 (m, 1H), 7.51 (d, *J* = 8.8 Hz, 1H), 7.28 - 7.21 (m, 2H).

Second step

2-(2-(4,4-difluoropiperidin-1-yl)-4-nitrophenoxy)pyridine

At room temperature, palladium acetate (22.82 mg, 101.66 μmol), 4,5-bis(diphenylphosphino)-9,9-dimethylxanthene (117.65 mg, 203.33 μmol) and cesium carbonate (662.49 mg, 2.03 mmol) were added to a 1,4- dioxane solution (3 mL) of 2-(2-bromo-4-nitrophenoxy)pyridine **55b** (300 mg, 1.02 mmol) and 4,4-difluorpiperidine **1b** (492.58 mg, 4.07 mmol) , and the system was subjected to nitrogen purging for three times. The reaction was carried out at 100°C for 18 hours. The reaction solution was concentrated under reduced pressure. The crude product was separated and purified by silica gel column chromatography (eluent: A system) to obtain 2-(2-(4,4-difluoropiperidin-1-yl)-4-nitrophenoxy)pyridine **55c** (130 mg), with a yield of 38.1%.
MS m/z (ESI): 336.0 [M+1]

Third step

3-(4,4-difluoropiperidin-1-yl)-4-(pyridin-2-yloxy)aniline

At room temperature, palladium on carbon (10%) (47.61 mg, 447.35 μmol) was added into a methanol solution (3 mL) of 2-(2-(4,4-difluoropiperidin-1-yl)-4-nitrophenoxy)pyridine **55c** (150 mg, 447.35 μmol), and the system was subjected to hydrogen purging for three times. Then, the reaction was carried out at 25°C for 2 hours. The reaction solution was filtered, the filter cake was washed with methanol (10 mL), and the filtrate was concentrated under reduced pressure to obtain 3-(4,4-difluoropiperidin-1-yl)-4-(pyridin-2-yloxy)aniline **55d** (130 mg), with a yield of 95.2%.
MS m/z (ESI): 306.2 [M+1]

Fourth step

N-(3-(4,4-difluoropiperidin-1-yl)-4-(pyridin-2-yloxy)phenyl)-4-(ethylsulfonamido)-2-(6-azaspiro [2.5]octan-6-yl)benzamide

At room temperature, 3-(4,4-difluoropiperidin-1-yl)-4-(pyridin-2-yloxy)aniline **55d** (130 mg, 425.78 μmol) and 4-(ethylsulfonamido)-2-(6-azaspiro[2.5]octan-6-yl)benzoic acid **7a** (172.91 mg, 510.94 μmol) were added into N,N-dimethylformamide (3 mL), then (7-azabenzotriazole-1-yloxy)tripyrrolidinophosphonium hexafluorophosphate (333.00 mg, 638.67 μmol) and N,N-diisopropylethylamine (110.06 mg, 851.56 μmol) were added, and the system was subjected to nitrogen purging for three times. The reaction was carried out at 65°C for 18 hours. The mixture was poured into water (10 mL), the mixture was extracted with ethyl acetate (10 mL×3), and a combined organic phase was washed with brine (20 mL), dried with anhydrous sodium sulfate, filtered, and concentrated to dryness under reduced pressure. The crude product was separated by preparative liquid chromatography (separation column AKZONOBEL Kromasil; 250×21.2 mm I.D.; 5 μm, 20 mL/min; mobile phase A: 0.05% TFA+$H_2O$, mobile phase B: $CH_3CN$) to obtain N-(3-(4,4-difluoropiperidin-1-yl)-4-(pyridin-2-yloxy)phenyl)-4-(ethylsulfonamido)-2-(6-azaspiro [2.5]octan-6-yl)benzamide **55** (130 mg), with a yield of 48.8%.

MS m/z (ESI): 626.2 [M+1]
$^1$H NMR (400 MHz, DMSO-$d_6$) δ 11.75 (s, 1H), 10.16 (s, 1H), 8.14 - 8.11 (m, 1H), 7.85 (d, *J* = 8.4 Hz, 1H), 7.83 - 7.78 (m, 1H), 7.63 (d, *J* = 2.4 Hz, 1H), 7.43 - 7.39 (m, 1H), 7.19 (d, *J* = 2.0 Hz, 1H), 7.13 (d, *J* = 8.8 Hz, 1H), 7.11 - 7.08 (m, 1H), 7.07 - 7.04 (m, 1H), 6.94 (d, *J* = 8.0 Hz, 1H), 3.24 - 3.17 (m, 2H), 3.07 - 3.03 (m, 4H), 3.01 - 2.96 (t, *J* = 5.3 Hz, 4H), 1.83 -

1.73 (m, 4H), 1.61 - 1.54 (m, 4H), 1.21 (t, *J* = 7.2 Hz, 3H), 0.36 (s, 4H).

Example 56 was synthesized according to the synthesis method in Example 55 of the present invention, and a structure and characterization data were as shown in the following table:

| Serial number and structure of example | MS m/z (ESI) | $^1$H NMR |
|---|---|---|
| 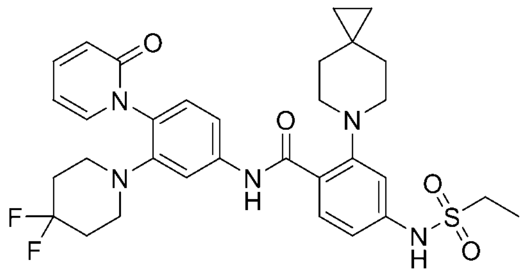 **56** | 626.2 [M+1] | $^1$H NMR (400 MHz, DMSO-$d_6$) δ 11.84 (s, 1H), 10.31 (s, 1H), 7.83 (d, *J* = 8.8 Hz, 1H), 7.78 (d, *J* = 2.0 Hz, 1H), 7.58 - 7.50 (m, 2H), 7.49 - 7.45 (m, 1H), 7.26 (d, *J* = 8.4 Hz, 1H), 7.16(d, *J* = 2.4 Hz, 1H), 7.05 - 7.00 (m, 1H), 6.55 - 6.49 (m, 1H), 6.33 - 6.28 (m, 1H), 3.20 - 3.14 (m, 2H), 3.01 - 2.96 (m, 4H),2.95 - 2.84 (m, 4H), 1.96 - 1.78 (m, 4H), 1.57 (s, 4H), 1.20 (t, *J* = 7.2 Hz, 3H), 0.36 (s, 4H). |

Example 57

N-(3-(4,4-difluoropiperidin-1-yl)-4-(methoxy-d3)phenyl)-4-(ethylsulfonamido)-2-(6-azaspiro[2. 5]octan-6-yl)benzamide

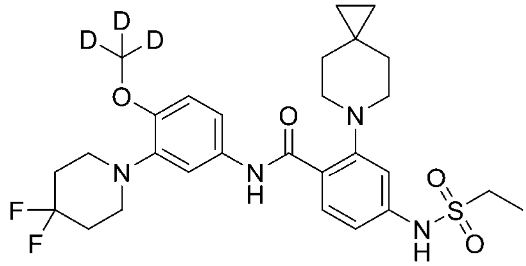

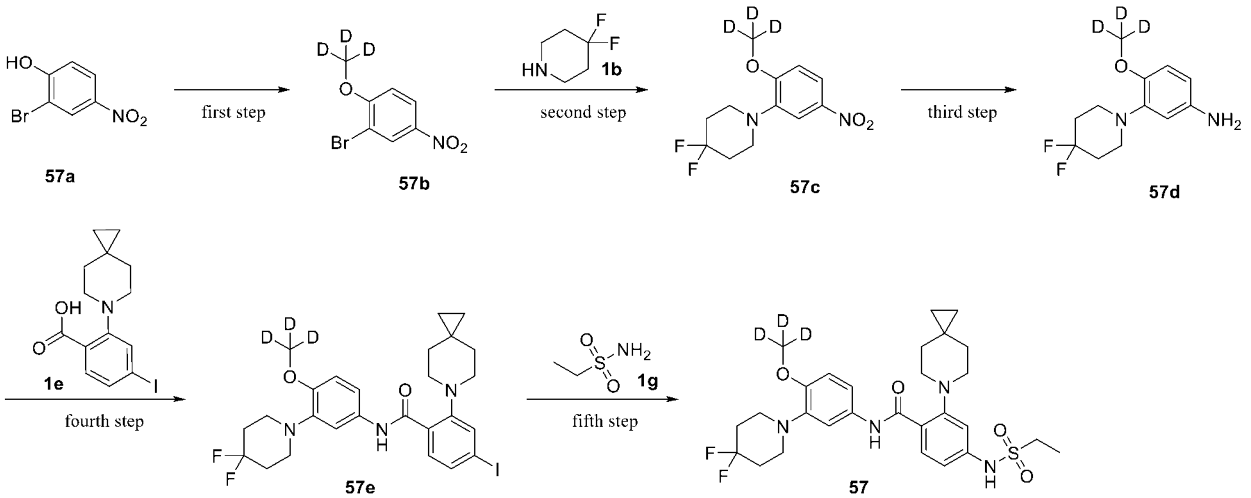


First step

2-bromo-1-(methoxy-d3)-4-nitrobenzene

At room temperature, 2-bromo-4-nitro-phenol **57a** (1.0 g, 4.59 mmol, commercially available) and potassium

carbonate (1.27 g, 9.17 mmol) were dissolved in N,N-dimethylformamide (20 mL), then triiodomethane (997.39 mg, 6.88 mmol) was added, and the mixture was stirred at room temperature for 6 hours. The reaction solution was concentrated to dryness. The obtained crude product was separated and purified by silica gel column chromatography (eluent: A system) to obtain 2-bromo-1-(methoxy-d3)-4-nitrobenzene **57b** (1.05 g), with a yield of 97.4%.
MS m/z (ESI): 234.8 [M+1]

Second step

4,4-difluoro-1-(2-(methoxy-d3)-5-nitrophenyl)piperidine

At room temperature, 2-bromo-1-(methoxy-d3)-4-nitrobenzene **57b** (1.0 g, 4.25 mmol) and 4,4-difluoropiperidine (772.99 mg, 6.38 mmol) were dissolved in toluene (20 mL), then tris(dibenzylideneacetone)dipalladium (194.79 mg, 212.72 μmol), R-(+)-1,1'-binaphthyl-2,2'-bis(diphenylphosphine) (264.91 mg, 425.44 μmol) and sodium tert-butoxide (1.23 g, 12.76 mmol) were added, the system was subjected to argon purging for 5 minutes, heated to 100°C, and stirred to react for 18 hours. Water (20 mL) was added to the reaction solution and the system was extracted with ethyl acetate (50 mL×3), and a combined organic phase was washed with saturated sodium chloride solution (20 mL), dried with anhydrous sodium sulfate, filtered and concentrated under reduced pressure. The crude product was separated and purified by silica gel column chromatography (eluent: A system) to obtain 4,4-difluoro-1-(2-(methoxy-d3)-5-nitrophenyl)piperidine **57c** (0.87 g), with a yield of 74.3%.
MS m/z (ESI): 276.1 [M+1]

Third step

3-(4,4-difluoropiperidin-1-yl)-4-(methoxy-d3)aniline

At room temperature, 4,4-difluoro-1-(2-(methoxy-d3)-5-nitrophenyl)piperidine **57c** (870 mg, 3.16 mmol) was dissolved in methanol (50 mL), then 10% palladium on carbon (336.35 mg, 3.16 mmol) was added, the system was subjected to hydrogen purging for 3 times, and stirred at room temperature for 18 hours. The reaction solution was filtered under reduced pressure, and the filtrate was concentrated to obtain 3-(4,4-difluoropiperidin-1-yl)-4-(methoxy-d3)aniline **57d** (0.75 g), with a yield of 96.7%.
MS m/z (ESI): 245.9 [M+1]

Fourth step

N-(3-(4,4-difluoropiperidin-1-yl)-4-(methoxy-d3)phenyl)-4-iodo-2-(6-azaspiro[2.5]octan-6-yl)be nzamide

At room temperature, 2-(6-azaspiro[2.5]octan-6-yl)-4-iodo-benzoic acid **1e** (780 mg, 2.18 mmol) was dissolved in acetonitrile (50 mL), then 1-methylimidazole (537.88 mg, 6.55 mmol) and N,N,N',N'-tetramethylchloroformamidinium hexafluorophosphate (1.84 g, 6.55 mmol) were added, the mixture was stirred for 1 hour, then 3-(4,4-difluoropiperidin-1-yl)-4-(methoxy-d3)aniline **57d** (696.32 mg, 2.84 mmol) was added, the thus obtained mixture was heated to 60°C, and stirred for 3 hours. The reaction solution was concentrated under reduced pressure. The crude product was separated and purified by silica gel column chromatography (eluent: A system) to obtain N-(3-(4,4-difluoropiperidin-1-yl)-4-(methoxy-d3) phenyl)-4-iodo-2-(6-azaspiro[2.5]octan-6-yl)be nzamide **57e** (0.7 g), with a yield of 54.8%.
MS m/z (ESI): 585.2 [M+1]

Fifth step

N-(3-(4,4-difluoropiperidin-1-yl)-4-(methoxy-d3)phenyl)-4-(ethylsulfonamido)-2-(6-azaspiro[2. 5]octan-6-yl)benzamide

At room temperature, N-(3-(4,4-difluoropiperidin-1-yl)-4-(methoxy-d3)phenyl)-4-iodo-2-(6-azaspiro[2.5]octan-6-yl)be nzamide **57e** (700 mg, 1.20 mmol) and ethyl sulfonamide **1g** (196.09 mg, 1.80 mmol) were dissolved in N,N-dimethylformamide (5 mL), then cuprous iodide (114.05 mg, 598.85 μmol), 2-(methylamino)acetic acid (106.71 mg, 1.20 mmol) and tripotassium phosphate trihydrate (956.89 mg, 3.59 mmol) were added, the system was subjected to argon purging for 3 times, heated to 110°C, and stirred for 6 hours. The reaction solution was concentrated, and then separated by preparative liquid chromatography (separation column AKZONOBEL Kromasil; 250×21.2 mm I.D.; 5 μm, 20 mL/min; mobile phase A: 0.05% $NH_4CO_3$+$H_2O$, mobile phase B: $CH_3CN$) to obtain N-(3-(4,4-difluoropiperidin-1-yl)-4-(methoxy-d3)phenyl)-4-(ethylsulfonamido)-2-(6-azaspiro[2. 5]octan-6-yl)benzamide **57** (360 mg), with a yield of 52.2%.

MS m/z (ESI): 566.3 [M+1]

$^1$H NMR (400 MHz, DMSO-d6) δ 11.60 (s, 1H), 10.13 (s, 1H), 7.83 (d, J = 8.4 Hz, 1H), 7.43 (d, J = 2.5 Hz, 1H), 7.37 (dd, J = 8.7, 2.4 Hz, 1H), 7.17 (d, J = 2.2 Hz, 1H), 7.04 (dd, J = 8.5, 2.1 Hz, 1H), 6.96 (d, J = 8.6 Hz, 1H), 3.19 (q, J = 7.3 Hz, 2H), 3.10 (t, J = 5.9 Hz, 4H), 2.96 (t, J = 5.4 Hz, 4H), 2.16-2.05 (m, 4H), 1.60 - 1.47 (m, 4H), 1.21 (t, J = 7.3 Hz, 3H), 0.35 (s, 4H).

Example 58 was synthesized according to the synthesis method in Example 57 of the present invention, and a structure and characterization data were as shown in the following table:

| Serial number and structure of example | MS m/z (ESI) | $^1$H NMR |
|---|---|---|
| 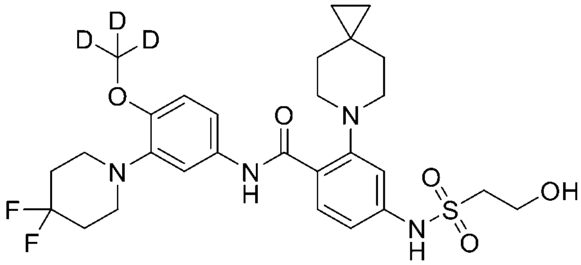 **58** | 582.3 [M+1] | 1H NMR (400 MHz, DMSO-d6) δ 11.82 (s, 1H), 7.81 (d, J = 8.5 Hz, 1H), 7.43 (d, J = 2.5 Hz, 1H), 7.37 (dd, J = 8.7, 2.4 Hz, 1H), 7.09 (d, J = 2.1 Hz, 1H), 6.99 - 6.92 (m, 2H), 3.74 (t, J = 6.6 Hz, 2H), 3.24 (t, J = 6.6 Hz, 2H), 3.11 (t, J = 5.6 Hz, 4H), 2.95 (t, J = 5.3 Hz, 4H), 2.21-2.03 (m, 4H), 1.56 (s, 4H), 0.36 (s, 4H). |

## Biological evaluation

### Test Example 1. Determination of the inhibition of the compounds of the present invention on OVCAR-3 cell proliferation

Influences of the compounds of the present invention on OVCAR-3 cell proliferation were determined by the following method. OVCAR-3 cells (containing TP53 R248Q mutation) were purchased from the Cell Bank of Shanghai Institute of Biochemistry and Cell Biology, Chinese Academy of Sciences, and cultured in an RPMI 1640 culture medium containing 10% fetal bovine serum, 100 U penicillin and 100 μg/mL streptomycin. A cell viability was determined by a CellTiter-Glo® Luminescent Cell Viability Assay kit (Promega, article number G7573).

An experimental method was operated according to steps in the instruction of the kit, which was briefly described as follows: a test compound was dissolved in DMSO first to prepare a 10 mM stock solution, then the stock solution was diluted with the culture medium to prepare a test sample, and a final concentration of the compound was in a range of 1000 nM-0.015 nM. Cells in a logarithmic growth phase were inoculated into a 96-well cell culture plate at a density of 1000 cells per well, cultured in a 5% $CO_2$ incubator at 37°C overnight, and then the test compound was added to continuously culture for 72 hours. After the culture was ended, 50 uL of CellTiter-Glo detection solution was added to each well , shaken for 5 minutes, and then allowed to stand for 10 minutes, and subsequently, a luminance value of each well of the sample was read by a microplate reader in a Luminescence mode. By comparing with the numerical value of a control group (0.3% DMSO), a percentage inhibition rate of the compound at each concentration point was calculated, and then nonlinear regression analysis was carried out based on concentration logarithm-inhibition rate of the compound in GraphPad Prism 5 software, so as to obtain an $IC_{50}$ value of the compound for inhibiting the cell proliferation, which was as shown in Table 1.

Table 1 $IC_{50}$ data of the compounds of the present invention for inhibiting OVCAR-3 cell proliferation

| Serial number of example | $IC_{50}$ (nM) | Serial number of example | $IC_{50}$ (nM) | Serial number of example | $IC_{50}$ (nM) |
|---|---|---|---|---|---|
| Control compound AMG 650 | 64.5 | **18** | 6.5 | **39** | 3.8 |
| **1** | 16.5 | **19** | 6.3 | **40** | 6.0 |
| **2** | 42.9 | **20** | 8.5 | **41** | 6.2 |
| **3** | 3.7 | **22** | 19.9 | **43** | 18.8 |
| **4** | 18.9 | **23** | 51.3 | **44** | 45.5 |
| **5** | 33.1 | **24** | 17.6 | **45** | 2.5 |

(continued)

| Serial number of example | $IC_{50}$ (nM) | Serial number of example | $IC_{50}$ (nM) | Serial number of example | $IC_{50}$ (nM) |
|---|---|---|---|---|---|
| **6** | 11.1 | **25** | 3.5 | **46** | 3.7 |
| **7** | 11.1 | **27** | 8.1 | **47** | 5.8 |
| **8** | 21.2 | **28** | 12.4 | **48** | 14.4 |
| **9** | 15.2 | **29** | 15.9 | **49** | 12.6 |
| **10** | 17.3 | **30** | 13.3 | **50** | 19.1 |
| **11** | 17.7 | **31** | 15.9 | **51** | 27.4 |
| **12** | 2.5 | **32** | 5.9 | **52** | 17.9 |
| **13** | 5.9 | **34** | 49.8 | **53** | 9.5 |
| **14** | 5.9 | **35** | 11.1 | **54** | 38.9 |
| **15** | 3.7 | **36** | 11.1 | **55** | 44.7 |
| **16** | 6.2 | **37** | 3.6 | **57** | 8.7 |
| **17** | 3.2 | **38** | 27.1 | **58** | 2.2 |

Conclusion: the compounds of the present invention have a relatively good inhibitory effect on OVCAR-3 cell proliferation $IC_{50}$<50 nM.

Note: a structure of the AMG 650 (prepared in Example 4 according to the published patent WO2020132648A1) was as follows:

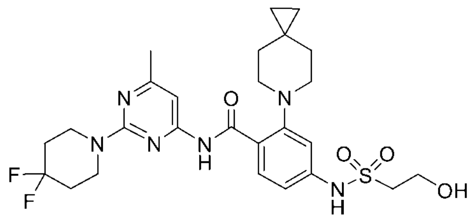

**Test Example 2. Determination of the inhibition of the compounds of the present invention on HT-29 cell proliferation**

Influences of the compounds of the present invention on HT-29 cell proliferation were determined by the following method. HT-29 cells (containing TP53 R273H mutation) were purchased from the Cell Bank of Shanghai Institute of Biochemistry and Cell Biology, Chinese Academy of Sciences, and cultured in a McCOY's 5A culture medium containing 10% fetal bovine serum, 100 U penicillin and 100 μg/mL streptomycin. A cell viability was determined by a CellTiter-Glo® Luminescent Cell Viability Assay kit (Promega, article number G7573).

An experimental method was operated according to steps in the instruction of the kit, which was briefly described as follows: a test compound was dissolved in DMSO first to prepare a 10 mM stock solution, then the stock solution was diluted with the culture medium to prepare a test sample, and a final concentration of the compound was in a range of 1000 nM-0.015 nM. Cells in a logarithmic growth phase were inoculated into a 96-well cell culture plate at a density of 1000 cells per well, cultured in a 5% $CO_2$ incubator at 37°C overnight, and then the test compound was added to continuously culture for 120 hours. After the culture was ended, 50 uL of CellTiter-Glo detection solution was added to each well, shaken for 5 minutes, and then allowed to stand for 10 minutes, and subsequently, a luminance value of each well of the sample was read by a microplate reader in a Luminescence mode. By comparing with the numerical value of a control group (0.3% DMSO), a percentage inhibition rate of the compound at each concentration point was calculated, and then nonlinear regression analysis was carried out based on concentration logarithm-inhibition rate of the compound in GraphPad Prism 5 software, so as to obtain an $IC_{50}$ value of the compound for inhibiting the cell proliferation, which was as shown in Table 2.

Table 2 $IC_{50}$ data of the compounds of the present invention for inhibiting HT-29 cell proliferation

| Serial number of example | $IC_{50}$ (nM) | Serial number of example | $IC_{50}$ (nM) | Serial number of example | $IC_{50}$ (nM) |
|---|---|---|---|---|---|
| Control compound AMG 650 | 61.7 | **18** | 0.64 | **39** | 0.26 |
| **1** | 10.2 | **19** | 0.44 | **40** | 8.2 |
| **3** | 4 | **20** | 6.0 | **41** | 1.8 |
| **6** | 13.3 | **22** | 16.8 | **45** | 6.2 |
| **7** | 18.3 | **25** | 2.6 | **46** | 0.15 |
| **9** | 14.5 | **26** | 30.8 | **47** | 0.54 |
| **10** | 27.1 | **28** | 8.4 | **49** | 9.6 |
| **11** | 16.3 | **29** | 18.4 | **53** | 6.8 |
| **12** | 3.9 | **30** | 7.9 | **57** | 4.5 |
| **13** | 11.5 | **31** | 8.3 | **58** | 1.5 |
| **14** | 10.1 | **32** | 4.7 | | |
| **15** | 4.7 | **35** | 6.2 | | |
| **16** | 4.6 | **36** | 9.3 | | |
| **17** | 3.3 | **37** | 3.8 | | |

Conclusion: the compounds of the present invention have a relatively good inhibitory effect on HT-29 cell proliferation $IC_{50}$<50 nM.

**Test Example 3. Testing of the inhibition of the compounds of the present invention on KIF18A enzyme activity**

Inhibition degrees of the compounds of the present invention on recombinant human KIF18A enzyme activity in vitro were determined by the following method. An ADP-Glo™ Kinase Assay kit (article number V9102) from Promega Company was used in this method. Detailed experimental operation referred to the instruction of the kit.

An experimental process was briefly described as follows: a test compound was dissolved in DMSO first to prepare a stock solution, then the stock solution was gradiently diluted with a reaction buffer A (15 mm Tris, pH 7.5, 10 mm $MgCl_2$, 0.01% Pluronic F-68), and a final concentration of the test compound in the reaction system was in a range of 10000 nM-0.15 nM; and a KIF18A protein and ATP working solution was prepared by using a reaction buffer B (15 mM Tris, pH 7.5, 10 mM $MgCl_2$, 0.01% Pluronic F-68, 37.5 μg/ml tubulin, 1.25 μM paclitaxel). The reaction was carried out in a 384-well microplate. The test compound and the recombinant human KIF18A protein (final concentration of 100 nM, entrusted to GenScript for expression) were added into the wells and incubated at room temperature for 20 minutes, and subsequently, an ATP solution (component V915A from the ADP-Glo™ Kinase Assay kit, final concentration of 60 μM) was added to the reaction solution and the system was incubated at room temperature for 20 minutes. Subsequently, 5 μL of ADP-Glo Reagent was added to the reaction systemand the system was incubated at room temperature for 50 minutes. Then, 10 μL of Kinase Detection Reagent was added to the reaction system and the system was incubated at room temperature for 30 minutes. After the incubation was ended, a chemiluminiscence intensity value of each well was determined by a microplate reader in a Luminescence mode. By comparing with the luminous intensity value of a control group (0.1% DMSO), a percentage inhibition rate of the compound at each concentration was calculated, and nonlinear regression analysis was carried out based on concentration logarithm value-inhibition rate of the compound in GraphPad Prism 5 software, so as to obtain an $IC_{50}$ value of the compound, which can be seen in Table 3.

Table 3 $IC_{50}$ data of the compounds of the present invention for inhibiting KIF18A enzyme activity

| Serial number of example | $IC_{50}$ (nM) | Serial number of example | $IC_{50}$ (nM) | Serial number of example | $IC_{50}$ (nM) |
|---|---|---|---|---|---|
| Control compound AMG 650 | 173 | **26** | 106 | **43** | 115 |
| **2** | 29.5 | **27** | 30.9 | **44** | 130 |

(continued)

| Serial number of example | $IC_{50}$ (nM) | Serial number of example | $IC_{50}$ (nM) | Serial number of example | $IC_{50}$ (nM) |
|---|---|---|---|---|---|
| **12** | 62.4 | **28** | 59.1 | **45** | 103 |
| **13** | 82.9 | **29** | 79.5 | **46** | 148 |
| **14** | 120 | **30** | 79 | **47** | 155 |
| **15** | 69.4 | **31** | 134 | **48** | 102 |
| **16** | 147 | **33** | 3.8 | **49** | 70.4 |
| **17** | 121 | **34** | 90 | **50** | 74.3 |
| **18** | 149 | **37** | 39.8 | **51** | 107 |
| **19** | 98.6 | **39** | 122 | **52** | 125 |
| **20** | 50.8 | **41** | 155 | **53** | 142 |
| **25** | 33.9 | **42** | 88 | **58** | 91.7 |

Conclusion: the compounds of the present invention have a significant inhibitory effect on KIF18A enzyme activity $IC_{50}$<200 nM.

**Test Example 4. Pharmacokinetic testing of the compounds of the present invention in mice**

1. Experimental purpose

ICR mice were taken as test animals, and intragastrically administered with a control compound AMG650 and compounds 47 and 53 of the present invention, and then drug concentrations in plasma at different moments were determined by an LC/MS/MS method, so as to study pharmacokinetic characteristics of the compounds of the present invention in the mice.

2. Experimental scheme

2.1 Experimental drugs and animals

Control compound AMG650, and Compounds 47 and 53.
ICR mice, male, 20-22 g, purchased from Vital River Laboratory Animal Technology Co., Ltd.

2.2 Preparation of drugs

A proper amount of to-be-tested compound was weighed, proper amounts of DMA, 30% HS-15 and Saline were added in sequence, and the mixture was well mixed by ultrasonic vortex to prepare 1 mg/mL drug preparations respectively, wherein DMSO: 30% HS-15: Saline=5: 5: 90 (v: v: v).

2.3 Administration

ICR mice in each to-be-tested compound injection group (9 mice in each group) were fasted overnight and then intragastrically administered with the compound (PO, an administration dosage of the compound was 10 mg/kg and an administration volume of the compound was 10 mL/kg), and were fed 4 hours after administration.

3. Operation

0.25 hours, 0.5 hours, 1 hour, 2 hours, 4 hours, 6 hours, 8 hours and 24 hours before and after administration, about 0.1 μL of blood was collected through an orbit, and a complete blood sample was placed in an EDTA-K2-containing anticoagulation tube. The collected blood samples were placed on ice, and plasma was centrifugally separated (centrifugation conditions: 1500 g, 10 minutes). The collected plasma was stored at -40°C - -20°C before analysis.
A content of the to-be-tested compound in the plasma of the mice after intragastric administration was determined by LC-MS/MS.

4. Results of pharmacokinetic parameters

Pharmacokinetic parameters of to-be-tested compounds can be seen in Table 4 below.

Table 4 Pharmacokinetic parameters of to-be-tested compounds in mice

| Serial number of compound | Pharmacokinetic experiment | | | |
|---|---|---|---|---|
| | Administration mode (Administration dosage) | Plasma concentration Cmax (ng/mL) | Area under the curve $AUC_{0-\infty}$ (ng·h/mL) | Half-life period T1/2 (h) |
| Control compound AMG650 | Oral administration (10 mg/kg) | 3050 | 52500 | 9.64 |
| Example 47 | Oral administration (10 mg/kg) | 3500 | 44100 | 5.45 |
| Example 53 | Oral administration (10 mg/kg) | 5792 | 83656 | 12.12 |

Conclusion: a high plasma concentration and a large area under the curve can be seen for compounds 47 and 53 of the present invention, and these compounds have good pharmacokinetic properties.

**Test Example 5. Pharmacodynamic testing of Compounds 47 and 53 of the present invention in mice**

1. Experimental purpose

Anti-tumor effects and Safeties of the Compounds 47 and 53 of the present invention in a BALB/c nude mouse animal model subjected to subcutaneous xenotransplantation of OVCAR-3 human ovarian adenocarcinoma tumor mass were evaluated.

2. Experimental animals

BALB/c; nude mice, female, 6-7 weeks old, purchased from Jiangsu Gempharmatech Biotechnology Co., Ltd.

3. Preparation of test compounds

Mice in a solvent control group were given DMA: CrEL: 5%GS=10: 10: 80 (v: v: v). AMG650: a proper amount of AMG-650 was weigned, proper amounts of DMA, CrEL and 5% GS were added in sequence, and the mixture was well mixed by ultrasonic vortex to prepare 0.8 mg/mL drug preparations respectively, wherein DMA: CrEL : 5% GS=10: 10: 80 (v: v: v).

Compound 47: a proper amount of Compound 47 was added with proper amounts of DMA, CrEL and 5% GS in sequence, and evenly mixed by ultrasonic vortex to prepare 0.8 mg/mL drug preparations respectively, wherein DMA, CrEL and 5% GS=10: 10: 80 (v: v: v).

Compound 53: a proper amount of Compound 53 was weighed, proper amounts of DMA, CrEL and 5% GS were added in sequence, and the mixture was well mixed by ultrasonic vortex to prepare 0.8 mg/mL drug preparations respectively, wherein DMA: CrEL : 5% GS=10: 10: 80 (v: v: v).

4. Acquisition of inoculation tumor mass

An appropriate OVCAR-3 tumor-bearing animal was selected, and a 1 mm×1 mm×1 mm OVCAR-3 tumor mass was acquired aseptically and placed into normal saline for later use, which was used for subcutaneous tumor inoculation in a right side of back of the BALB/c nude mice.

5. Animal inoculation and grouping

About 1 mm×1 mm×1 mm OVCAR-3 tumor mass was subcutaneously inoculated in the right side of back of the female BALB/c nude mice. When an average tumor volume reached about 100-250 $mm^3$, the mice were randomly grouped based on tumor size, with 6 mice in each group.

6. Administration and observation to animals

Animals in each group were given the test compound once per day according to an animal weight at a fixed time every day by oral administration (po), the animals started to be administered with the test compound for the first time on the day of grouping for 35 consecutive days, and the animal weight was recorded every day.
The 1st group (G1) was a solvent control group; and
The 2nd-4th groups (G2-G3) were orally administered with AMG-650, Compound 47 and Compound 53 at an administration dosage of 8 mg/kg once per day (QD).
Tumor formation at an inoculation site of the animals in each group was observed, and a tumor volume was measured twice per week, and calculated according to the following formula:
a tumor volume (TV), a relative tumor volume (RTV), a relative tumor proliferation rate (T/C) and a relative tumor inhibition rate (TGI) were calculated according to the following formulas:

(1)

$$\text{TV (tumor volume)} = 1/2 \times a \times b^2,$$

wherein a and b respectively represented a tumor length and a tumor width;
(2)

$$\text{RTV (relative tumor volume)} = V_t/V_0,$$

wherein $V_0$ was a tumor volume measured at the time of grouping, and Vt was a tumor volume in each measurement;
(3)

$$T/C\ (\%) = T_{RTV}/\ C_{RTV} \times 100\%,$$

wherein $T_{RTV}$ was RTV of a treatment group, and $C_{RTV}$ was RTV of a solvent control group; and
(4)

$$\text{TGI\%} = (1 - T/C) \times 100\%,$$

wherein T and C were relative tumor volumes of the treatment group and the solvent control group at a specific time point respectively.

7. Results

Table 5 Pharmacodynamic analysis table on various groups of to-be-tested compounds in OVCAR-3 human ovarian adenocarcinoma tumor model

| Group | 35 days after administration | | |
|---|---|---|---|
| | Tumor volume (x±S) | Relative tumor volume (x±S) | TGI% |
| 1st group<br>Solvent control group | 954±241 | 4.96±0.98 | - |
| 2nd group<br>AMG650 (8 mg/kg) | 313±76 | 1.58±0.27 | 68.1% |
| 3rd group<br>Compound 47 (8 mg/kg) | 40±20 | 0.22±0.11 | 95.5% |
| 4th group<br>Compound 53 (8 mg/kg) | 21±9 | 0.10±0.04 | 98.0% |
| Note: 1. the data were expressed by "mean value ± standard error"; | | | |

Conclusion: under conditions set in this experiment, in the BALB/c nude mouse animal model subjected to subcutaneous xenotransplantation of OVCAR-3 human ovarian adenocarcinoma tumor mass, compared with the solvent control group and the AMG650, both of Compounds 47 and 53 of the present invention show a more significant inhibitory effect on tumor growth at 8 mg/kg.

## Claims

**1.** A compound as shown in general formula (I), or a stereoisomer thereof, a tautomer thereof or a pharmaceutically acceptable salt thereof:

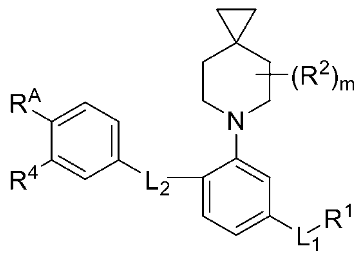

**(I)**

wherein:

$R^A$ is selected from $-OR^B$, $-NR^BR^C$, $-S(=O)_rR^B$, $-C(=O)R^5$, $-C(=O)OR^5$, $-NHC(=O)R^5$, $-NHC(=O)OR^5$, $-C(=O)NR^6R^7$, $-CH_2OR^5$, $-CH_2NR^6R^7$, 5-10 membered aryl, 5-10 membered heteroaryl or 6-14 membered fused ring, wherein the aryl, the heteroaryl or the fused ring is optionally further substituted by one or more $R^a$;

each $R^B$ is the same or different, and is independently selected from deuterated alkyl, 5-10 membered aryl, 5-10 membered heteroaryl or 6-14 membered fused ring, wherein the aryl, the heteroaryl or the fused ring is optionally further substituted by one or more $R^a$;

$R^C$ is selected from a hydrogen atom or alkyl;

each $R^a$ is the same or different, and is independently selected from halogen, hydroxyl, cyano, alkyl, cycloalkyl or alkoxy, wherein the alkyl, the cycloalkyl or the alkoxy is optionally further substituted by one or more substituents selected from halogen, hydroxyl, cyano, alkyl or alkoxy;

$R^1$ is selected from a hydrogen atom, cyano, halogen, alkyl, hydroxyl, cycloalkyl, heterocyclyl, aryl, heteroaryl, $-OR^5$, $-C(=O)R^5$, $-C(=O)OR^5$, $-NHC(=O)R^5$, $-NHC(=O)OR^5$, $-NR^6R^7$, $-C(=O)NR^6R^7$, $-CH_2NHC(=O)OR^5$, $-CH_2NR^6R^7$ or $-S(=O)_rR^5$, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

$L_1$ is selected from a bond, $-C_{1-6}$ alkylene-, $-C_{0-4}$ alkylene-$NR^cS(=O)(=NH)$-$C_{0-4}$ alkylene-, $-C_{0-4}$ alkylene-S-$C_{0-4}$ alkylene-, $-C_{0-4}$ alkylene-S(=O)-$C_{0-4}$ alkylene-, $-C_{0-4}$ alkylene-$SO_2$-$C_{0-4}$ alkylene-, $-C_{0-4}$ alkylene-S(=O)(=NH)-$C_{0-4}$ alkylene-, $-C_{0-4}$ alkylene-$NR^cS0_2$-$C_{0-4}$ alkylene-, $-C_{0-4}$ alkylene-$SO_2NR^c$-$C_{0-4}$ alkylene-, $-C_{0-4}$ alkylene-O-$C_{0-4}$ alkylene-, $-C_{0-4}$ alkylene-$NR^c$-$C_{0-4}$ alkylene-, $-C_{0-4}$ alkylene-$NR^cSO_2NR^c$-$C_{0-4}$ alkylene-, $-C_{0-4}$ alkylene-$NR^cC(O)NR^c$-$C_{0-4}$ alkylene-, $-C_{0-4}$ alkylene-$C(O)NR^c$-$C_{0-4}$ alkylene-, $-C_{0-4}$ alkylene-$NR^cC(O)$-$C_{0-4}$ alkylene-, $-C_{0-4}$ alkylene-P-$C_{0-4}$ alkylene-, $-C_{0-4}$ alkylene-$P(=O)_2$-$C_{0-4}$ alkylene, $-C_{0-4}$ alkylene-C(=O)-$C_{0-4}$ alkylene- or $-C_{0-4}$ alkylene-C(=N(OH))-$C_{0-4}$ alkylene-, wherein the $-C_{1-6}$ alkylene- or the $-C_{0-4}$ alkylene- is optionally further substituted by one or more substituents selected from halogen, hydroxyl, cyano, cycloalkyl or alkoxy;

$R^c$ is selected from a hydrogen atom or alkyl;

each $R^2$ is the same or different, and is independently selected from halogen, hydroxyl, cyano, alkyl, cycloalkyl or alkoxy, wherein the alkyl, the cycloalkyl or the alkoxy is optionally further substituted by one or more substituents selected from halogen, hydroxyl, cyano, alkyl or alkoxy;

$L_2$ is selected from

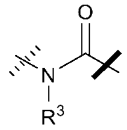 or 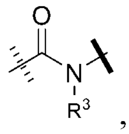 ,

wherein " . . . . . ," represents a linking site of the group to

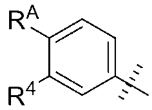

in general formula (I); and " - " represents a linking site of the group to

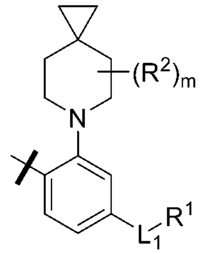

in general formula (1);

each $R^3$ is independently selected from a hydrogen atom or alkyl, wherein the alkyl is optionally further substituted by one or more substituents selected from halogen, hydroxyl, cyano or alkoxy; and $R^3$ is preferably a hydrogen atom;

$R^4$ is selected from cyano, halogen, alkyl, hydroxyl, cycloalkyl, heterocyclyl, aryl, heteroaryl, $-OR^5$, $-C(=O)R^5$, $-C(=O)OR^5$, $-NHC(=O)R^5$, $-NHC(=O)OR^5$, $-NR^6R^7$, $-C(=O)NR^6R^7$, $-CH_2NHC(=O)OR^5$, $-CH_2NR^6R^7$ or $-S(O)_rR^5$, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

each $R^5$ is independently selected from a hydrogen atom, alkyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

each of $R^6$ and $R^7$ is independently selected from a hydrogen atom, hydroxyl, halogen, alkyl, alkoxy, cycloalkyl, heterocyclyl, aryl or heteroaryl, wherein the alkyl, the alkoxy, the cycloalkyl, the heterocyclyl, the aryl or the heteroaryl is optionally further substituted by one or more substituents selected from hydroxyl, halogen, nitro, cyano, alkyl, alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$ or $-NR^9C(=O)R^{10}$;

or, $R^6$ and $R^7$ form a 4-8 membered heterocyclyl together with atoms to which $R^6$ and $R^7$ are linked, wherein the 4-8 membered heterocyclyl contains one or more N, O or S(O)r, and the 4-8 membered heterocyclyl is optionally further substituted by one or more substituents selected from hydroxyl, halogen, nitro, cyano, alkyl, alkoxy, cycloalkyl, heterocyclyl, aryl, heteroaryl, =O, $-C(=O)R^8$, $-C(=O)OR^8$, $-OC(=O)R^8$, $-NR^9R^{10}$, $-C(=O)NR^9R^{10}$, $-SO_2NR^9R^{10}$or $-NR^9C(=O)R^{10}$;

each of $R^8$, $R^9$ and $R^{10}$ is independently selected from a hydrogen atom, alkyl, amino, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or a carboxylate group;

m is 0, 1 or 2; and

each r is independently 0, 1 or 2.

**2.** The compound, or the stereoisomer thereof, the tautomer thereof or the pharmaceutically acceptable salt thereof according to claim 1, which is a compound as shown in formula (II), or a stereoisomer thereof, a tautomer thereof or a pharmaceutically acceptable salt thereof:

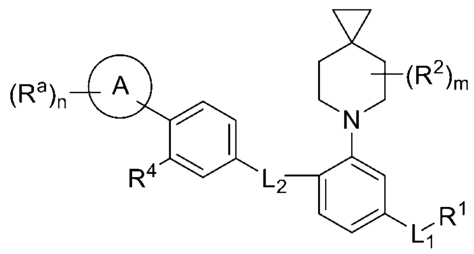

**(II)**

wherein: ring A is selected from 5-10 membered aryl, 5-10 membered heteroaryl or 6-14 membered fused ring; each $R^a$ is the same or different, and is independently selected from halogen, hydroxyl, cyano, $C_1$-$C_3$ alkyl, cyclopropyl or methoxy;
n is 0, 1 or 2; and
$L_1$, $L_2$, $R^1$, $R^2$, $R^4$ and m are as defined in claim 1.

**3.** The compound, or the stereoisomer thereof, the tautomer thereof or the pharmaceutically acceptable salt thereof according to claim 1, wherein $R^A$ is selected from $-OCD_3$, $-C(=O)OH$, $-C(=O)NH_2$, $-C(=O)NHCH_3$, $-C(=O)N(CH_3)_2$, $-C(=O)CH_3$, $-CH_2OH$, $-CH_2OCH_3$ and

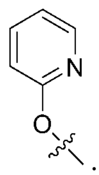

**4.** The compound, or the stereoisomer thereof, the tautomer thereof or the pharmaceutically acceptable salt thereof according to claim 2, wherein n is 0 or 1.

**5.** The compound, or the stereoisomer thereof, the tautomer thereof or the pharmaceutically acceptable salt thereof according to claim 2 or 4, wherein

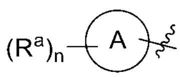

is selected from the following groups:

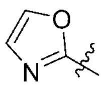, 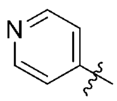, 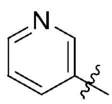, 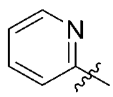, 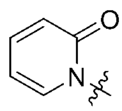, 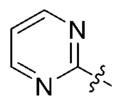, 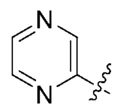,

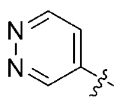, 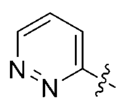, 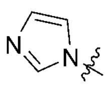, 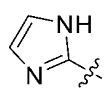, 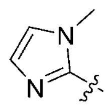, 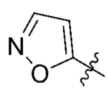, 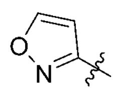, 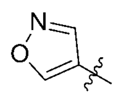, 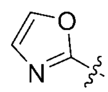,

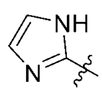, 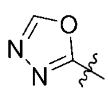, 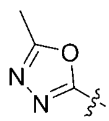, 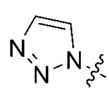, 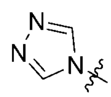, 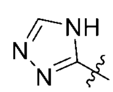, 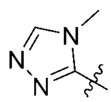, 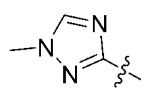, 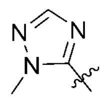,

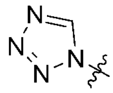, 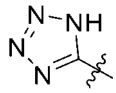, 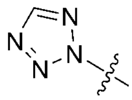, 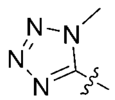, 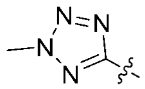 or 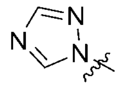.

**6.** The compound, or the stereoisomer thereof, the tautomer thereof or the pharmaceutically acceptable salt thereof according to any one of claims 1 to 3, wherein m is 0.

**7.** The compound, or the stereoisomer thereof, the tautomer thereof or the pharmaceutically acceptable salt thereof according to any one of claims 1 to 3, wherein:

$L_1$ is selected from a bond, -$NR^c$-$C_{0-4}$ alkylene-, -$NR^cSO_2$-$C_{0-4}$ alkylene-, -$SO_2NR^c$-$C_{0-4}$ alkylene-, -$NR^cSO_2NR^c$-, -S(=O)(=NH)-, -$NR^c$S(=O)(=NH)-, -$C_{1-4}$ alkylene-, -S(=O)-, -O-, -C(=O)-, -C(=O)$NR^c$-$C_{0-4}$ alkylene-, -$C_{0-4}$ alkylene-$SO_2$-$C_{0-4}$ alkylene-, -C=N(OH)- or -$NR^c$-C(=O)-, wherein the -$C_{0-4}$ alkylene- or the -$C_{1-4}$ alkylene- is optionally further substituted by one or more substituents selected from halogen, hydroxyl, cyano, cyclopropyl or methoxy; and

each $R^c$ is independently selected from a hydrogen atom or methyl.

**8.** The compound, or the stereoisomer thereof, the tautomer thereof or the pharmaceutically acceptable salt thereof according to claim 7, wherein $L_1$ is selected from a bond, -$NHSO_2CH_2CH_2$-, -$SO_2NHCH_2CH_2$-, -$SO_2$-, -$CH_2SO_2$-, -$NHSO_2$-, -$SO_2NH$-, -$NHC(CH_3)_2CH_2$-, -C(=O)$NHCH_2CH_2$-, -C(=O)$NHC(CH_3)_2CH_2$-, -C(=O)$N(CH_3)CH_2CH_2$-, -$CH(CH_3)(OH)CH_2$-, -$NHSO_2CH(CH_3)CH_2$-, -$SO_2NHC(CH_3)_2CH_2$-, -C(=O)NH-, -$NHCH_2CH_2$ or -$CH_2SO_2CH_2CH_2$-.

**9.** The compound, or the stereoisomer thereof, the tautomer thereof or the pharmaceutically acceptable salt thereof according to any one of claims 1 to 3, wherein $R^1$ is selected from a hydrogen atom, hydroxyl, alkyl, heterocyclyl, cycloalkyl or heteroaryl, wherein the alkyl, the heterocyclyl, the cycloalkyl or the heteroaryl is optionally further substituted by one or more substituents selected from hydroxyl or alkyl.

**10.** The compound, or the stereoisomer thereof, the tautomer thereof or the pharmaceutically acceptable salt thereof according to any one of claims 1 to 3, wherein

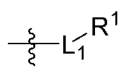

is

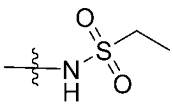, 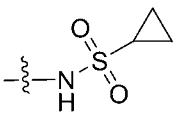 or 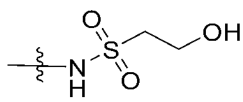.

**11.** The compound, or the stereoisomer thereof, the tautomer thereof or the pharmaceutically acceptable salt thereof according to any one of claims 1 to 3, wherein $L_2$ is selected from

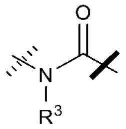,

wherein " ..... ." represents a linking site of the group to

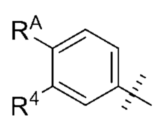

in general formula (I); and "-" represents a linking site of the group to

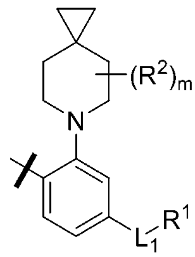

in general formula (I); and
$R^3$ is a hydrogen atom.

**12.** The compound, or the stereoisomer thereof, the tautomer thereof or the pharmaceutically acceptable salt thereof according to any one of claims 1 to 3, wherein $R^4$ is selected from $-OR^b$, $-NHR^b$, 3-6 membered cycloalkyl or 3-6 membered heterocyclyl, wherein the cycloalkyl or the heterocyclyl is optionally further substituted by one or more substituents selected from halogen, alkyl and hydroxyl; and
$R^b$ is selected from alkyl, 3-6 membered cycloalkyl or 3-6 membered heterocyclyl, wherein the alkyl, the cycloalkyl or the heterocyclyl is optionally further substituted by one or more halogens.

**13.** The compound, or the stereoisomer thereof, the tautomer thereof or the pharmaceutically acceptable salt thereof according to any one of claims 1 to 3, wherein $R^4$ is halogen, methyl, difluoromethyl, trifluoromethyl, methoxy, trifluoroethoxy,

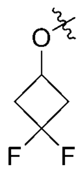 , 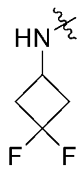 ,

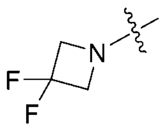 , 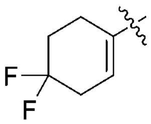 , 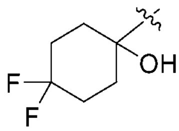 , 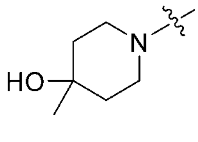 , 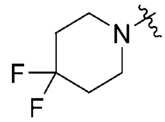 or 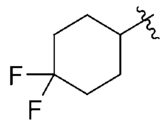 .

**14.** The compound, or the stereoisomer thereof, the tautomer thereof or the pharmaceutically acceptable salt thereof according to any one of claims 1 to 13, wherein the compound is:

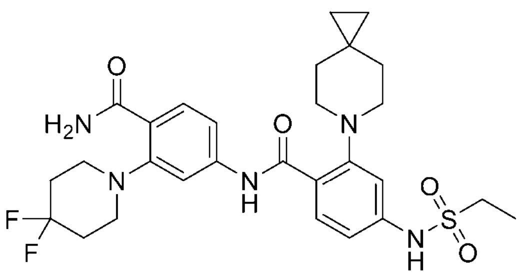

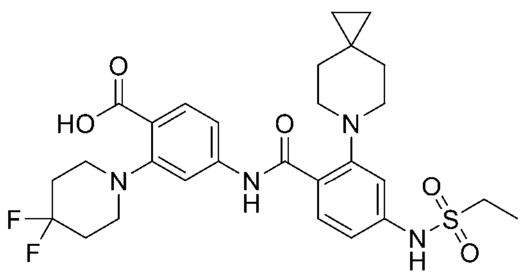

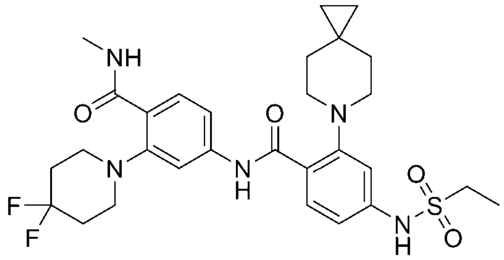

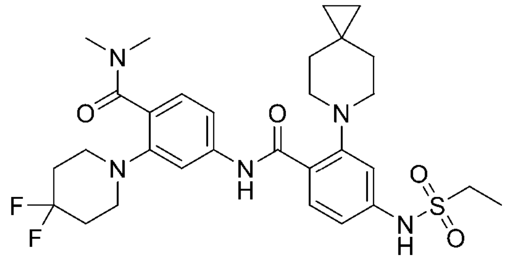

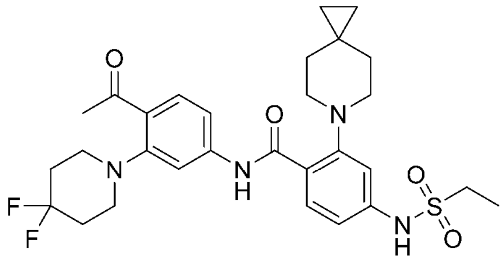

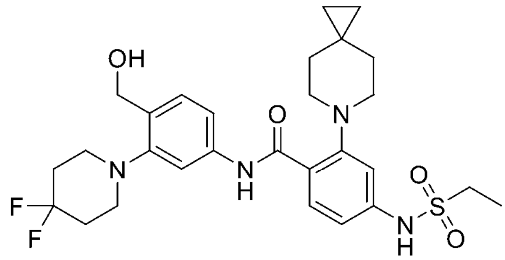

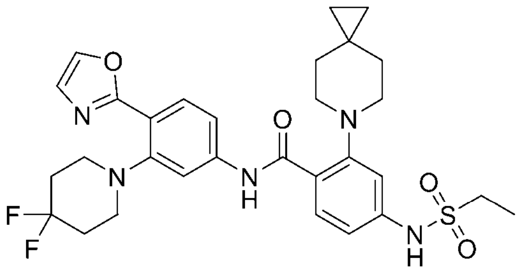

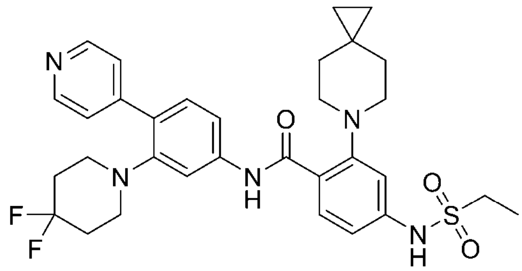

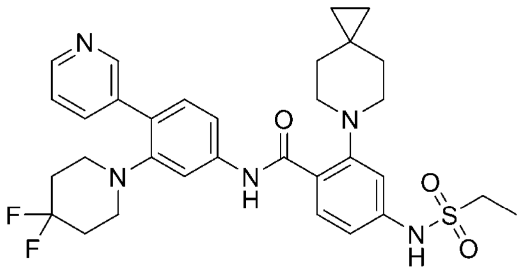

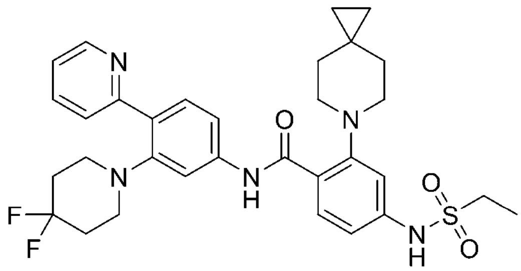

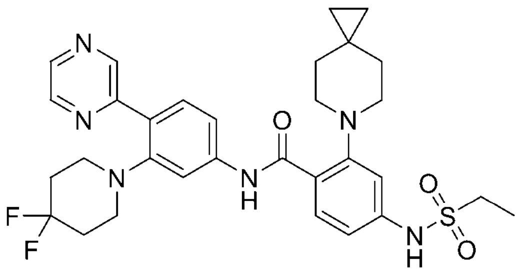

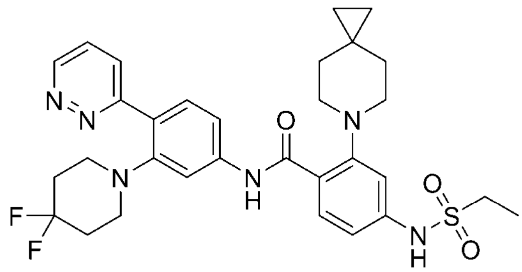

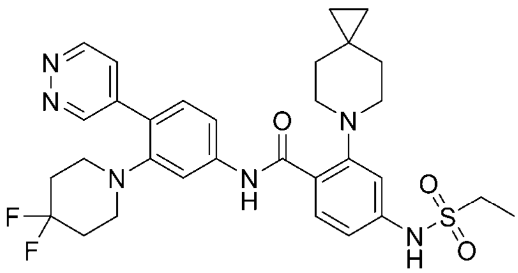

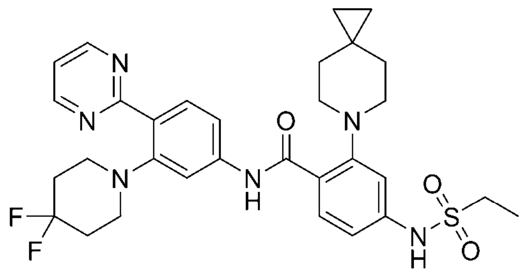

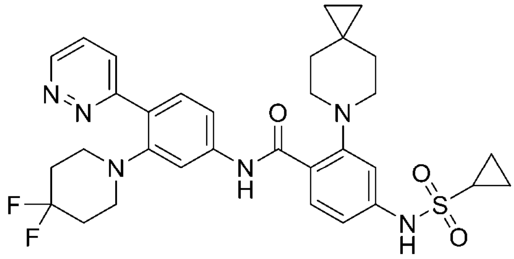

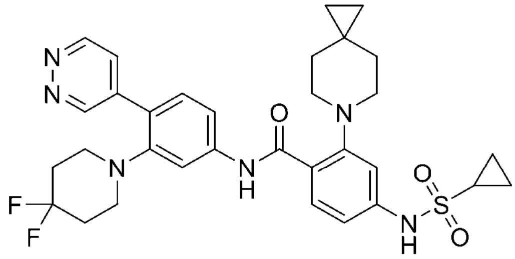

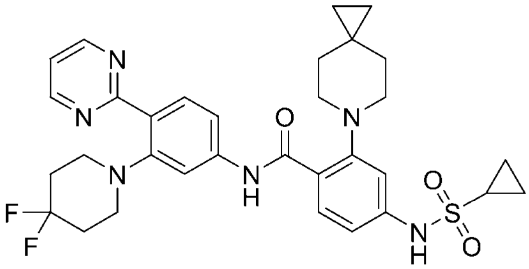

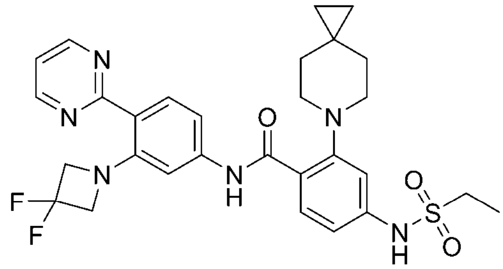

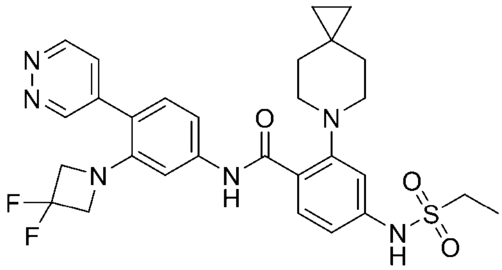

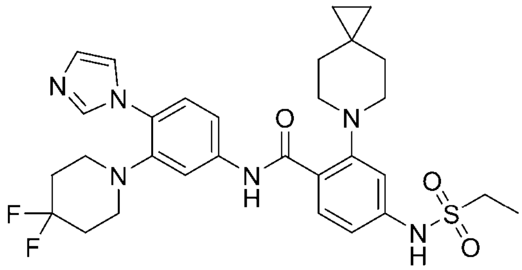

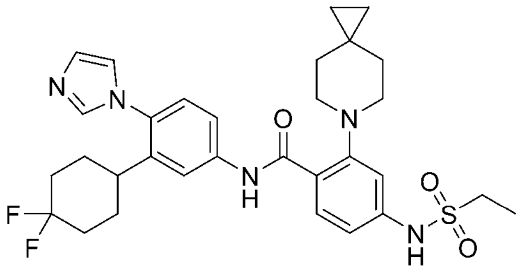

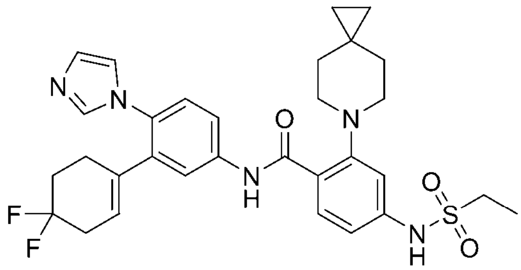

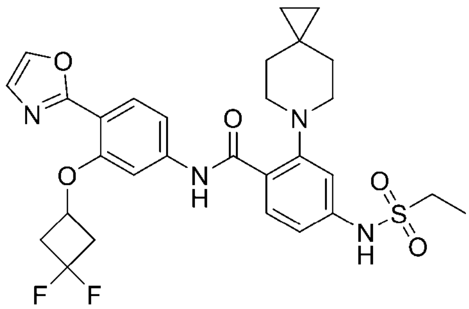

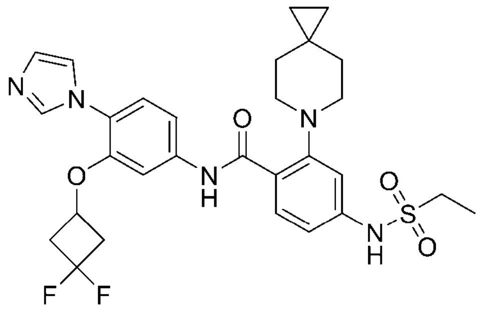

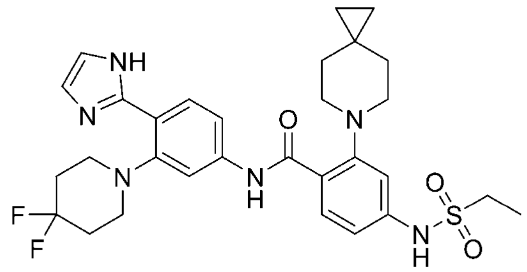

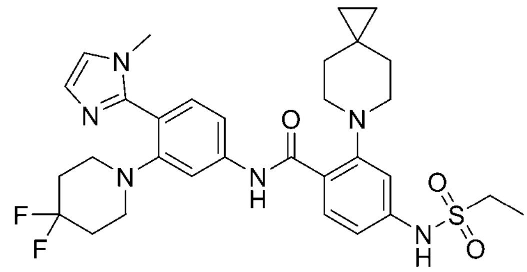

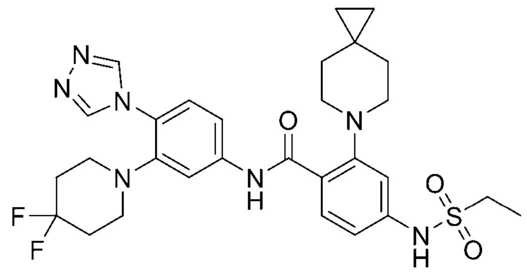

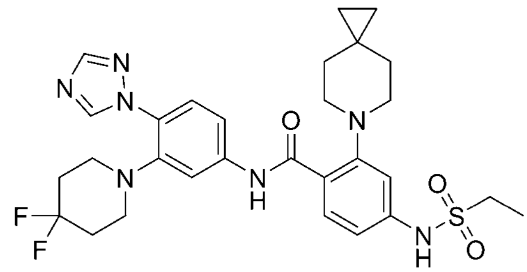

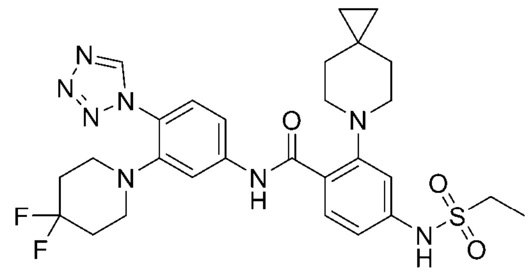

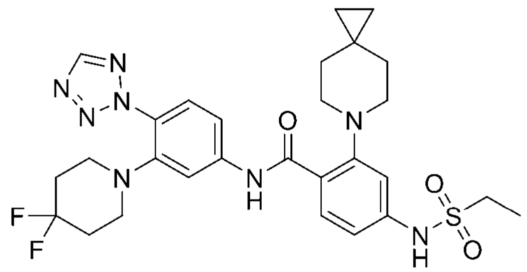

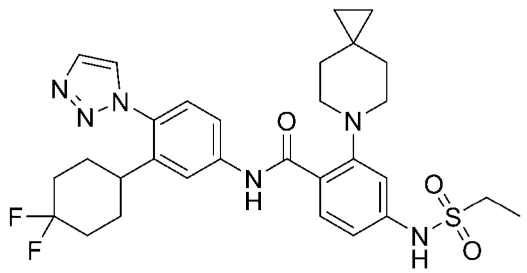

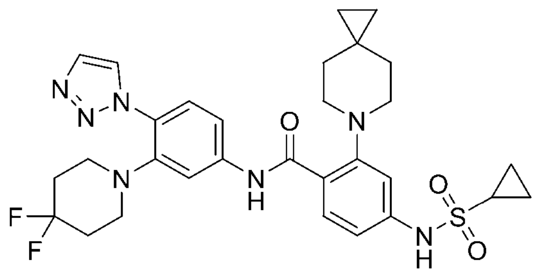

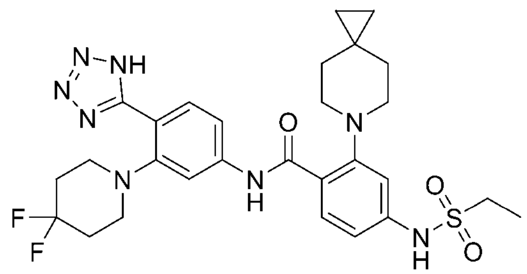

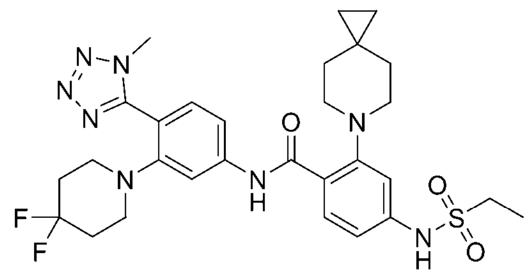

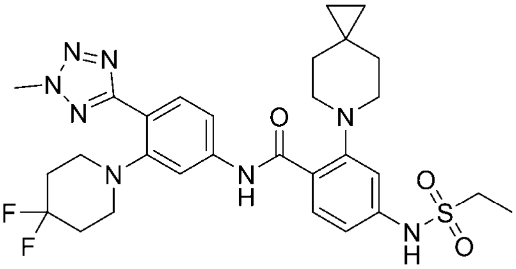

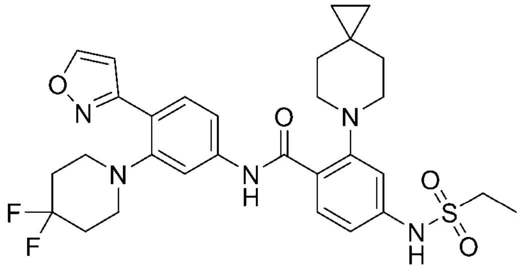

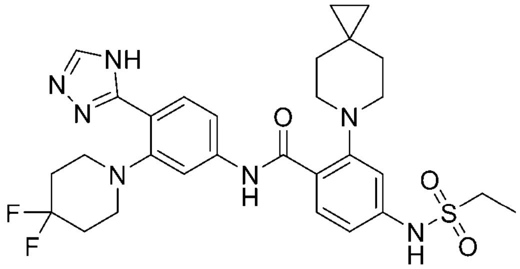

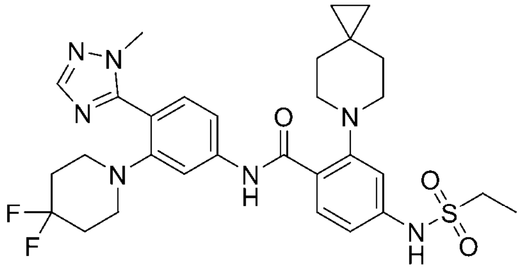

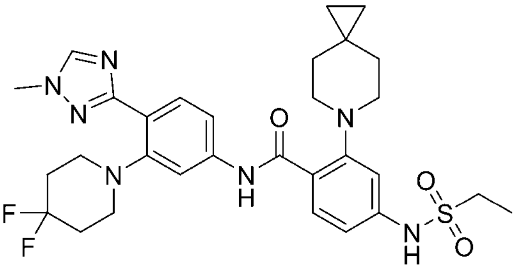

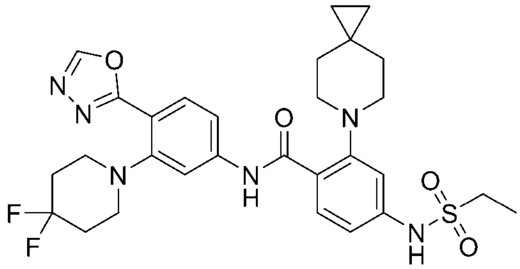

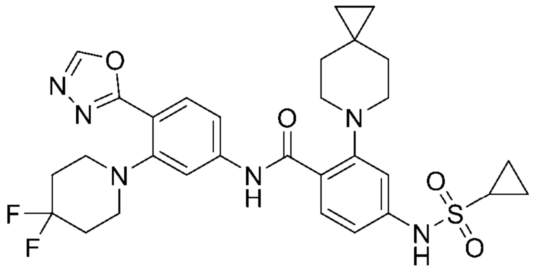

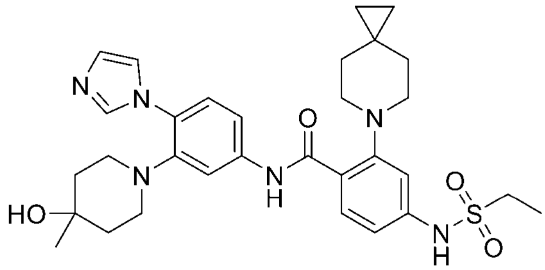

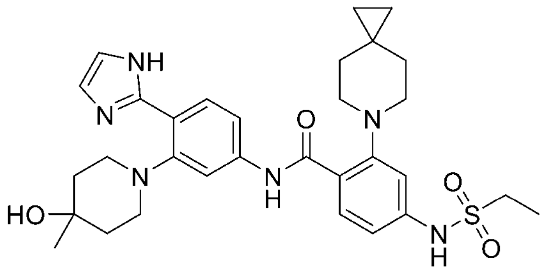

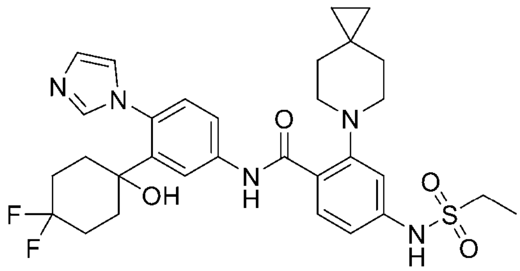

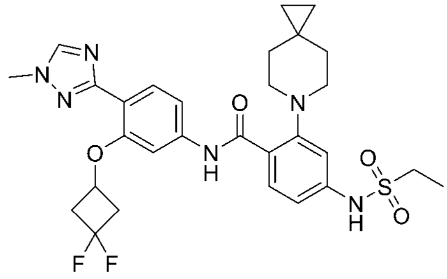

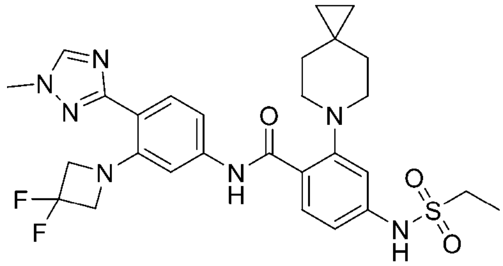

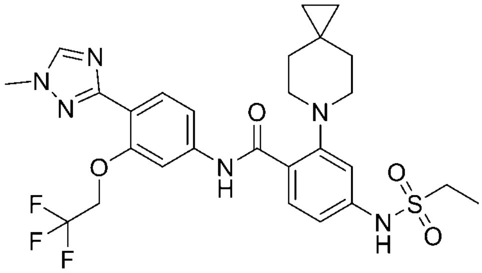

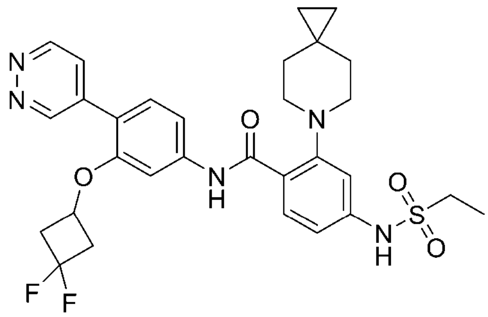

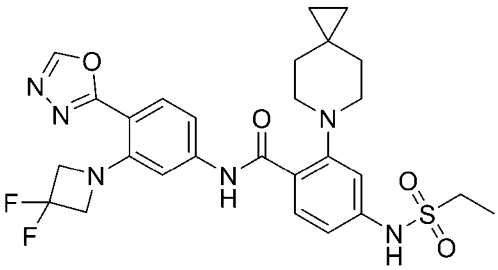

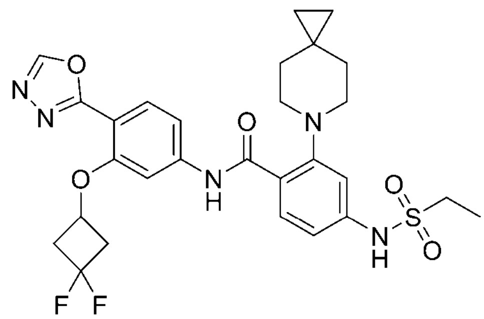

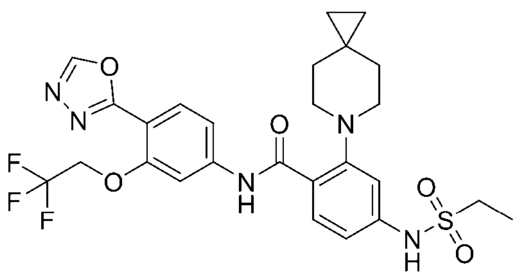

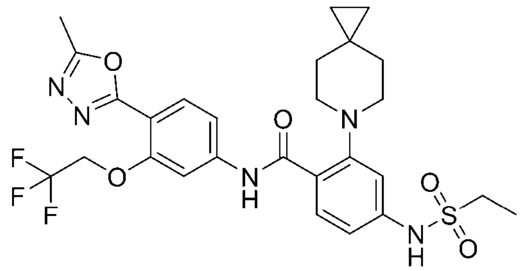

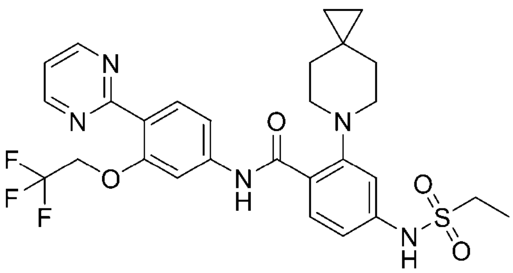

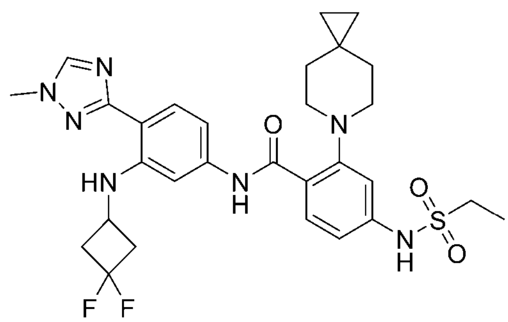

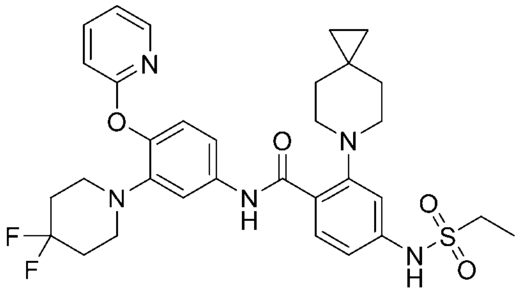

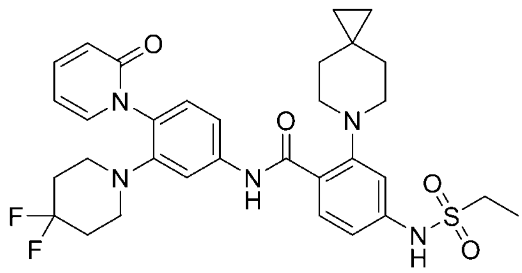

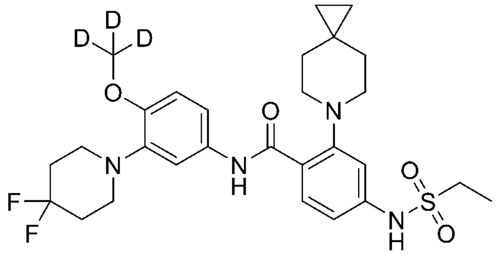

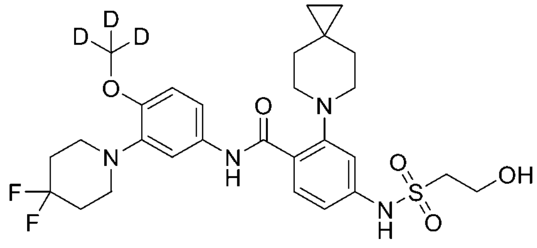

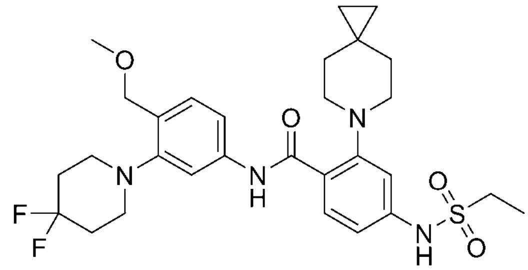

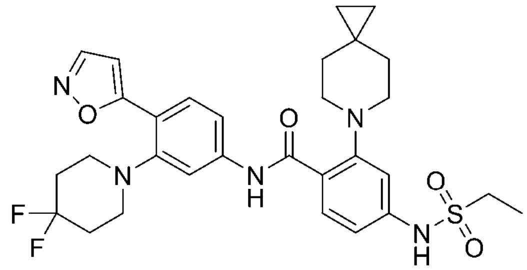

or

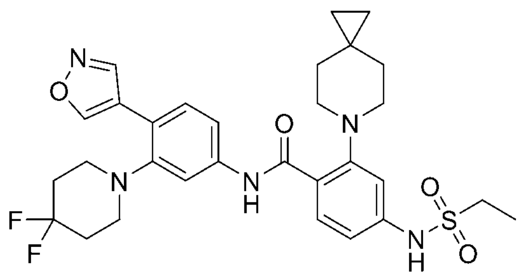

.

**15.** A pharmaceutical composition, wherein the pharmaceutical composition contains an effective amount of the compound, or the stereoisomer thereof, the tautomer thereof or the pharmaceutically acceptable salt thereof according to any one of claims 1 to 14, and a pharmaceutically acceptable carrier, an excipient or a combination of the pharmaceutically acceptable carrier and the excipient.

**16.** Use of the compound, or the stereoisomer thereof, the tautomer thereof or the pharmaceutically acceptable salt thereof according to any one of claims 1 to 14, or the pharmaceutical composition according to claim 15 in the preparation of a KIF18A inhibitor.

**17.** Use of the compound, or the stereoisomer thereof, the tautomer thereof or the pharmaceutically acceptable salt thereof according to any one of claims 1 to 14, or the pharmaceutical composition according to claim 15 in the preparation of a medicament for treating a KIF18A-mediated disease, wherein the KIF18A-mediated disease is preferably cancer.

**18.** The use according to claim 17, wherein the cancer is selected from hepatocellular carcinoma, glioblastoma, colon cancer, breast cancer, lung cancer, cholangiocarcinoma, pancreatic cancer, prostate cancer, bladder cancer, head

cancer, neck cancer, cervical cancer, ovarian cancer, synovial sarcoma, rhabdomyosarcoma, colorectal cancer and lung adenocarcinoma.

**19.** Use of the compound, or the stereoisomer thereof, the tautomer thereof or the pharmaceutically acceptable salt thereof according to any one of claims 1 to 14, or the pharmaceutical composition according to claim 15 in the preparation of a medicament for treating cancer.

**20.** The use according to claim 19, wherein the cancer is selected from hepatocellular carcinoma, glioblastoma, colon cancer, breast cancer, lung cancer, cholangiocarcinoma, pancreatic cancer, prostate cancer, bladder cancer, head cancer, neck cancer, cervical cancer, ovarian cancer, synovial sarcoma, rhabdomyosarcoma, colorectal cancer and lung adenocarcinoma.

**INTERNATIONAL SEARCH REPORT**

International application No.
**PCT/CN2024/106286**

**A. CLASSIFICATION OF SUBJECT MATTER**

C07D 401/12(2006.01)i; C07D 401/14(2006.01)i; C07D 405/14(2006.01)i; C07D 413/14(2006.01)i; A61K 31/444(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

CNTXT; ENTXTC; CNABS; DWPI; WOTXT; EPTXT; USTXT; STN-REGISTRY; STN-HCAPLUS: 浙江海正药业, 上海昂睿医药, 仇宗兴, 张盼盼, 彭记磊, 曹海, 汪泽峰, 时亚琼, 叶成, 徐代旺, 徐肖杰, 周厚江, 苯甲酰胺, 氮杂螺, 辛烷, 磺酰胺, 癌症, 瘤, 根据通式I的structural formula search, KIF18A, Kinesin, azaspiro+, oct, Benzamide, sulfonyl, amino, +cancer+, +tumor+

**C. DOCUMENTS CONSIDERED TO BE RELEVANT**

| Category* | Citation of document, with indication, where appropriate, of the relevant passages | Relevant to claim No. |
|---|---|---|
| X | WO 2023088441 A1 (WIGEN BIOMEDICINE TECHNOLOGY (SHANGHAI) CO., LTD.) 25 May 2023 (2023-05-25)<br>claims 1-36, description, tables 1 and 3-7, and page 44, paragraphs 3 and 4 | 1, 3, 6-20 |
| A | WO 2023088441 A1 (WIGEN BIOMEDICINE TECHNOLOGY (SHANGHAI) CO., LTD.) 25 May 2023 (2023-05-25)<br>claims 1-36, description, tables 1 and 3-7, and page 44, paragraphs 3 and 4 | 2, 4, 5 |
| X | CN 114302880 A (AMGEN INC.) 08 April 2022 (2022-04-08)<br>claims 1-35, and description, table A | 1, 3, 6-20 |
| A | CN 114302880 A (AMGEN INC.) 08 April 2022 (2022-04-08)<br>claims 1-35, and description, table A | 2, 4, 5 |
| X | WO 2022268230 A1 (HANGZHOU INNOGATE PHARMACEUTICAL CO., LTD.) 29 December 2022 (2022-12-29)<br>claims 1-15, and description, tables 1-3 | 1, 3, 6-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 September 2024** | **19 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/106286**

**C. DOCUMENTS CONSIDERED TO BE RELEVANT**

| Category* | Citation of document, with indication, where appropriate, of the relevant passages | Relevant to claim No. |
|---|---|---|
| A | WO 2022268230 A1 (HANGZHOU INNOGATE PHARMACEUTICAL CO., LTD.) 29 December 2022 (2022-12-29)<br>claims 1-15, and description, tables 1-3 | 2, 4, 5 |
| PX | CN 118108704 A (WIGEN BIOMEDICINE TECHNOLOGY (SHANGHAI) CO., LTD.) 31 May 2024 (2024-05-31)<br>claims 1-32, and description, tables 2-5 | 1, 3, 6-20 |
| PX | WO 2024146593 A1 (ZHEJIANG HISUN PHARMACEUTICAL CO., LTD. et al.) 11 July 2024 (2024-07-11)<br>claims 1-20, and description, tables 2-4 | 1, 3, 6-20 |

Form PCT/ISA/210 (second sheet) (July 2022)

**INTERNATIONAL SEARCH REPORT**
**Information on patent family members**

International application No.
**PCT/CN2024/106286**

| Patent document cited in search report | Publication date (day/month/year) | Patent family member(s) | Publication date (day/month/year) |
|---|---|---|---|
| WO 2023088441 A1 | 25 May 2023 | CA 3237625 A1 | 25 May 2023 |
| | | KR 20240108489 A | 09 July 2024 |
| | | AU 2022389558 A1 | 23 May 2024 |
| | | MX 2024005996 A1 | 16 May 2024 |
| | | CN 118215657 A | 18 June 2024 |
| CN 114302880 A | 08 April 2022 | CA 3146693 A1 | 11 February 2021 |
| | | US 2022289724 A1 | 15 September 2022 |
| | | EP 4007752 A1 | 08 June 2022 |
| | | JP 2022542967 A | 07 October 2022 |
| | | WO 2021026098 A1 | 11 February 2021 |
| | | AU 2020326627 A1 | 17 March 2022 |
| | | MX 2022001295 A1 | 22 February 2022 |
| WO 2022268230 A1 | 29 December 2022 | AU 2022298761 A1 | 15 February 2024 |
| | | JP 2024526218 A | 17 July 2024 |
| | | EP 4361142 A1 | 01 May 2024 |
| | | KR 20240040742 A | 28 March 2024 |
| | | CA 3224249 A1 | 29 December 2022 |
| | | CN 117980298 A | 03 May 2024 |
| CN 118108704 A | 31 May 2024 | None | |
| WO 2024146593 A1 | 11 July 2024 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020132648 A
- WO 2020176765 A
- WO 2020132648 A1